# EUROPÄISCHE PATENTSCHRIFT

(11) **EP 1 917 248 B1**
(45) Veröffentlichungstag und Bekanntmachung des Hinweises auf die Patenterteilung: **19.08.2015**
(21) Anmeldenummer: 06776283.1
(22) Anmeldetag: 18.07.2006
(51) Int. Cl.: C07D 231/06, C07D 409/06, C07D 417/06, C07D 409/14, A61K 31/4155, A61P 35/00

(54) **1-ACYLDIHYDROPYRAZOLDERIVATE**
1-ACYLDIHYDROPYRAZOL DERIVATIVES
DERIVES DE 1-ACYLDIHYDROPYRAZOL

(30) Priorität: 16.08.2005 DE 102005038537
(43) Veröffentlichungstag der Anmeldung: 07.05.2008
(73) Patentinhaber: Merck Patent GmbH, 64293 Darmstadt (DE)
(72) Erfinder: DORSCH, Dieter, 64372 Ober-Ramstadt (DE); SCHADT, Oliver, 63517 Rodenbach (DE); BLAUKAT, Andree, 64367 Muehltal (DE)
(86) Internationale Anmeldenummer: PCT/EP2006/007055
(87) Internationale Veröffentlichungsnummer: WO 2007/019933

(56) Entgegenhaltungen:
- EP-A2- 0 537 580
- WO-A-01/32173
- WO-A2-03/079973

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten.

Die vorliegende Erfindung betrifft insbesondere Verbindungen und die Verwendung von Verbindungen, bei denen die Hemmung, Regulierung und/oder Modulation der Signaltransduktion von Met-Kinase eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Rolle der Rezeptortyrosinkinase Met bei der menschlichen Onkogenese, sowie die Möglichkeit der Inhibierung der HGF(hepatocycte growth factor)-abhängigen Met-Aktivierung wird von S. Berthou et al. in Oncogene, Vol. 23, Nr. 31, Seiten 5387-5393 (2004) beschrieben. Der dort beschriebene Inhibitor SU11274, eine Pyrrol-Indolin-Verbindung, ist potentiell zur Krebsbekämpfung geeignet. Ein anderer Met-Kinase-Inhibitor zur Krebstherapie ist von J.G. Christensen et al. in Cancer Res. 2003, 63(21), 7345-55 beschrieben. Von einem weiterem Tyrosinkinase-Inhibitor zur Krebsbekämpfung berichten H. Hov et al. in Clinical Cancer Research Vol. 10, 6686-6694 (2004). Die Verbindung PHA-665752, ein Indolderivat, ist gegen den HGF-Rezeptor c-Met gerichtet. Weiter wird dort berichtet, daß HGF und Met erheblich zum malignen Prozess verschiedener Krebsformen, wie z.B. multipler Myeloma, betragen.

Die Synthese von kleinen Verbindungen, die die Signaltransduktion der Tyrosinkinasen und/oder Serin/Threonin-Kinasen, insbesondere der Met-Kinase spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Im einzelnen betrifft die vorliegende Erfindung einzelne Verbindungen gemäß Anspruch 1 umfaßt von der Formel I, die die Signaltransduktion der Met-Kinase hemmen, regulieren und/oder modulieren, Zusammensetzungen, die diese Verbindungen enthalten, sowie Verfahren zu ihrer Verwendung zur Behandlung von Met-Kinasebedingten Krankheiten und Leiden wie Angiogenese, Krebs, Tumorentstehung, -wachstum und - verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Met-Kinasehemmern behandelt werden. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom.

Die vorliegende Erfindung richtet sich auf Verfahren zur Regulation, Modulation oder Hemmung der Met-Kinase zur Vorbeugung und/oder Behandlung von Erkrankungen im Zusammenhang mit unregulierter oder gestörter Met-Kinase-Aktivität. Insbesondere lassen sich die Verbindungen der Formel I gemäß Anspruch 1 auch bei der Behandlung gewisser Krebsformen einsetzen. Weiterhin können die Verbindungen der Formel I gemäß Anspruch 1 verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I gemäß Anspruch 1 zur Isolierung und zur Untersuchung der Aktivität oder Expression von Met-Kinase verwendet werden. Außerdem eigenen sie sich insbesondere zur Verwendung in diagnostischen Verfahren zu Erkrankungen im Zusammenhang mit unregulierter oder gestörter Met-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszenz nachweisbar (Ross et al., 2002, Biochem. J.).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

Andere 4,5-Dihydropyrazole zur Krebsbekämpfung sind in WO 03/079973 A2 beschrieben.

Chinolinderivate sind als Met-Kinase-Inhibitoren in EP 1 411 046 A1 offenbart.

Pyrrol-indolin-derivate kennt man als Met-Kinase-Inhibitoren aus WO 02/096361 A2.

Die Verbindungen 1-Benzylcarbonyl-3-phenyl-4,5-dihydro-1*H*-pyrazol (CA-Nr. 312607-55-9) und 1-Benzylcarbonyl-3-(4-chlor-phenyl)-4,5-dihydro-1*H-*pyrazol (CA-Nr. 350707-73-2) sind bekannt (keine weitere Literaturangabe).

### ZUSAMMENFASSUNG DER ERFINDUNG

Der Anmeldungsgegenstand bezieht sich auf die Definition der Ansprüche; jede Offenbarung, die über den Umfang der Ansprüche hinausgeht, dient nur zu Informationszwecken.

Die Erfindung betrifft einzelne Verbindungen gemäß Anspruch 1 umfaßt von der Formel I 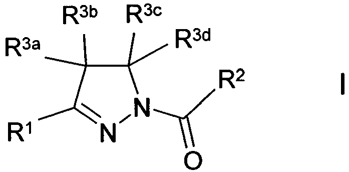 worin
- R¹: Ar oder Het,
- R²: -[C(R⁴)₂]ₙ-Ar oder -[C(R⁴)₂]ₙ-Het,
- R³ R^{3a}, R^{3b}, R^{3c}, R^{3d}: jeweils unabhängig voneinander H oder A,
- R⁴: H, A oder OR³,
- A: unverzweigtes oder verzweigtes Alkyl mit 1-10 C-Atomen,
worin 1-7 H-Atome durch F, Cl und/oder Br ersetzt sein können, und/oder worin eine oder zwei CH₂-Gruppen durch O, S, SO, SO₂ und/oder CH=CH-Gruppen ersetzt sein können,
oder
cyclisches Alkyl mit 3-7 C-Atomen,
- Ar: unsubstituiertes oder ein-, zwei- oder dreifach durch Hal, A, OR³, N(R³)₂, SR³, NO₂, CN, COOR³, CON(R³)₂, NR³COA, NR³SO₂A, SO₂N(R³)₂, S(O)ₘA, CO-Het¹, Het¹, O[C(R³)₂]ₚN(R³), O[C(R³)₂]pHet¹, NHCONH[C(R³)₂]ₚN(R³), NHCOO[C(R³)₂]ₚN(R³), NHCOO[C(R³)₂]ₚHet¹, CHO und/oder COA substituiertes Phenyl, Naphthyl oder Biphenyl,
- Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OR³, N(R³)₂, SR³, N02, CN, COOR³, CON(R³)₂, O[C(R³)₂]ₚN(R³), O[C(R³)₂]ₚHet¹, NHCONH[C(R³)₂]pN(R³), NHCOO[C(R³)₂]ₚN(R³), NR³COA, NR³SO₂A, COR³, SO₂N(R³)₂, S(O)ₘA, CHO, COA, =S, =NH, =NA und/oder =O (Carbonylsauerstoff) substituiert sein kann,
- Het¹: einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
- Hal: F, Cl, Br oder I,
- m: 0, 1 oder 2,
- n: 1 oder 2,
- p: 1, 2, 3 oder 4,
bedeuten,
sowie ihre pharmazeutisch verwendbaren Solvate, Salze, Tautomere und Stereoisomere, einschließlich deren Mischungen in allen Verhältnissen, wobei die Verbindungen 1-Benzylcarbonyl-3-phenyl-4,5-dihydro-1*H*-pyrazol und 1-Benzylcarbonyl-3-(4-chlor-phenyl)-4,5-dihydro-1*H*-pyrazol ausgenommen sind.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I gemäß Anspruch 1 und ihre Salze sowie ein Verfahren zur Herstellung von Verbindungen der Formel I gemäß Anspruch 1 sowie ihrer pharmazeutisch verwendbaren Salze, Solvate, Tautomeren und Stereoisomeren, dadurch gekennzeichnet, daß man
a) eine Verbindung der Formel II

   R¹-CO-CH₂CH₂-L II,

   worin R¹ die in Anspruch 1 entsprechende Bedeutung hat und L Cl, Br, I oder eine freie oder reaktionsfähig funktionell abgewandelte OH-Gruppe bedeutet,
   mit Hydrazin und einer Verbindung der Formel III

   R²-COOH III,

   worin R² die in Anspruch 1 entsprechende Bedeutung hat,
   umsetzt,
   oder
b) zur Herstellung von Verbindungen der Formel I,
   worin R^{3a}, R^{3b}, R^{3c} und R^{3d} H bedeuten,
   i) eine Verbindung der Formel II mit einer Verbindung der Formel IV

      H₂N-NH-CO-R² IV,

      worin R² die in Anspruch 1 entsprechende Bedeutung hat,
      umsetzt,
      oder
   ii) eine Verbindung der Formel V 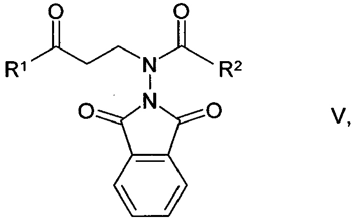 worin R¹ und R² die in Anspruch 1 entsprechenden Bedeutungen haben,
      mit Hydrazin oder Methylhydrazin umsetzt,
      oder
   iii) eine Verbindung der Formel VI

      R¹-CO-CH=CH₂ VI,

      worin R¹ die in Anspruch 1 entsprechende Bedeutung hat,
      mit Hydrazin und einer Verbindung der Formel VII

      L-CO-R² VII,

      worin R² die in Anspruch 1 entsprechende Bedeutung hat und
      L Cl, Br, I oder eine freie oder reaktionsfähig funktionell abgewandelte OH-Gruppe bedeutet,
      umsetzt,
   oder
c) zur Herstellung von Verbindungen der Formel I,
   worin
   - R^{3a}, R^{3b}: H,
   - R^{3c}: H oder A,
   - R^{3d}: H oder A,
   bedeuten,
   mit der Maßgabe, daß mindestens einer der Reste R^{3c} oder R^{3d} A bedeutet,
   eine Verbindung der Formel VIII 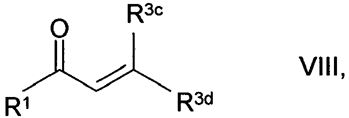 worin
   R¹ und A die in Anspruch 1 entsprechenden Bedeutungen haben,
   - R^{3a}, R^{3b}: H,
   - R^{3c}: H oder A,
   - R^{3d}: H oder A,
   bedeuten,
   mit der Maßgabe, daß mindestens einer der Reste R^{3c} oder R^{3d} A bedeutet,
   mit Hydrazin und einer Verbindung der Formel III umsetzt,
   oder
d) zur Herstellung von Verbindungen der Formel I,
   worin
   - R^{3c}, R^{3d}: H,
   - R^{3a}: H oder A,
   - R^{3b}: H oder A,
   bedeuten,
   mit der Maßgabe, daß mindestens einer der Reste R^{3a} oder R^{3b} A bedeutet,
   eine Verbindung der Formel IX 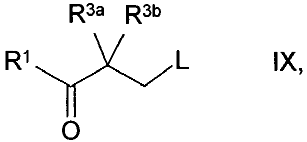 worin
   R¹ und A die in Anspruch 1 entsprechenden Bedeutungen haben,
   - R^{3c}, R^{3d}: H,
   - R^{3a}: H oder A,
   - R^{3b}: H oder A,
   bedeuten,
   mit der Maßgabe, daß mindestens einer der Reste R^{3a} oder R^{3b} A bedeutet,
   und L Cl, Br, I oder eine freie oder reaktionsfähig funktionell abgewandelte OH-Gruppe bedeutet,
   mit Hydrazin und einer Verbindung der Formel III umsetzt,
   und/oder
   eine Base oder Säure der Formel I in eines ihrer Salze umwandelt.

Vor- und nachstehend haben die Reste R¹, R², R^{3a}, R^{3b}, R^{3c}, R^{3d} die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methyl-sulfonamido)-phenyl, o-, m- oder p-(Methylsulfonyl)-phenyl, o-, m- oder p-Methylsulfanylphenyl, o-, m- oder p-Cyanphenyl, o-, m- oder p-Carboxyphenyl, o-, m- oder p-Methoxycarbonylphenyl, o-, m- oder p-Formylphenyl, o-, m- oder p-Acetylphenyl, o-, m- oder p-Aminosulfonylphenyl, o-, m- oder p-(Morpholin-4-ylcarbonyl)-phenyl, o-, m- oder p-(Morpholin-4-ylcarbonyl)-phenyl, o-, m- oder p-(3-Oxo-morpholin-4-yl)-phenyl, o-, m- oder p-(Piperidinyl-carbonyl)-phenyl, o-, m- oder p-[2-(Morpholin-4-yl)ethoxy]-phenyl, o-, m- oder p-[3-(N,N-Diethylamino)propoxy]-phenyl, o-, m- oder p-[3-(3-Diethylaminopropyl)-ureido]-phenyl, o-, m- oder p-(3-Diethylamino-propoxy-carbonylamino)-phenyl, weiter bevorzugt 2,3-, 2,4-, 2,5-, 2,6-, 3,4-oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitro-phenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethyl-aminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

In einer weiteren Ausführungsform bedeutet Ar vorzugsweise unsubstituiertes oder ein-, zwei- oder dreifach durch Hai, A, OR³, N(R³)₂, CN, NO₂, SR³, CON(R³)₂, NR³COA, NR³SO₂A, S(O)ₘA, CO-Het¹, Het¹, O(CH₂)ₚN(R³), O(CH₂)ₚHet¹, NHCOO[C(R³)₂]ₚHet¹, NHCONH(CH₂)pN(R³) und/oder NHCOO(CH₂)pN(R³) substituiertes Phenyl.

Het bedeutet, ungeachtet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7-oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.

Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3-oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder-5-pyrimidinyl, 1-, 2- oder 3-Piperazinyl, 1,2,3,4-Tetrahydro-1-, -2-, -3-, -4-, -5-, -6-, -7- oder-8-chinolyl, 1,2,3,4-Tetrahydro-1-,-2-,-3-, -4-, -5-, -6-, -7- oder -8-isochinotyl, 2-, 3-, 5-, 6-, 7-oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl, 2,3-Dihydro-2-oxo-furanyl, 3,4-Dihydro-2-oxo-1*H-*chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

In einer weiteren Ausführungsform bedeutet Het vorzugsweise einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, NH₂ und/oder =O (Carbonylsauerstoff) substituiert sein kann.

Het¹ bedeutet vorzugsweise einen einkernigen gesättigten Heterocyclus mit 1 bis 2 N und/oder O-Atomen, der ein- oder zweifach durch =O (Carbonylsauerstoff) substituiert sein kann. Het¹ bedeutet besonders bevorzugt Piperidin-1-yl, Pyrrolidin-1-yl, Morpholin-4-yl, Piperazin-1-yl, 1,3-Oxazolidin-3-yl oder Imidazolidinyl, wobei die Reste auch ein- oder zweifach durch =O substituiert sein können.

Het² bedeutet vorzugsweise Benzo[1,2,5]thiadiazolyl, Indolyl, Pyridyl, Pyrimidinyl, Thienyl, Furyl oder Benzodioxolyl, wobei die Reste ein- oder zweifach durch Hal substituiert sein können.

R¹ bedeutet vorzugsweise unsubstituiertes oder ein-, zwei- oder dreifach durch Hal, OA, OH, CN, NO₂, NH₂, NHA, NA₂, SA und/oder NHSO₂A substituiertes Phenyl,
oder
unsubstituiertes oder einfach durch Hal substituiertes Thienyl oder Furanyl, 3,4-Dihydro-2-oxo-1*H*-chinazolinyl, Dibenzofuranyl, 2-Oxo-2,3-dihydro-benzoxazolyl, 2-Oxo-2,3-dihydro-benzimidazolyl, Indolyl, 2-Oxo-1,3-dihydro-indolyl oder 3-Amino-1*H*-indazolyl.

R² bedeutet vorzugsweise -[C(R⁴)₂]ₙ-Ar' oder -[C(R⁴)₂]ₙ-Het², wobei
- Ar': unsubstituiertes oder ein-, zwei- oder dreifach durch Hal, OH, OA, CN, NHCOA, S(O)ₘA, CO-Het¹, Het¹, O(CH₂)ₚN(R³), O(CH₂)ₚHet¹, NHCOO[C(R³)₂]ₚHet¹, NHCONH(CH₂)ₚN(R³) und/oder NHCOO(CH₂)ₚN(R³) substituiertes Phenyl,
- Het¹: einen einkernigen gesättigten Heterocyclus mit 1 bis 2 N und/oder O-Atomen, der ein- oder zweifach durch A und/oder =O (Carbonylsauerstoff) substituiert sein kann,
- Het²: einen ein-, zwei- oder dreikernigen aromatischen oder ungesättigten Heterocyclus mit 1 bis 4 N-, O- und/oder S-Atomen, der unsubstituiert oder ein-, zwei- oder dreifach durch Hal und/oder =O substituiert sein kann,
bedeuten.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsverbindungen der Formeln II, III, IV, V, VI, VII, VIII und IX sind in der Regel bekannt. Sind sie neu, so können sie aber nach an sich bekannten Methoden hergestellt werden.

Verbindungen der Formel I können vorzugsweise erhalten werden, indem man eine Verbindung der Formel II zuerst mit Hydrazin, vorzugsweise Hydrazinhydrat, umsetzt.

In den Verbindungen der Formel II bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Zu der Reaktionslösung gibt man dann eine Carbonsäure der Formel III. Die Reaktion gelingt vorzugsweise in Gegenwart eines Dehydratisierungsmittels, wie z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin.

Die Umsetzung erfolgt in der Regel in einem inerten Lösungsmittel.
Die Reaktionszeit liegt je nach den angewendeten Bedingungen zwischen einigen Minuten und 14 Tagen, die Reaktionstemperatur zwischen etwa -15° und 150°, normalerweise zwischen -5° und 90°, besonders bevorzugt zwischen 20° und 60°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylen-glykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Dichlormethan und/oder DMF.

Verbindungen der Formel I, worin R^{3a}, R^{3b}, R^{3c} und R^{3d} H bedeuten, können weiter vorzugsweise erhalten werden, indem man eine Verbindung der Formel II mit einer Verbindung der Formel IV umsetzt.

Die Umsetzung erfolgt in der Regel in einem inerten Lösungsmittel, wie oben angegeben. Besonders bevorzugt ist Acetonitril, Dichlormethan und/oder DMF.

Die Umsetzung erfolgt in der Regel in Gegenwart eines säurebindenden Mittels vorzugsweise einer organischen Base wie DIPEA, Triethylamin, Dimethylanilin, Pyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Verbindungen der Formel I, worin R^{3a}, R^{3b}, R^{3c} und R^{3d} H bedeuten, können weiter vorzugsweise erhalten werden, indem man eine Verbindung der Formel V mit Hydrazin oder Methylhydrazin umsetzt.

Die Umsetzung erfolgt in der Regel in einem inerten Lösungsmittel, wie oben angegeben. Besonders bevorzugt ist THF.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Die Verbindungen der Formel V erhält man durch Umsetzung einer Verbindung der Formel III mit N-Aminophthalimid und anschließender Umsetzung der Zwischenverbindung mit einer Verbindung der Formel II.

Verbindungen der Formel I, worin R^{3a}, R^{3b}, R^{3c} und R^{3d} H bedeuten, können weiter vorzugsweise erhalten werden, indem man eine Verbindung der Formel VI mit Hydrazin und einer Verbindung der Formel VII umsetzt.

In den Verbindungen der Formel VII bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Aryl sulfonyloxy mit 6-10 C-Atomen (bevorzugt Phenyl- oder p-Tolylsulfonyloxy).

Die Umsetzung erfolgt in der Regel in einem inerten Lösungsmittel, wie oben angegeben. Besonders bevorzugt ist Acetonitril, Dichlormethan und/oder DMF.

Die Umsetzung erfolgt in der Regel in Gegenwart eines säurebindenden Mittels vorzugsweise einer organischen Base wie DIPEA, Triethylamin, Dimethylanilin, Pyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Verbindungen der Formel I,
worin
- R^{3a}, R^{3b}: H,
- R^{3c}: H oder A,
- R^{3d}: H oder A,
bedeuten,
mit der Maßgabe, daß mindestens einer der Reste R^{3c} oder R^{3d} A bedeutet,
können weiter vorzugsweise erhalten werden, indem man eine Verbindung der Formel VIII,
worin
R¹ und A die in Anspruch 1 angegebenen Bedeutungen haben,
- R^{3a}, R^{3b}: H,
- R^{3c}: H oder A,
- R^{3d}: H oder A,
bedeuten,
mit der Maßgabe, daß mindestens einer der Reste R^{3c} oder R^{3d} A bedeutet,
mit Hydrazin und einer Verbindung der Formel III umsetzt.

Die Reaktion gelingt vorzugsweise durch Umsetzung einer Verbindung der Formel VIII mit Hydrazin, vorzugsweise Hydrazinhydrat, und anschließender Zugabe einer Verbindung der Formel III.

Die Reaktion gelingt vorzugsweise in Gegenwart eines Dehydratisierungsmittels, wie z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin.

Die Umsetzung erfolgt in der Regel in einem inerten Lösungsmittel, wie oben angegeben. Vorzugsweise wird die Umsetzung in DMF durchgeführt. Die Reaktionszeit liegt je nach den angewendeten Bedingungen zwischen einigen Minuten und 14 Tagen, die Reaktionstemperatur zwischen etwa -15° und 150°, normalerweise zwischen -5° und 90°, besonders bevorzugt zwischen 20° und 60°C.

Verbindungen der Formel I,
worin
- R^{3c}, R^{3d}: H,
- R^{3a}: H oder A,
- R^{3b}: H oder A,
bedeuten,
mit der Maßgabe, daß mindestens einer der Reste R^{3a} oder R^{3b} A bedeutet,
können weiter vorzugsweise erhalten werden, indem man eine Verbindung der Formel IX,
worin
R¹ und A die in Anspruch 1 angegebenen Bedeutungen haben,
- R^{3c}, R^{3d}: H,
- R^{3a}: H oder A,
- R^{3b}: H oder A,
bedeuten,
mit der Maßgabe, daß mindestens einer der Reste R^{3a} oder R^{3b} A bedeutet,
mit Hydrazin und einer Verbindung der Formel III umsetzt.

Die Reaktion erfolgt vorzugsweise durch Umsetzung einer Verbindung der Formel IX mit Hydrazin, vorzugsweise Hydrazinhydrat, und anschließender Zugabe einer Verbindung der Formel III.

Die Reaktion gelingt vorzugsweise in Gegenwart eines Dehydratisierungsmittels, wie z.B. eines Carbodiimids wie N,N'-Dicyclohexylcarbodümid ("DCCI"), 1,1'-Carbonyl-diimidazol oder N-3-Dimethylaminopropyl-N'-ethyl-carbodiimid ("DAPECI"), ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin.

Die Umsetzung erfolgt in der Regel in einem inerten Lösungsmittel, wie oben angegeben. Vorzugsweise wird die Umsetzung in DMF durchgeführt. Die Reaktionszeit liegt je nach den angewendeten Bedingungen zwischen einigen Minuten und 14 Tagen, die Reaktionstemperatur zwischen etwa -15° und 150°, normalerweise zwischen -5° und 90°, besonders bevorzugt zwischen 20° und 60°C.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I gemäß Anspruch 1 werden größtenteils konventionell hergestellt. Sofern die Verbindung der Formel I gemäß Anspruch 1 eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetall-hydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I gemäß Anspruch 1 zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I gemäß Anspruch 1 die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxy-ethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I gemäß Anspruch 1, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlörid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I gemäß Anspruch 1 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I gemäß Anspruch 1 mit Metallen oder Aminen wie Alkalimetalle und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I gemäß Anspruch 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder ÖI-in-Wasser-Flüssigemulsionen oder Wasser-in-ÖI-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I gemäß Anspruch 1 sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I gemäß Anspruch 1 sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I gemäß Anspruch 1 hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I gemäß Anspruch 1 und/oder ihrer pharmazeutisch verwendbaren Salze, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihrer pharmazeutisch verwendbaren Salze, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I gemäß Anspruch 1 und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom. Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.

Die Verwendung von Verbindungen der Formel I gemäß Anspruch 1 und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Ebenfalls umfasst ist die Verwendung der Verbindungen der Formel I gemäß Anspruch 1 und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die vorliegende Erfindung umfasst auch die Verwendung Verbindungen der Formel I gemäß Anspruch 1 und/oder ihre physiologisch unbedenklichen Salze und Solvate zur Herstellung eines Arzneimittels zur Behandlung oder Vorbeugung von Retina-Vaskularisierung.

Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.

Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I gemäß Anspruch 1 können an Patienten zur Behandlung von Krebs, insbesondere schnell wachsenden Tumoren, verabreicht werden.

Besonders bevorzugt ist die Verwendung zur Herstellung eines Arzneimittels zur Behandlung von Krankheiten, die durch Inhibierung von Met-Kinase durch die Verbindungen nach Anspruch 1 beeinflußt werden. Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I gemäß Anspruch 1 können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und Iodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (CI I 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανß3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und In-vivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffrnann-La Roche) |
| | Iproplatin | |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | |
| | | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | |
| | 7-Ethyl-10-hydroxycamptothecin | TAS-103 (Taiho) |
| | | Elsamitrucin (Spectrum) |
| | Topotecan | J-107088 (Merck & Co) |
| | Dexrazoxanet (TopoTarget) | BNP-1350 (BioNumerik) |
| | | CKD-602 (Chong Kun Dang) |
| | Pixantron (Novuspharrna) | |
| | Rebeccamycin-Analogon (Exelixis) | KW-2170 (Kyowa Hakko) |
| | BBR-3576 (Novuspharrna) | |
| | | |
| Antitumor-Antibiotika | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubicin | |
| | Mitoxantron (Novantron) | |
| | | |
| Antimitotische Mittel | Paclitaxel | SB 408075 |
| | Docetaxel | (GlaxoSmithKline) |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) | Nolatrexed (Eximias) |
| | ZD-9331 (BTG) | CoFactor™ (BioKeys) |
| | | |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | 06-Benzylguanin (Paligent) |
| | Thymectacin (NewBiotics) | |
| | Edotreotid (Novartis) | |
| | | |
| Farnesyltransferase-Inhibitoren | Arglabin (NuOncology Labs) | Tipifarnib (Johnson & Johnson) |
| | lonafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| Histonacetyltransferase-Inhibitoren | Tacedinalin (Pfizer) | Pivaloyloxymethylbutyrat (Titan) |
| | SAHA (Aton Pharma) | |
| | MS-275 (ScherinG AG) | Depsipeptid (Fujisawa) |
| | | |
| Metalloproteinase-Inhibitoren Ribonucleosidred uktase-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | | Didox (Molecules for Health) |
| | Galliummaltolat (Titan) | |
| | Triapin (Vion) | |
| | | |
| TNF-alpha-Agonisten / Antagonisten | Virulizin (Lorus Therapeutics) | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys) |
| | Oncophage (Antigenics) | |
| | GMK (Progenics) | Pentrix (Australian Cancer Technology) |
| | Adenokarzinom-Impfstoff (Biomira) | |
| | | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe (CTL Immuno) | MGV (Progenics) |
| | | !3-Alethin (Dovetail) |
| | Melanom-Impfstoff (CTL Immuno) | CLL-Thera (Vasogen) |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteron- | Goserelin |
| | caproat | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol (EntreMed) |
| | Tamoxifen | |
| | Toremofin | Arzoxifen (Eli Lilly) |
| | Dexamethason | |
| | | |
| Photodynamische Mittel | Talaporfin (Light Sciences) | Pd-Bacteriopheophorbid (Yeda) |
| | Theralux (Theratechnologies) | |
| | | Lutetium-Texaphyrin (Pharmacyclics) |
| | Motexafin-Gadolinium (Pharmacyclics) | |
| | | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science) | PKC412 (Novartis) |
| | | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 (GlaxoSmithKline) | |
| | EKB-509 (Wyeth) | |
| | EKB-569 (Wyeth) | |
| Verschiedene Mittel | SR-27897 (CCK-A-Inhibitor, Sanofi-Synthelabo) | BCX-1777 (PNP-Inhibitor, BioCryst) |
| | | Ranpirnase (Ribonuclease-Stimulans, Alfacell) |
| | Tocladesin (cyclisches-AMP-Agonist, Ribapharm) | |
| | Alvocidib (CDK-Inhibitor, Aventis) | Galarubicin (RNA-Synthese-Inhibitor, Dong-A) |
| | CV-247 (COX-2-Inhibitor, Ivy Medical) | |
| | | Tirapazamin |
| | P54 (COX-2-Inhibitor, Phytopharm) | (Reduktionsmittel, SRI International) |
| | CapCell™ (CYP450-Stimulans, Bavarian | N-Acetylcystein (Reduktionsmittel, |
| | Nordic) | Zambon) |
| | GCS-IOO (gal3-Antagonist, GlycoGenesys) | R-Flurbiprofen (NF-kappaB-Inhibitor, Encore) |
| | | 3CPA (NF-kappaB-Inhibitor, Active Biotech) |
| | G17DT-Immunogen (Gastrin-Inhibitor, Aphton) | |
| | | Seocalcitol (Vitamin-D- |
| | Efaproxiral (Oxygenator, Allos Therapeutics) | Rezeptor-Agonist, Leo) |
| | | 131-I-TM-601 (DNA-Antagonist, TransMolecular) |
| | PI-88 (Heparanase-Inhibitor, Progen) | |
| | Tesmilifen (Histamin-Antagonist, YM BioSciences) | Eflornithin (ODC-Inhibitor, ILEX Oncology) |
| | | Minodronsäure (Osteoclasten-Inhibitor, Yamanouchi) |
| | Histamin (Histamin-H2-Rezeptor- Agonist, Maxim) | |
| | Tiazofurin (IMPDH-Inhibitor, Ribapharm) | Indisulam (p53-Stimulans, Eisai) |
| | Cilengitid (Integrin-Antagonist, Merck KGaA) | Aplidin (PPT-Inhibitor, PharmaMar) |
| | SR-31747 (IL-1-Antagonist, Sanofi-Synthelabo) | Rituximab (CD20-Antikörper, Genentech) |
| | | Gemtuzumab (CD33-Antikörper, Wyeth Ayerst) |
| | CCI-779 (mTOR-Kinase-Inhibitor, Wyeth) | |
| | | PG2 (Hämatopoese-Verstärker, Pharmagenesis) |
| | Exisulind (PDE-V-Inhibitor, Cell Pathways) | |
| | CP-461 (PDE-V-Inhibitor, Cell Pathways) | Immunol™ (Triclosan-Oralspülung, Endo) |
| | AG-2037 (GART-Inhibitor, Pfizer) | Triacetyluridin (Uridin-Prodrug, Wellstat) |
| | WX-UK1 (Plasminogenaktivator-Inhibitor, Wilex) | SN-4071 (Sarkom-Mittel, Signature BioScience) |
| | | TransMID-107™ (Immunotoxin, KS Biomedix) |
| | PBI-1402 (PMN-Stimulans, ProMetic LifeSciences) | |
| | Bortezomib (Proteasom-Inhibitor, Millennium) | PCK-3145 (Apoptose-Förderer, Procyon) |
| | SRL-172 (T-Zell-Stimulans, SR Pharma) | Doranidazol (Apoptose-Förderer, Pola) |
| | TLK-286 (Glutathion-S-Transferase-Inhibitor, Telik) | CHS-828 (cytotoxisches Mittel, Leo) |
| | | trans-Retinsäure (Differentiator, NIH) MX6 (Apoptose-Förderer, MAXIA) |
| | PT-100 (Wachstumsfaktor-Agonist, Point Therapeutics) | |
| | Midostaurin (PKC-Inhibitor, Novartis) | Apomin (Apoptose-Förderer, ILEX Oncology) |
| | Bryostatin-1 (PKC-Stimulans, GPC Biotech) | Urocidin (Apoptose-Förderer, Bioniche) |
| | CDA-II (Apoptose-Förderer, Everlife) | Ro-31-7453 (Apoptose-Förderer, La Roche) |
| | SDX-101 (Apoptose-Förderer, Salmedix) | Brostallicin (Apoptose-Förderer, Pharmacia) |
| | Ceflatonin (Apoptose-Förderer, ChemGenex) | |
| | | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I gemäß Anspruch 1 wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity- (Sorg et al., J. of. Biomolecular Screening, 2002, 7, 11-19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidase-konjugierten Antikörper durch Chemilumineszenz nachweisbar (Ross et al., 2002, Biochem. J.).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert. Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 µl 0,9% NaCl-Lösung gewaschen. Die Messung der gebundenen Radioaktivität erfolgt mittels eines Szintillationsmessgerätes (Topcount NXT, Fa. Perkin-Elmer).

Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC50) erfolgt unter Verwendung des Programms RS1_MTS ().

Kinase-Reaktionsbedingungen pro well:
30 µl Assaypuffer
10 µl zu testende Substanz in Assaypuffer mit 10 % DMSO
10 µl ATP (Endkonzentration 1 µM kalt, 0,35 µCi ³³P-ATP)
50 µl Gemisch Met Kinase/Substrat in Assaypuffer;
   (10 ng Enzym/well, 50 ng pAGLT/well)

Verwendete Lösungen:
- Assay-Puffer:
   50 mM HEPES
   3 mM Magnesiumchlorid
   3 µM Natrium orthovanadat
   3 mM Mangan (II) chlorid
   1 mM Dithiothreitol (DTT)
   pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez. Aktivität 954 U/mg;
- Poly-Ala-Glu-Lys-Tyr, 6:2:5:1: Sigma Kat.-Nr. P1152

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺
FAB (Fast Atom Bombardment) (M+H)⁺
ESI (Electrospray Ionization) (M+H)⁺
APCI-MS (atmospheric pressure chemical ionization - mass spectrometry) (M+H)⁺.

### Retentionszeit Rₜ [min] : Bestimmung erfolgt mit HPLC

Säule: Chromolith SpeedROD, 50 x 4.6 mm² (Best.Nr. 1.51450.0001) von Merck
Gradient: 5.0 min, t = 0 min, A:B = 95:5, t = 4.4 min: A:B = 25:75,
t = 4.5 min bis t = 5.0 min: A:B = 0:100
Fluß: 3.00 ml/min
Laufmittel A: Wasser + 0,1% TFA (Trifluoressigsäure),
Laufmittel B: Acetonitril + 0,08% TFA
Wellenlänge: 220 nm

### Beispiel 1

Die Herstellung von 1-(Benzo[1,2,5]thiadiazol-5-yl-methylcarbonyl)-3-(2,4-dimethoxy-phenyl)-4,5-dihydro-1*H*-pyrazol ("A1") erfolgt analog nachstehendem Schema 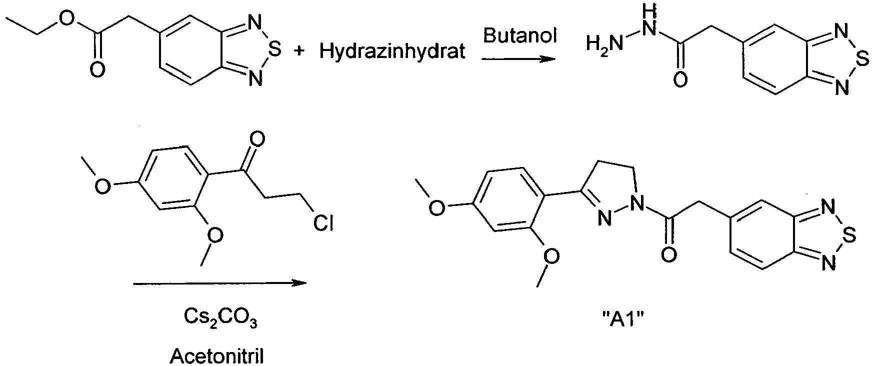
1.1 Eine Lösung von 15.0 g (67.5 mmol) Benzo[1,2,5]thiadiazol-5-yl-essigsäureethylester in 40 ml 1-Butanol wird mit 6.8 ml (140 mmol) Hydrazinhydrat versetzt und die entstandene Lösung 2 Stunden zum Sieden erhitzt. Man lässt das Reaktionsgemisch abkühlen und saugt den entstandenen Niederschlag ab. Der Niederschlag wird mit Ethanol gewaschen und getrocknet: Benzo[1,2,5]thiadiazol-5-yl-essigsäurehydrazid als farblose Kristalle; ESI 209.
1.2 Eine Suspension von 104 mg (0.50 mmol) Benzo[1,2,5]thiadiazol-5-yl-essigsäurehydrazid und 114 mg (0.5 mmol) 3-Chlor-1-(2,4-dimethoxy-phenyl)-propan-1-on in 4 ml Acetonitril wird mit 326 mg (1.00 mmol) Caesiumcarbonat versetzt und 18 Stunden bei Raumtemperatur gerührt. Das Reaktionsgemisch wird filtriert. Das Filtrat wird eingedampft und an einer Kieselgelsäule mit Ethylacetat/Petrolether als Laufmittel chromatographiert: "A1" als farblose Kristalle; ESI 383.

### Beispiel 2

Die Herstellung von 1-(Benzo[1,2,5]thiadiazol-5-yl-methylcarbonyl)-3-(4-fluor-phenyl)-4,5-dihydro-1*H*-pyrazol ("A2") erfolgt analog nachstehendem Schema 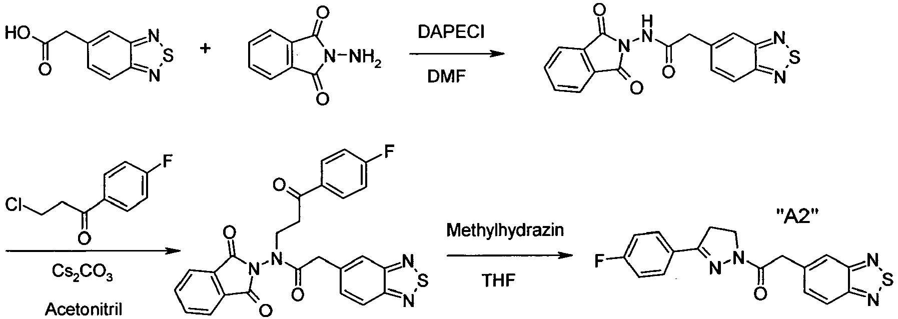
2.1 Eine Lösung von 971 mg (5.00 mmol) Benzo[1,2,5]thiadiazol-5-yl-essigsäure und 811 mg (5.00 mmol) *N*-Aminophthalimid wird mit 1.25 g (6.5 mmol) *N*-(3-Dimethylaminopropyl)-*N*'-ethylcarbodiimidhydrochlorid (DAPECI) versetzt und 18 Stunden bei Raumtemperatur gerührt. Das Reaktionsgemisch wird auf Wasser gegeben, der entstandene Niederschlag abfiltriert, mit Wasser gewaschen und im Vakuum getrocknet: 2-Benzo[1,2,5]thiadiazol-5-yl-*N*-(1,3-dioxo-1,3-dihydro-isoindol-2-yl)-acetamid als farblose Kristalle; ESI 339.
2.2 Eine Lösung von 169 mg (0.50 mmol) 2-Benzo[1,2,5]thiadiazol-5-yl-N-(1,3-dioxo-1,3-dihydro-isoindol-2-yl)-acetamid und 93.3 mg (0.5 mmol) 3-Chlor-1-(4-fluorphenyl)-propan-1-on in 5 ml Acetonitril wird mit 218 mg (0.67 mmol) Caesiumcarbonat versetzt und 18 Stunden bei Raumtemperatur gerührt. Das Reaktionsgemisch wird filtriert und das Filtrat wird eingedampft: 2-Benzo[1,2,5]thiadiazol-5-yl-N-(1,3-dioxo-1,3-dihydro-isoindol-2-yl)-N-[3-(4-fluorphenyl)-3-oxo-propyl]-acetamid als farbloser Feststoff; ESI 489. Dieses Material wird ohne weitere Reinigung für die nächste Reaktionsstufe eingesetzt.
2.3 Eine Lösung von 150 mg (0.37 mmol) 2-Benzo[1,2,5]thiadiazol-5-yl-N-(1,3-dioxo-1,3-dihydro-isoindol-2-yl)-N-[3-(4-fluorphenyl)-3-oxo-propyl]-acetamid in 5 ml Tetrahydrofuran wird mit 20.7 mg Methylhydrazin versetzt und 18 Stunden bei Raumtemperatur gerührt. Das Reaktionsgemisch wird eingedampft und der Rückstand an einer Kieselgelsäule mit Petrolether/Ethylacetat chromatographiert: "A2" als farblose Kristalle; ESI 341.

### Beispiel 3

Die Herstellung von 1-(Benzo[1,2,5]thiadiazol-5-yl-methylcarbonyl)-3-(3,4-dimethoxy-phenyl)-4,5-dihydro-1*H*-pyrazol ("A3") erfolgt analog nachstehendem Schema

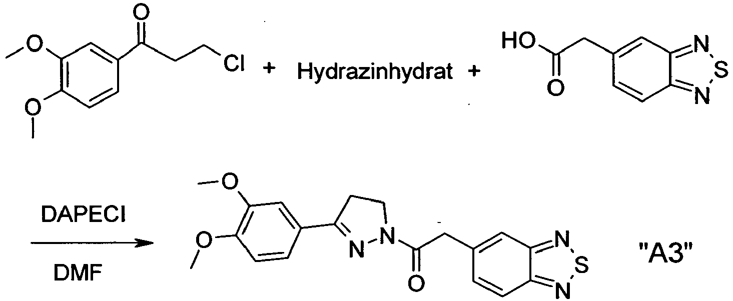

Eine Lösung von 105 mg (2.1 mmol) Hydrazinhydrat in 2 ml DMF wird unter Stickstoff mit 229 mg (1.00 mmol) 3-Chlor-1-(3,4-dimethoxy-phenyl)-propan-1-on versetzt. Das Reaktionsgemisch wird unter Stickstoff 1 Stunde auf 50° C erhitzt. Man lässt auf Raumtemperatur abkühlen, gibt 194 mg (1.00 mmol) Benzo[1,2,5]thiadiazol-5-yl-essigsäure und 249 g (1.3 mmol) DAPECI zu und rührt 18 Stunden bei Raumtemperatur. Das Reaktionsgemisch wird auf Wasser gegeben, der entstandene Niederschlag abfiltriert und mit Wasser gewaschen. Der Rückstand wird durch präparative HPLC gereinigt: "A3" als farblose Kristalle; ESI 383.

### Beispiel 4

Die Herstellung von 1-(3-Methoxy-benzylcarbonyl)-3-(3-chlor-phenyl)-4,5-dihydro-1*H*-pyrazol ("A4"), ESI 329, erfolgt analog nachstehendem Schema (nach M. E. Camacho et al, J. Med. Chem. 2004, 47, 5641-5650)

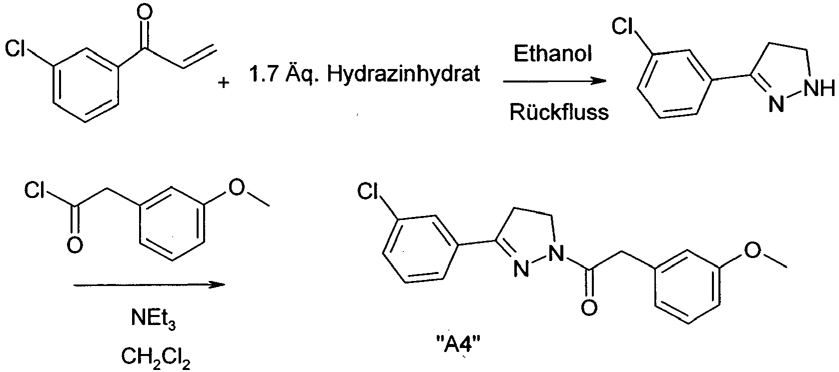

Analog werden die nachstehenden Verbindungen erhalten

| Nr. | Name | ESI | |
|---|---|---|---|
| | Struktur | | |
| "A5" | 1-(3-Methoxy-benzylcarbonyl)-3-(4-chlorphenyl)-4,5-dihydro-1*H*-pyrazol | 329 | |
| | 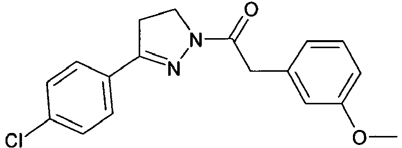 | | |
| "A6" | 1-(4-Methoxy-benzylcarbonyl)-3-(3-chlorphenyl)-4,5-dihydro-1*H*-pyrazol | 329 | |
| | 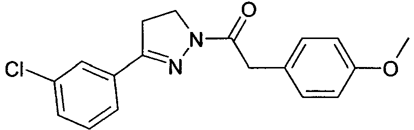 | | |
| "A7" | 1-(Thiophen-2-ylmethylcarbonyl)-3-(3-chlor-phenyl)-4,5-dihydro-1*H*-pyrazol | 305 | |
| | 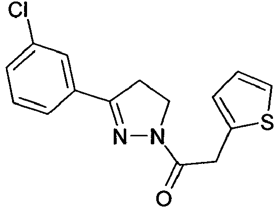 | | |
| "A8" | 1-Benzylcarbonyl-3-(3-chlor-phenyl)-4,5-dihydro-1*H*-pyrazol | 299 | |
| | 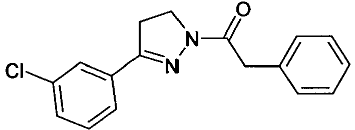 | | |
| "A9" | 1-(4-Fluor-benzylcarbonyl)-3-(4-methoxyphenyl)-4,5-dihydro-1*H*-pyrazol | 313 | |
| | 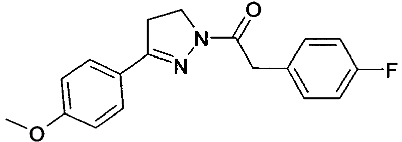 | | |
| "A10" | 1-(3-Methoxy-benzylcarbonyl)-3-(4-methoxyphenyl)-4,5-dihydro-1*H*-pyrazol | 325 | |
| | 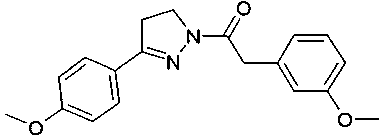 | | |
| "A11" | 1-(4-Methoxy-benzylcarbonyl)-3-(4-methoxy-phenyl)-4,5-dihydro-1*H*-pyrazol | 325 | |
| | 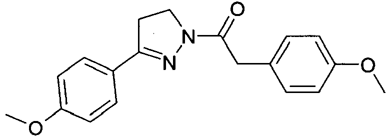 | | |
| "A12" | 1-Benzylcarbonyl-3-(4-methoxy-phenyl)-4,5-dihydro-1*H*-pyrazol | 295 | |
| | 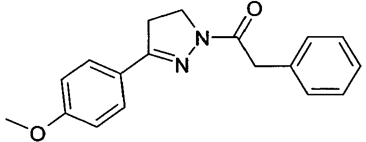 | | |
| "A13" | 1-(4-Fluor-benzylcarbonyl)-3-(2-methoxyphenyl)-4,5-dihydro-1*H*-pyrazol | 313 | |
| | 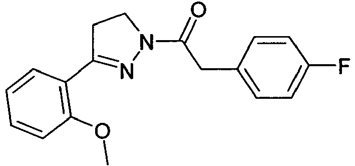 | | |
| "A14" | 1-(3-Methoxy-benzylcarbonyl)-3-(2-methoxyphenyl)-4,5-dihydro-1*H*-pyrazol | 325 | |
| | 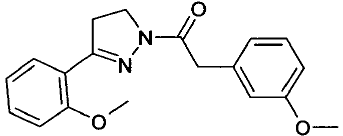 | | |
| "A15" | 1-(4-Methoxy-benzylcarbonyl)-3-(2-methoxyphenyl)-4,5-dihydro-1*H*-pyrazol | 325 | |
| | 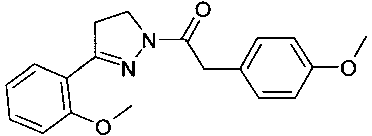 | | |
| "A16" | 1-(Thiophen-2-ylmethylcarbonyl)-3-(2-methoxy-phenyl)-4,5-dihydro-1*H*-pyrazol | 301 | |
| | 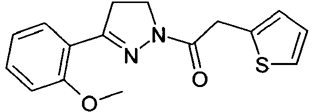 | | |
| "A17" | 1-Benzylcarbonyl-3-(2-methoxy-phenyl)-4,5-dihydro-1*H*-pyrazol 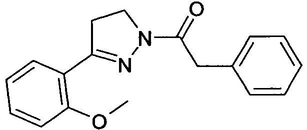 | 295 | |
| "A18" | 1-(3-Methoxy-benzylcarbonyl)-3-(4-dimethylamino-phenyl)-4,5-dihydro-1*H-*pyrazol | 338 | |
| | 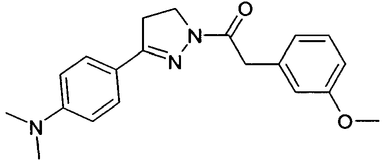 | | |
| "A19" | 1-(4-Methoxy-benzylcarbonyl)-3-(4-dimethylamino-phenyl)-4,5-dihydro-1*H-*pyrazol | 338 | |
| | 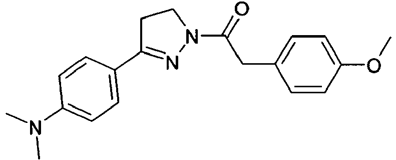 | | |
| "A20" | 1-(Thiophen-2-ylmethylcarbonyl)-3-(4-dimethylamino-phenyl)-4,5-dihydro-1*H-*pyrazol | 314 | |
| | 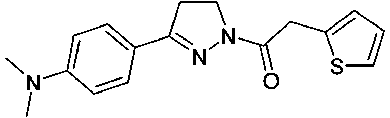 | | |
| "A21" | 1-(4-Fluor-benzylcarbonyl)-3-(4-fluor-phenyl)-4,5-dihydro-1*H*-pyrazol | 301 | |
| | 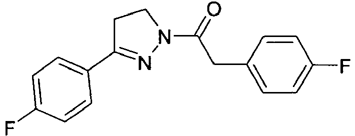 | | |
| "A22" | 1-(4-Methoxy-benzylcarbonyl)-3-(4-fluor-phenyl)-4,5-dihydro-1*H*-pyrazol | 313 | |
| | 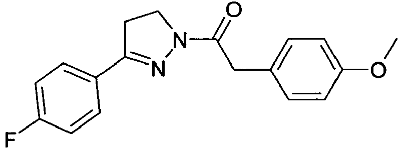 | | |
| "A23" | 1-(Thiophen-2-ylmethylcarbonyl)-3-(4-fluorphenyl)-4,5-dihydro-1*H*-pyrazol | 289 | |
| | 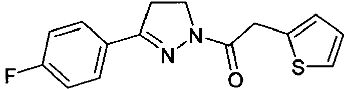 | | |
| "A24" | 1-Benzylcarbonyl-3-(4-fluor-phenyl)-4,5-dihydro-1*H*-pyrazol | 283 | |
| | 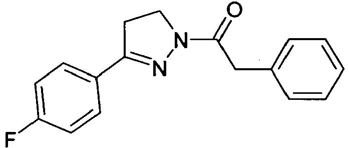 | | |

### Beispiel 5

Die Herstellung von 1-(Benzo[1,2,5]thiadiazol-5-yl-methylcarbonyl)-3-(3,4-dimethoxy-phenyl)-5,5-dimethyl-4,5-dihydro-1*H*-pyrazol ("A25") erfolgt analog nachstehendem Schema

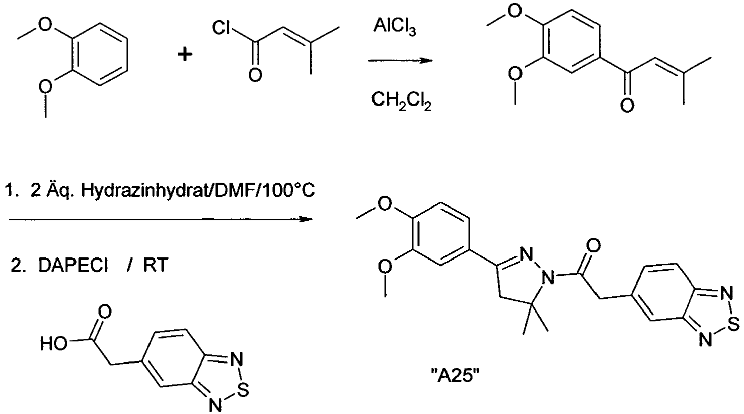

### Beispiel 6

Die Herstellung von 1-(Benzo[1,2,5]thiadiazol-5-yl-methylcarbonyl)-3-(3,4-dimethoxy-phenyl)-4,4-dimethyl-4,5-dihydro-1*H*-pyrazol ("A26") erfolgt analog nachstehendem Schema

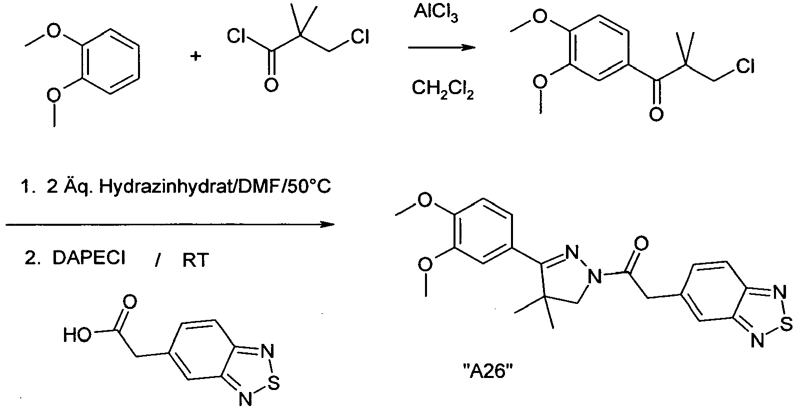

### Beispiel 7

Die Herstellung von 1-[3-(2-Morpholin-4-ylethoxy)-benzylcarbonyl]-3-(5-Chlor-thiophen-2-yl)-4,5-dihydro-1*H*-pyrazol ("A27") erfolgt analog nachstehendem Schema 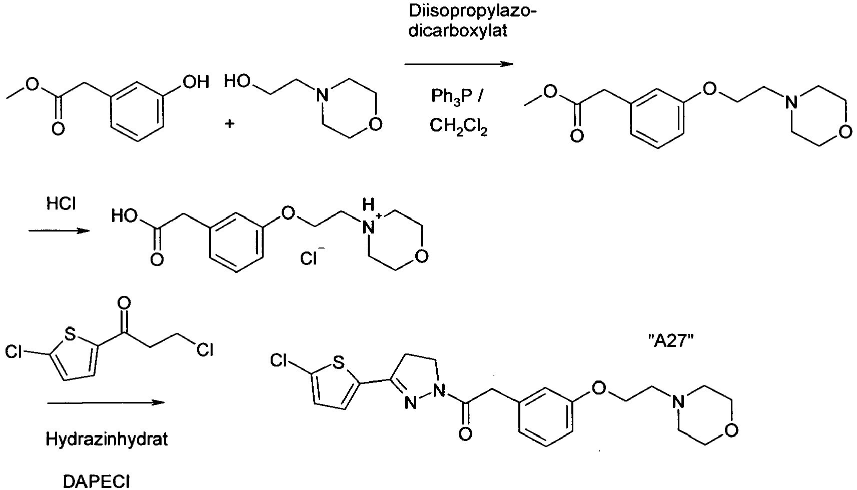
7.1 Zu einer auf -10° C gehaltenen Lösung von 3.94 g (22.8 mmol) 3-Hydroxyphenylessigsäuremethylester, 4.24 ml (34.6 mmol) 2-Morpholinoethanol und 8.90 g (33.9 mmol) Triphenylphosphin in 200 ml Dichlormethan werden unter Rühren 6.77 ml (34.8 mmol) Diisopropylazo-dicarboxylat zugetropft. Das Reaktionsgemisch wird noch eine Stunde bei -10° C, dann 18 Stunden bei Raumtemperatur gerührt. Das Reaktionsgemisch wird dreimal mit 1 N Salzsäure extrahiert. Die vereinigten wässrigen Phasen werden mit 32%iger wässriger Natronlauge alkalisch gestellt und dreimal mit Ethylacetat extrahiert. Die organischen Phasen werden über Natriumsulfat getrocknet und eingedampft: [3-(2-Morpholin-4-yl-ethoxy)-phenyl]-essigsäuremethylester als gelbliches Öl; ESI 280.
7.2 Eine Lösung von 2.38 g (8.00 mmol) [3-(2-Morpholin-4-yl-ethoxy)-phenyl]-essigsäuremethylester in 12 ml 25%iger wässriger HCl wird zwei Tage bei Raumtemperatur belassen und anschließend im Vakuum eingedampft: [3-(2-Morpholin-4-yl-ethoxy)-phenyl]-essigsäure Hydrochlorid als farbloser Feststoff; ESI 266.
7.3 Analog Beispiel 3 erhält man durch Umsetzung von [3-(2-Morpholin-4-yl-ethoxy)-phenyl]-essigsäure Hydrochlorid mit 3-Chlor-1-(5-chlor-thiophen-2-yl)-propan-1-on die Verbindung "A27".

Analog erhält man die nachstehenden Verbindungen

| Nr. | Name | ESI | |
|---|---|---|---|
| | Struktur | | |
| "A28" | 1-[3-(3-Diethylaminopropoxy)-benzylcarbonyl]-3-(5-Chlor-thiophen-2-yl)-4,5-dihydro-1*H-*pyrazol | | |
| | 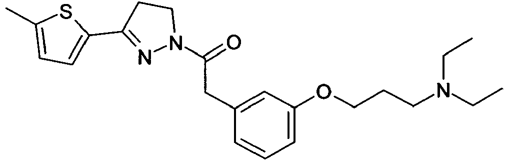 | | |
| "A29" | 1-[3-(3-Diethylaminopropoxy)-benzylcarbonyl]-3-(3,4-dimethoxy-phenyl)-4,5-dihydro-1*H-*pyrazol | | |
| | 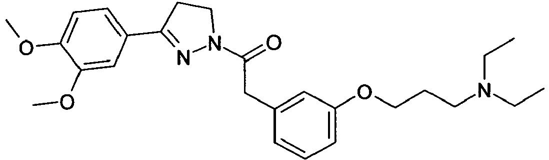 | | |
| "A59" | 1-[4-(Morpholin-4-ylethoxy)-benzylcarbonyl]-3-(3,4-dimethoxy-phenyl)-4,5-dihydro-1*H*-pyrazol 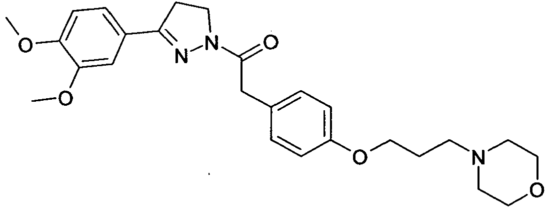 | | |
| "A60" | 1-[4-(3-Diethylaminopropoxy)-benzylcarbonyl]-3-(5-Chlor-thiophen-2-yl)-4,5-dihydro-1*H-*pyrazol | | |
| | 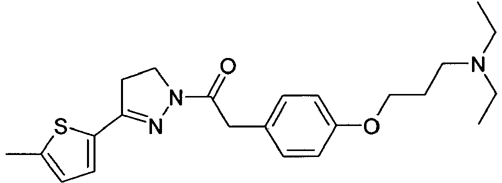 | | |
| "A71" | 1-[4-(Morpholin-4-ylethoxy)-benzylcarbonyl]-3-(5-Chlor-thiophen-2-yl)-4,5-dihydro-1*H*-pyrazol | | |
| | 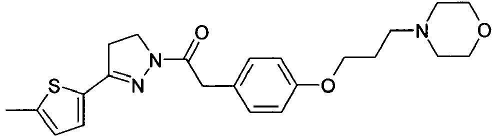 | | |

### Beispiel 7a

Die Verbindung 1-(Benzo[1,2,5]thiadiazol-5-yl-methylcarbonyl)-3-(5-chlor-furan-2-yl)-4,5-dihydro-1*H*-pyrazol ("A75") wird analog nachstehendem Schema erhalten

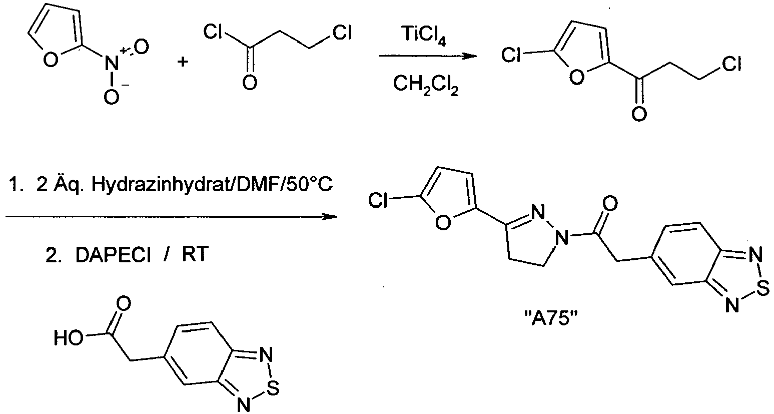

### Beispiel 8

Analog Beispiel 3 oder 7a und werden die nachstehenden Verbindungen erhalten

| Nr. | Name Struktur | ESI | |
|---|---|---|---|
| "A30" | 1-(3-Hydroxy-benzylcarbonyl)-3-(2,4-dimethoxy-phenyl)-4,5-dihydro-1*H*-pyrazol | 341 | |
| | 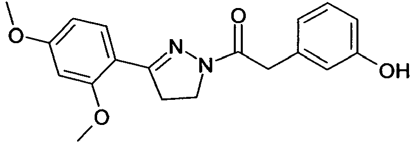 | | |
| "A31" | 1-(3-Hydroxy-benzylcarbonyl)-3-(4-fluor-phenyl)-4,5-dihydro-1*H*-pyrazol | 299 | |
| | 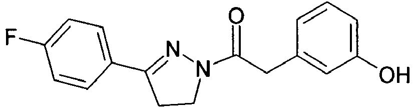 | | |
| "A32" | 1-(Benzo[1,2,5]thiadiazol-5-yl-methylcarbonyl)-3-phenyl-4,5-dihydro-1*H*-pyrazol | 323 | |
| | 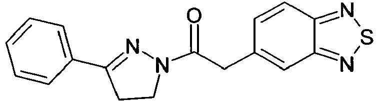 | | |
| "A33" | 1-(Benzo[1,2,5]thiadiazol-5-yl-methylcarbonyl)-3-(4-chlor-phenyl)-4,5-dihydro-1*H*-pyrazol | 357 | |
| | 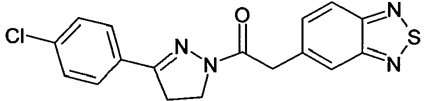 | | |
| "A34" | 1-(Benzo[1,2,5]thiadiazol-5-yl-methylcarbonyl)-3-(5-chlor-thiophen-2-yl)-4,5-dihydro-1*H*-pyrazol | 363 | |
| | 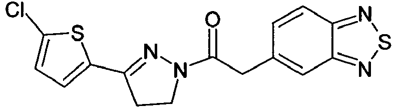 | | |
| "A35" | 1-(Benzo[1,2,5]thiadiazol-5-yl-methylcarbonyl)-3-(4-brom-phenyl)-4,5-dihydro-1*H*-pyrazol | 401,4 | |
| | 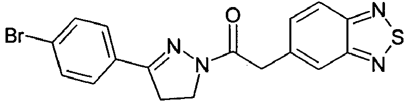 | | |
| "A36" | 1-(3-Hydroxy-benzylcarbonyl)-3-(4-chlorphenyl)-4,5-dihydro-1*H*-pyrazol | 315 | |
| | 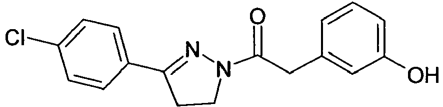 | | |
| "A37" | 1-(Benzo[1,2,5]thiadiazol-5-yl-methylcarbonyl)-3-(4-methylsulfonamido-phenyl)-4,5-dihydro-1*H-*pyrazol | 416 | |
| | 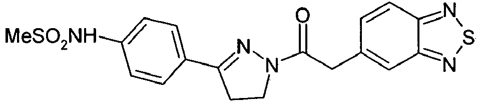 | | |
| "A38" | 1-(3-Hydroxy-benzylcarbonyl)-3-(3,4-dihydro-2-oxo-1*H*-chinazolin-6-yl)-4,5-dihydro-1*H*-pyrazol | | |
| | 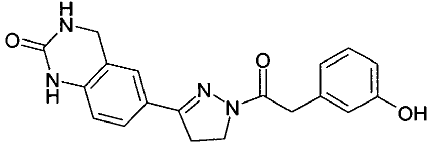 | | |
| "A39" | 1-(Benzo[1,2,5]thiadiazol-5-yl-methylcarbonyl)-3-(3,4-dihydro-2-oxo-1*H*-chinazolin-6-yl)-4,5-dihydro-1*H*-pyrazol | | |
| | 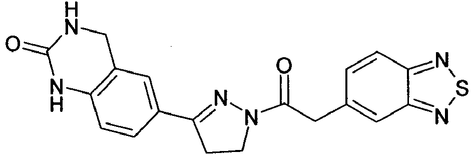 | | |
| "A40" | 1-(Benzo[1,2,5]thiadiazol-5-yl-methylcarbonyl)-3-(4-hydroxy-phenyl)-4,5-dihydro-1*H*-pyrazol | | |
| | 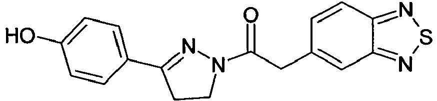 | | |
| "A41" | 1-(Benzo[1,2,5]thiadiazol-5-yl-methylcarbonyl)-3-(2-hydroxy-phenyl)-4,5-dihydro-1*H*-pyrazol | | |
| | 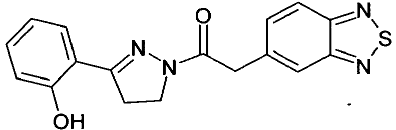 | | |
| "A42" | 1-(Benzo[1,2,5]thiadiazol-5-yl-methylcarbonyl)-3-(2-hydroxy-5-chlor-phenyl)-4,5-dihydro-1*H-*pyrazol | | |
| | 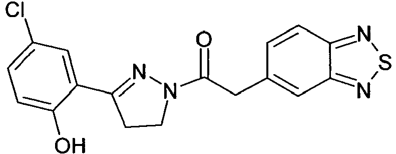 | | |
| "A43" | 1-(Benzo[1,2,5]thiadiazol-5-yl-methylcarbonyl)-3-(3-chlor-6-methoxy-phenyl)-4,5-dihydro-1*H*-pyrazol | | |
| | 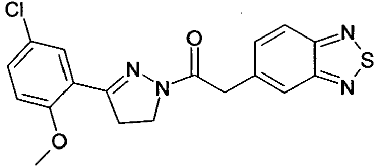 | | |
| "A44" | 1-(Benzo[1,2,5]thiadiazol-5-yl-methylcarbonyl)-3-(3-chlor-4-methoxy-phenyl)-4,5-dihydro-1*H-*pyrazol | | |
| | 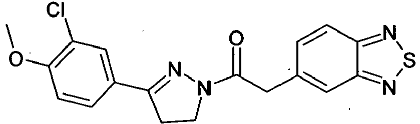 | | |
| "A45" | 1-(3-Hydroxy-benzylcarbonyl)-3-(5-chlor-thiophen-2-yl)-4,5-dihydro-1*H*-pyrazol | | |
| | 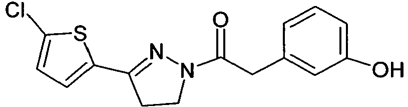 | | |
| "A46" | 1-(3-Methoxy-benzylcarbonyl)-3-(5-chlor-thiophen-2-yl)-4,5-dihydro-1*H*-pyrazol | | |
| | 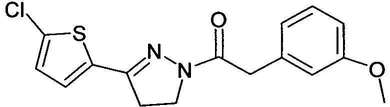 | | |
| "A47" | 1-[3-(3-oxo-morpholin-4-yl)-benzylcarbonyl]-3-(3,4-dimethoxy-phenyl)-4,5-dihydro-1*H*-pyrazol | | |
| | 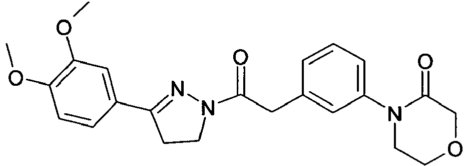 | | |
| "A48" | 1-(Phenethyl-carbonyl)-3-(3,4-dimethoxy-phenyl)-4,5-dihydro-1*H*-pyrazol | | |
| | 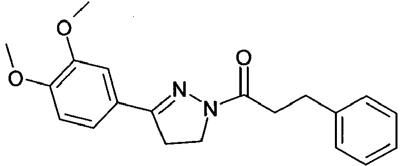 | | |
| "A49" | 1-[4-(Morpholin-4-ylcarbonyl)-phenyl-methylcarbonyl]-3-(3,4-dimethoxy-phenyl)-4,5-dihydro-1*H*-pyrazol | | |
| | 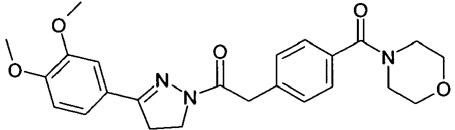 | | |
| "A50" | 1-[3-(3-Diethylamino-propoxycarbonylamino)-phenyl-methylcarbonyl]-3-(3,4-dimethoxy-phenyl)-4,5-dihydro-1*H*-pyrazol | | |
| | 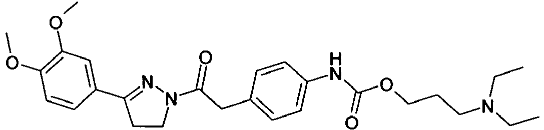 | | |
| "A51" | 1-{3-[3-(3-Diethylamino-propyl)-ureido]-phenyl-methylcarbonyl}-3-(3,4-dimethoxy-phenyl)-4,5-dihydro-1*H*-pyrazol | | |
| | 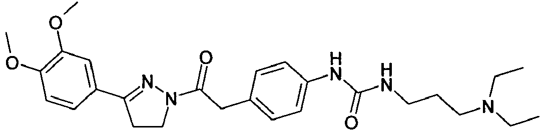 | | |
| "A52" | 1-Benzylcarbonyl-3-(2,4-dimethoxy-phenyl)-4,5-dihydro-1*H*-pyrazol | 325 | |
| | 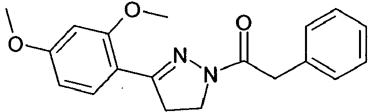 | | |
| "A53" | 1-(3-Methoxy-benzylcarbonyl)-3-(2,4-dimethoxyphenyl)-4,5-dihydro-1*H*-pyrazol | 355 | |
| | 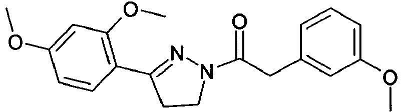 | | |
| "A54" | 1-(3-Methoxy-benzylcarbonyl)-3-(4-fluor-phenyl)-4,5-dihydro-1*H*-pyrazol | 313 | |
| | 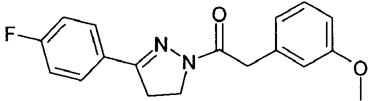 | | |
| "A55" | 1-(3-Methoxy-benzylcarbonyl)-3-(2,5-dimethoxyphenyl)-4,5-dihydro-1*H*-pyrazol | 355 | |
| | 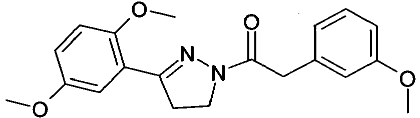 | | |
| "A56" | 1-(Benzo[1,2,5]thiadiazol-5-yl-methylcarbonyl)-3-(4-methylsulfanil-phenyl)-4,5-dihydro-1*H-*pyrazol | 369 | |
| | 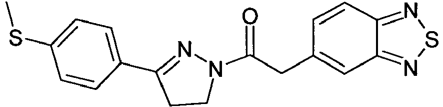 | | |
| "A57" | 1-(Benzo[1,2,5]thiadiazol-5-yl-methylcarbonyl)-3-(dibenzofuran-2-yl)-4,5-dihydro-1*H*-pyrazol | 413 | |
| | 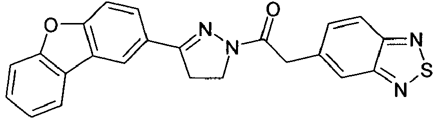 | | |
| "A58" | 1-(4-Hydroxy-benzylcarbonyl)-3-(4-chlorphenyl)-4,5-dihydro-1*H*-pyrazol | 315 | |
| | 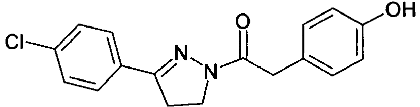 | | |
| "A61" | 1-(4-Hydroxy-benzylcarbonyl)-3-(2,4-dimethoxyphenyl)-4,5-dihydro-1*H*-pyrazol | 341 | |
| | 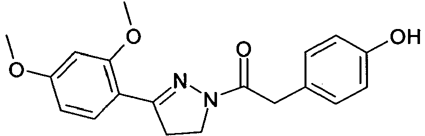 | | |
| "A62" | 1-(3-Hydroxy-benzylcarbonyl)-3-(3,4-dimethoxyphenyl)-4,5-dihydro-1*H*-pyrazol | 341 | |
| | 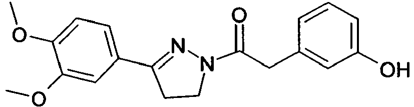 | | |
| "A63" | 1-(3-Chlor-benzylcarbonyl)-3-(2,4-dimethoxy-phenyl)-4,5-dihydro-1*H*-pyrazol | 359 | |
| | 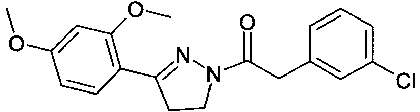 | | |
| "A64" | 1-(2-Benzo[1,2,5]thiadiazol-5-yl-propionyl)-3-(4-fluor-phenyl)-4,5-dihydro-1*H*-pyrazol | 355 | |
| | 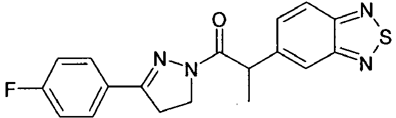 | | |
| "A65" | 1-[2-Hydroxy-2-(3-hydroxy-phenyl)-acetyl]-3-(4-fluor-phenyl)-4,5-dihydro-1*H*-pyrazol | 315 | |
| | 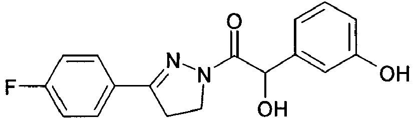 | | |
| "A66" | 1-[2-Hydroxy-2-(3-hydroxy-phenyl)-acetyl]-3-(4-chlor-phenyl)-4,5-dihydro-1*H*-pyrazol | 331 | |
| | 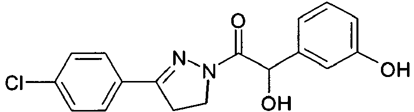 | | |
| "A67" | 1-(2-Hydroxy-benzylcarbonyl)-3-(2,4-dimethoxyphenyl)-4,5-dihydro-1*H*-pyrazol | 341 | |
| | 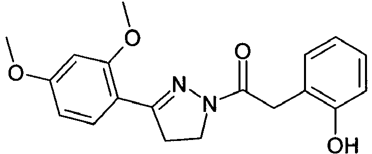 | | |
| "A68" | 1-(3-Hydroxy-benzylcarbonyl)-3-(2-oxo-1,3-dihydro-indol-5-yl)-4,5-dihydro-1*H*-pyrazol | 336 | |
| | 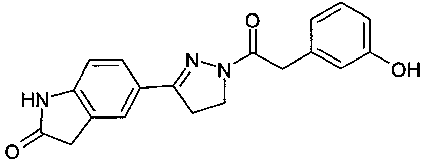 | | |
| "A69" | 1-(4-Hydroxy-benzylcarbonyl)-3-(4-fluor-phenyl)-4,5-dihydro-1*H*-pyrazol | 299 | |
| | 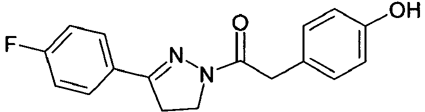 | | |
| "A70" | 1-(2-Hydroxy-benzylcarbonyl)-3-(4-fluor-phenyl)-4,5-dihydro-1*H*-pyrazol | 299 | |
| | 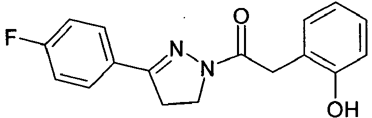 | | |
| "A72" | 1-(4-Fluor-benzylcarbonyl)-3-(5-chlor-thiophen-2-yl)-4,5-dihydro-1*H*-pyrazol | 323 | |
| | 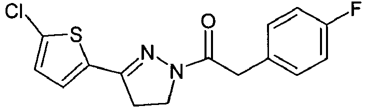 | | |
| "A73" | 1-(4-Methoxy-benzylcarbonyl)-3-(5-chlor-thiophen-2-yl)-4,5-dihydro-1*H*-pyrazol | | |
| | 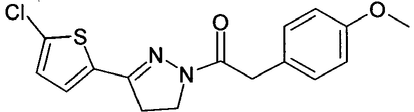 | | |
| "A74" | 1-(4-Acetamido-benzylcarbonyl)-3-(5-chlor-thiophen-2-yl)-4,5-dihydro-1*H*-pyrazol | 362 | |
| | 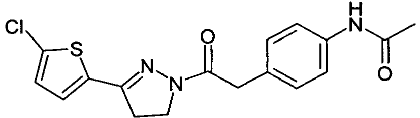 | | |
| "A76" | 1-(4-Hydroxy-3-methoxy-benzylcarbonyl)-3-(5-chlor-thiophen-2-yl)-4,5-dihydro-1*H*-pyrazol | | |
| | 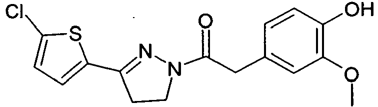 | | |
| "A77" | 1-(4-Methylsulfonyl-benzylcarbonyl)-3-(5-chlor-thiophen-2-yl)-4,5-dihydro-1*H*-pyrazol | | |
| | 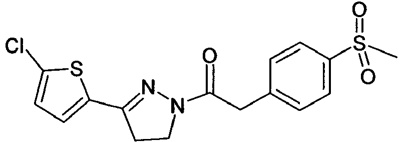 | | |
| "A78" | 1-(4-Ethoxy-benzylcarbonyl)-3-(5-chlor-thiophen-2-yl)-4,5-dihydro-1*H*-pyrazol | | |
| | 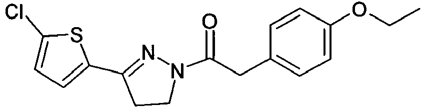 | | |
| "A79" | 1-(4-Hydroxy-3-chlor-benzylcarbonyl)-3-(5-chlor-thiophen-2-yl)-4,5-dihydro-1*H*-pyrazol | | |
| | 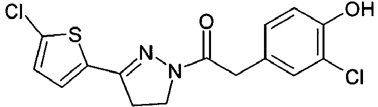 | | |
| "A80" | 1-(2-(4-Hydroxy-phenyl)-propionyl)-3-(5-chlor-thiophen-2-yl)-4,5-dihydro-1*H*-pyrazol | | |
| | 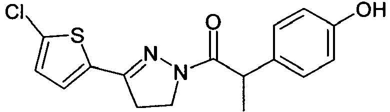 | | |
| "A81" | 1-(2-(Benzo[1,2,5]thiadiazol-5-yl)-propionyl)-3-(5-chlor-thiophen-2-yl)-4,5-dihydro-1*H*-pyrazol | | |
| | 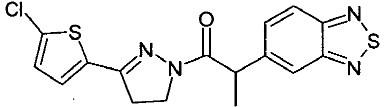 | | |
| "A82" | 1-(2-(4-Fluor-phenyl)-propionyl)-3-(5-chlor-thiophen-2-yl)-4,5-dihydro-1*H*-pyrazol | | |
| | 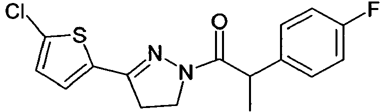 | | |
| "A84" | 1-(Benzo[1,2,5]thiadiazol-5-yl-methylcarbonyl)-3-(2-hydroxy-phenyl)-4,5-dihydro-1*H*-pyrazol | 339 | |
| | 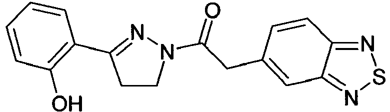 | | |
| "A85" | 1-(3-Chlor-benzylcarbonyl)-3-(3,4-dimethoxyphenyl)-4,5-dihydro-1*H*-pyrazol | 359 | |
| | 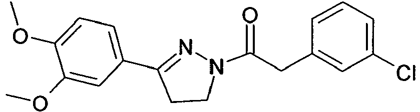 | | |
| "A86" | 1-(Benzo[1,2,5]thiadiazol-5-yl-methylcarbonyl)-3-(2-oxo-3,4-dihydro-1*H*-chinazolin-6-yl)-4,5-dihydro-1*H*-pyrazol | 393 | |
| | 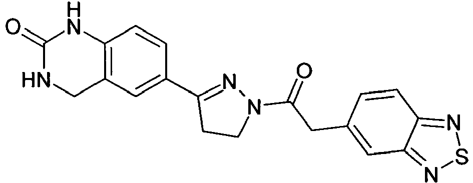 | | |
| "A87" | 1-(Benzo[1,2,5]thiadiazol-5-yl-methylcarbonyl)-3-(4-hydroxy-phenyl)-4,5-dihydro-1*H*-pyrazol | 339 | |
| | 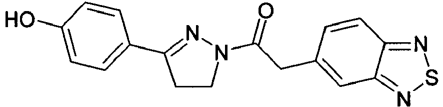 | | |
| "A88" | 1-(3-Hydroxy-benzylcarbonyl)-3-(2-oxo-3,4-dihydro-1*H*-chinazolin-6-yl)-4,5-dihydro-1*H-*pyrazol | 351 | |
| | 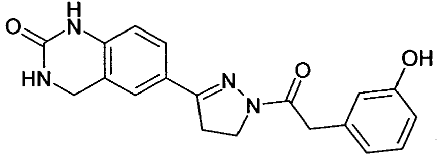 | | |
| "A89" | 1-(Benzo[1,2,5]thiadiazol-5-yl-methylcarbonyl)-3-(3-chlor-6-hydroxy-phenyl)-4,5-dihydro-1*H-*pyrazol | 373 | |
| | 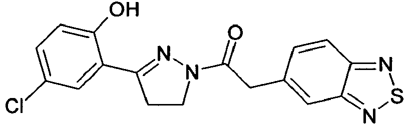 | | |
| "A90" | 1-(3-Hydroxy-benzylcarbonyl)-3-(5-chlor-thiophen-2-yl)-4,5-dihydro-1*H*-pyrazol | 321 | |
| | 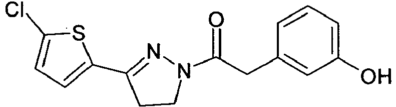 | | |
| "A91" | 1-(3-Hydroxy-benzylcarbonyl)-3-(4-hydroxyphenyl)-4,5-dihydro-1*H*-pyrazol | 297 | |
| | 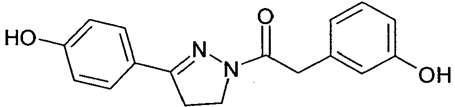 | | |
| "A92" | 1-(Benzo[1,3]dioxol-5-yl-methylcarbonyl)-3-(5-chlor-thiophen-2-yl)-4,5-dihydro-1*H*-pyrazol | 349 | |
| | 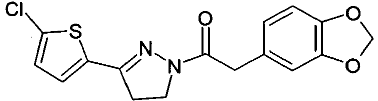 | | |
| "A93" | 1-(4-Hydroxy-benzylcarbonyl)-3-(5-chlor-thiophen-2-yl)-4,5-dihydro-1*H*-pyrazol | 321 | |
| | 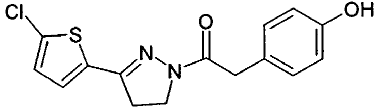 | | |
| "A94" | 1-(4-Methoxy-benzylcarbonyl)-3-(5-chlor-thiophen-2-yl)-4,5-dihydro-1*H*-pyrazol | 335 | |
| | 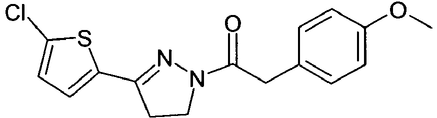 | | |
| "A95" | 1-(Benzo[1,2,5]thiadiazol-5-yl-methylcarbonyl)-3-(3-chlor-6-methoxy-phenyl)-4,5-dihydro-1*H-*pyrazol | 387 | |
| "A96" | 1-(4-Hydroxy-benzylcarbonyl)-3-(3,4-dimethoxyphenyl)-4,5-dihydro-1*H*-pyrazol | 341 | |
| "A97" | 1-(3-Chlor-4-hydroxy-benzylcarbonyl)-3-(5-chlor-thiophen-2-yl)-4,5-dihydro-1*H*-pyrazol | 355 | |
| "A98" | 3-(5-Chlor-thiophen-2-yl)-1-[2-(4-hydroxyphenyl)-propionyl]-4,5-dihydro-1*H*-pyrazol | 335 | |
| | 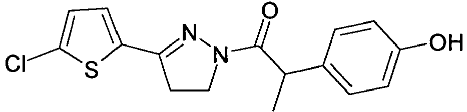 | | |
| "A99" | 1-(4-Methoxy-benzylcarbonyl)-3-(3,4-dimethoxyphenyl)-4,5-dihydro-1*H*-pyrazol | 355 | |
| "A100" | 3-(2-Oxo-3,4-dihydro-1*H*-chinazolin-6-yl)-1-[2-(4-hydroxy-phenyl)-propionyl]-4,5-dihydro-1*H-*pyrazol | 365 | |
| | 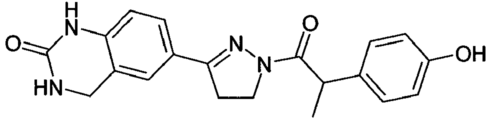 | | |
| "A101" | 1-[3-(3-Oxo-morpholin-4-yl)-benzylcarbonyl]-3-(5-chlor-thiophen-2-yl)-4,5-dihydro-1*H*-pyrazol | 404 | |
| | 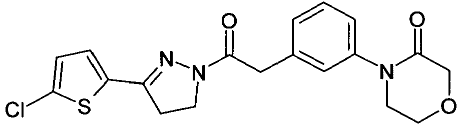 | | |
| "A102" | 1-[3-(3-Oxo-morpholin-4-yl)-benzylcarbonyl]-3-(3,4-dimethoxy-phenyl)-4,5-dihydro-1*H*-pyrazol | 424 | |
| "A103" | 3-(3-Chlor-6-hydroxy-phenyl)-1-[2-(benzo[1,2,5]-thiadiazol-5-yl)-propionyl]-4,5-dihydro-1*H-*pyrazol | 387 | |
| | 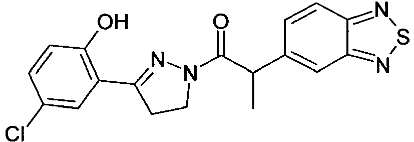 | | |
| "A104" | 1-[4-(3-Morpholin-4-yl-propoxy)-benzylcarbonyl]-3-(5-chlor-thiophen-2-yl)-4,5-dihydro-1*H*-pyrazol | 448 | |
| | 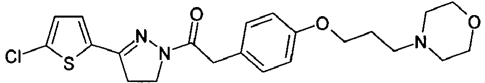 | | |
| "A105" | 1-[2-(1*H*-Benzimidazol-5-yl)-acetyl]-3-(5-chlor-thiophen-2-yl)-4,5-dihydro-1*H*-pyrazol | 345 | |
| | 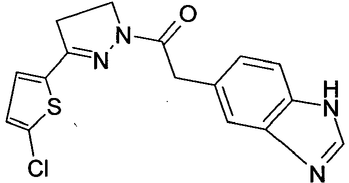 | | |
| "A106" | 3-(4-Chlor-phenyl)1-(3-phenyl-propionyl)-4,5-dihydro-1*H*-pyrazol | 313 | |
| | 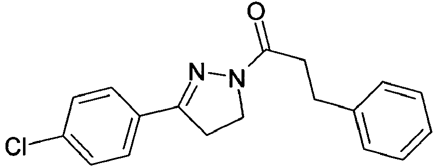 | | |
| "A107" | 1-(4-Methylsulfonyl-benzylcarbonyl)-3-(4-chlorphenyl)-4,5-dihydro-1*H*-pyrazol | 377 | |
| "A108" | 1-[2-(1*H*-Benzimidazo)-5-yl)-acetyl]-3-(3-chlor-6-hydroxy-phenyl)-4,5-dihydro-1*H*-pyrazol | 355 | |
| "A109" | 1-[3-(2-Morpholin-4-yl-ethoxy)-benzylcarbonyl]-3-(5-chlor-thiophen-2-yl)-4,5-dihydro-1*H*-pyrazol | 434 | |
| "A110" | 1-(Benzo[1,3]dioxol-5-yl-methylcarbonyl)-3-(3-chlor-6-hydroxy-phenyl)-4,5-dihydro-1*H*-pyrazol | 359 | |
| "A111" | 3-(4-Chlor-phenyl)1-[2-(4-hydroxy-phenyl)-propionyl]-4,5-dihydro-1*H*-pyrazol | 329 | |
| "A112" | 1-[2-(1*H*-Benzimidazol-5-yl)-acetyl]-3-(4-chlorphenyl)-4,5-dihydro-1*H*-pyrazol | 339 | |
| "A113" | 1-(3-Chlor-4-hydroxy-benzylcarbonyl)-3-(3-chlor-6-hydroxy-phenyl)-4,5-dihydro-1*H*-pyrazol | 365 | |
| "A114" | 1-(4-Hydroxy-benzylcarbonyl)-3-(3-chlor-6-methoxy-phenyl)-4,5-dihydro-1*H*-pyrazol | 345 | |
| "A115" | 1-(Benzo[1,3]dioxol-5-yl-methylcarbonyl)-3-(2-oxo-3,4-dihydro-1*H*-chinazolin-6-yl)-4,5-dihydro-1*H*-pyrazol | 411 | |
| "A116" | 3-(3,4-Dimethoxy-phenyl)-1-[2-(benzo[1,2,5]-thiadiazol-5-yl)-acetyl]-5,5-dimethyl-4,5-dihydro-1*H*-pyrazol | 411 | |
| "A117" | 1-{4-[3-(N,N-Diethoxy-amino)-propoxy]-benzylcarbonyl}-3-(4-chlor-phenyl)-4,5-dihydro 1*H*-pyrazol | 428 | |
| "A118" | 3-(5-Chlor-thiophen-2-yl)-1-[2-(chinoxalin-6-yl)-acetyl]-4,5-dihydro-1*H*-pyrazol | 357 | |
| "A119" | 3-(3-Chlor-6-hydroxy-phenyl)-1-[2-(chinoxalin-6-yl)-acetyl]-4,5-dihydro-1*H*-pyrazol | | |
| "A120" | 3-(3-Chlor-6-hydroxy-phenyl)-1-[2-(4-hydroxy-phenyl)-propionyl]-4,5-dihydro-1*H*-pyrazol | 345 | |
| "A121" | 1-(4-Hydroxy-benzylcarbonyl)-3-(3-chlor-6-hydroxy-phenyl)-4,5-dihydro-1*H*-pyrazol | 331 | |
| ¹H-NMR (d⁶-DMSO): δ = 3.38 (t, J = 10 Hz, 2H), 3.84 (s, 2H) 3.87 (t, J = 10 Hz, 2H), 6.68 (d, J = 8 Hz, 2H), 6.99 (d, J = 8.8 Hz, 1H), 7.07 (d, J = 8 Hz, 2H), 7.36 (dd, J₁ = 8.8 Hz, J₂ = 2.5 Hz, 1H), 7.58 (d, J = 2.5 Hz, 1H), 9.2 (bs, 1H), 10.3 (bs, 1H). | | | |
| "A122" | 1-(3-Chlor-benzylcarbonyl)-3-(3-chlor-6-hydroxyphenyl)-4,5-dihydro-1*H*-pyrazol | 349 | |
| "A123" | 1-(2-{4-[3-(N,N-Diethoxy-amino)-propoxy]-phenyl}-propionyl)-3-(4-chlor-phenyl)-4,5-dihydro-1*H*-pyrazol | 442 | |
| | 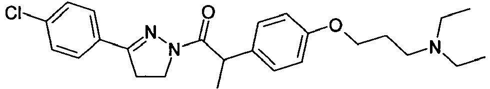 | | |
| "A124" | 1-[4-(Morpholin-4-ylcarbonyl)-benzylcarbonyl]-3-(5-chlor-thiophen-2-yl)-4,5-dihydro-1*H*-pyrazol | 418 | |
| "A125" | 1-[4-(1-Methyl-piperazin-4-ylcarbonyl)-benzylcarbonyl]-3-(5-chlor-thiophen-2-yl)-4,5-dihydro-1*H*-pyrazol | 431 | |
| "A126" | 1-[2-(Pyridin-3-yl)-acetyl]-3-(3-chlor-6-hydroxy-phenyl)-4,5-dihydro-1*H*-pyrazol | 316 | |
| "A127" | 1-[2-(Pyridin-2-yl)-acetyl]-3-(3-chlor-6-hydroxyphenyl)-4,5-dihydro-1*H*-pyrazol | 316 | |
| "A128" | 1-(4-Cyan-benzylcarbonyl)-3-(5-chlor-thiophen-2-yl)-4,5-dihydro-1*H*-pyrazol | 330 | |
| "A129" | 1-[2-(Pyridin-3-yl)-acetyl]-3-(5-chlor-thiophen-2-yl)-4,5-dihydro-1*H*-pyrazol | 306 | |
| "A130" | 1-[2-(Pyridin-2-yl)-acetyl]-3-(5-chlor-thiophen-2-yl)-4,5-dihydro-1*H*-pyrazol | 306 | |
| "A132" | 1-(9*H*-Carbazol-3-yl-methylcarbonyl)-3-(3-chlor-6-hydroxy-phenyl)-4,5-dihydro-1*H*-pyrazol | 404 | |
| "A134" | 1-{2-[2-(5-Chlor-thiophen-2-yl)-(1*H-*benzimidazol-5-yl]-acetyl}-3-(5-chlor-thiophen-2-yl)-4,5-dihydro-1*H*-pyrazol | 461 | |
| | 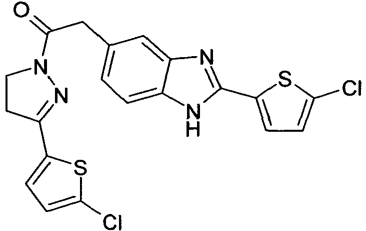 | | |
| "A135" | 1-{2-[2-(5-Chlor-thiophen-2-yl)-(1*H-*benzimidazol-5-yl]-acetyl}-3-(3-chlor-6-hydroxy-phenyl)-4,5-dihydro-1*H*-pyrazol | 471 | |
| "A136" | 3-(3-Chlor-6-hydroxy-phenyl)-1-[2-(2-aminobenzothiazol-6-yl)-propionyl]-4,5-dihydro-1*H-*pyrazol | 401 | |
| | 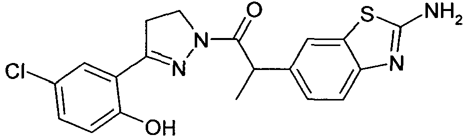 | | |
| "A137" | 3-(4-Fluor-phenyl)-1-[2-(2-amino-benzothiazol-6-yl)-propionyl]-4,5-dihydro-1*H*-pyrazol | 369 | |
| "A138" | 3-(4-Fluor-phenyl)-1-[2-(2-amino-benzimidazol-6-yl)-acetyl]-4,5-dihydro-1*H*-pyrazol | 338 | |
| "A139" | 1-(3-Nitro-benzylcarbonyl)-3-(3-chlor-6-hydroxy)-4,5-dihydro-1*H*-pyrazol | 360 | |
| "A140" | 3-(2,5-Dichlor-phenyl)-1-[2-(benzo[1,2,5]-thiadiazol-5-yl)-acetyl]-4,5-dihydro-1*H*-pyrazol | 392 | |
| "A141" | 3-(2,5-Dichlor-phenyl)-1-[2-(1*H*-benzimidazol-5-yl)-acetyl]-4,5-dihydro-1*H*-pyrazol | 374 | |
| ¹H-NMR (d⁶-DMSO): δ = 3.97 (t, J = 10 Hz, 2H), 3.92 (t, J = 10 Hz, 2H), 4.09 (s, 2H), 7.14 (dd, J₁ = 8.5 Hz, J₂ = 1.0 Hz, 1H), 7.50 (m, 2H), 7.55 (dd, J₁ = 8.5 Hz, J₂ = 2.0 Hz, 1H), 7.62 (d, J = 8.5 Hz, 1H), 7.78 (d, J = 2.0 Hz, 1H), 8.15 (s, 1H), 12.3 (bs, 1H). | | | |
| "A142" | 3-(4-Fluor-phenyl)-1-[2-(1*H*-benzotriazol-5-yl)-acetyl]-4,5-dihydro-1*H*-pyrazol | 324 | |
| "A143" | 3-(3-Chlor-6-hydroxy-phenyl)-1-[2-(1*H-*benzotriazol-5-yl)-acetyl]-4,5-dihydro-1*H*-pyrazol | 356 | |
| | | | |
| "A145" | 3-(2,5-Difluor-phenyl)-1-[2-(1*H*-benzimidazol-5-yl)-acetyl]-4,5-dihydro-1*H*-pyrazol | 341 | |
| "A146" | 3-(2,5-Difluor-phenyl)-1-[2-(4,5-dihydro-3-oxo-2H-pyridazin-6-yl)-acetyl]-4,5-dihydro-1*H-*pyrazol | 335 | |
| | 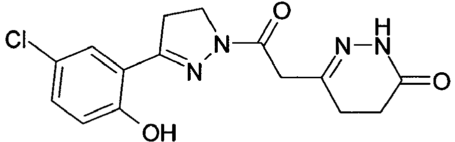 | | |
| "A149" | 1-(4-Hydroxy-benzylcarbonyl)-3-phenyl-4,5-dihydro-1*H*-pyrazol | 281 | |
| "A157" | 1-(4-Fluor-benzylcarbonyl)-3-(4-chlor-phenyl)-4,5-dihydro-1*H*-pyrazol | 317 | |
| "A158" | 3-(4-Chlor-phenyl)-1-[2-(thiophen-2-yl)-acetyl]-4,5-dihydro-1*H*-pyrazol | 305 | |
| "A185" | 1-(4-Fluor-benzylcarbonyl)-3-(4-methyl-phenyl)-4,5-dihydro-1*H*-pyrazol | 297 | |
| "A186" | 1-(3-Methoxy-benzylcarbonyl)-3-(4-methylphenyl)-4,5-dihydro-1*H*-pyrazol | 309 | |
| "A187" | 1-Benzylcarbonyl-3-(4-methyl-phenyl)-4,5-dihydro-1*H*-pyrazol | 279 | |
| "A188" | 1-(4-Fluor-benzylcarbonyl)-3-(3,4-dimethoxyphenyl)-4,5-dihydro-1*H*-pyrazol | 343 | |
| "A189" | 1-(3-Methoxy-benzylcarbonyl)-3-(3,4-dimethoxyphenyl)-4,5-dihydro-1*H*-pyrazol | 355 | |
| "A190" | 1-Benzylcarbonyl-3-(3,4-dimethoxy-phenyl)-4,5-dihydro-1*H*-pyrazol | 325 | |
| "A191" | 1-(4-Fluor-benzylcarbonyl)-3-(benzo[1,3]dioxol-5-yl)-4,5-dihydro-1*H*-pyrazol | 327 | |
| "A192" | 1-(3-Methoxy-benzylcarbonyl)-3-(benzo[1,3]dioxol-5-yl)-4,5-dihydro-1*H*-pyrazol- | 339 | |
| "A193" | 1-(4-Methoxy-benzylcarbonyl)-3-(benzo[1,3]dioxol-5-yl)-4,5-dihydro-1*H*-pyrazol | 339 | |
| "A194" | 1-[2-(Thiophen-2-yl)-acetyl]-3-(benzo[1,3]dioxol-5-yl)-4,5-dihydro-1*H*-pyrazol | 315 | |
| "A195" | 1-Benzylcarbonyl-3-(benzo[1,3]dioxol-5-yl)-4,5-dihydro-1*H*-pyrazol | 309 | |
| "A196" | 1-(4-Fluor-benzylcarbonyl)-3-(4-ethoxy-phenyl)-4,5-dihydro-1*H*-pyrazol | 327 | |
| "A197" | 1-(3-Methoxy-benzylcarbonyl)-3-(4-ethoxyphenyl)-4,5-dihydro-1*H*-pyrazol | 339 | |
| "A198" | 1-(4-Methoxy-benzylcarbonyl)-3-(4-ethoxyphenyl)-4,5-dihydro-1*H*-pyrazol | 339 | |
| "A199" | 1-Benzylcarbonyl-3-(4-ethoxy-phenyl)-4,5-dihydro-1*H*-pyrazol | 309 | |
| "A200" | 1-(4-Fluor-benzylcarbonyl)-3-(thiophen-2-yl)-4,5-dihydro-1*H*-pyrazol | 289 | |
| "A201" | 1-(4-Methoxy-benzylcarbonyl)-3-(thiophen-2-yl)-4,5-dihydro-1*H*-pyrazol | 301 | |
| "A202" | 1-[2-(Thiophen-2-yl)-acetyl]-3-(thiophen-2-yl)-4,5-dihydro-1*H*-pyrazol | 277 | |
| "A203" | 1-Benzylcarbonyl-3-(thiophen-2-yl)-4,5-dihydro-1*H*-pyrazol | 271 | |
| "A204" | 1-(4-Fluor-benzylcarbonyl)-3-[4-(morpholin-4-yl)-phenyl]-4,5-dihydro-1*H*-pyrazol | 368 | |
| "A205" | 1-(3-Methoxy-benzylcarbonyl)-3-[4-(morpholin-4-yl)-phenyl]-4,5-dihydro-1*H*-pyrazol | 380 | |
| "A206" | 1-(4-Methoxy-benzylcarbonyl)-3-[4-(morpholin-4-yl)-phenyl]-4,5-dihydro-1*H*-pyrazol | 380 | |
| "A207" | 1-[2-(Thiophen-2-yl)-acetyl]-3-[4-(morpholin-4-yl)-phenyl]-4,5-dihydro-1*H*-pyrazol | 356 | |
| "A208" | 1-(4-Fluor-benzylcarbonyl)-3-(2-fluor-phenyl)-4,5-dihydro-1*H*-pyrazol | 301 | |
| "A209" | 1-(4-Fluor-benzylcarbonyl)-3-(2,5-dimethoxyphenyl)-4,5-dihydro-1*H*-pyrazol | 343 | |
| "A210" | 1-(4-Methoxy-benzylcarbonyl)-3-(2,5-dimethoxyphenyl)-4,5-dihydro-1*H*-pyrazol | 355 | |
| "A211" | 1-[2-(Thiophen-2-yl)-acetyl]-3-(2,5-dimethoxyphenyl)-4,5-dihydro-1*H*-pyrazol | 331 | |
| "A212" | 1-Benzylcarbonyl-3-(2,5-dimethoxy-phenyl)-4,5-dihydro-1*H*-pyrazol | 325 | |
| "A213" | 1-[2-(Thiophen-2-yl)-acetyl]-3-(2,3,4-trimethoxyphenyl)-4,5-dihydro-1*H*-pyrazol | 361 | |
| "A214" | 1-Benzylcarbonyl-3-(2,3,4-trimethoxy-phenyl)-4,5-dihydro-1*H*-pyrazol | 355 | |
| "A215" | 1-(4-Fluor-benzylcarbonyl)-3-(4-dimethylaminophenyl)-4,5-dihydro-1*H*-pyrazol | 326 | |
| "A216" | 1-Benzylcarbonyl-3-(4-dimethylamino-phenyl)-4,5-dihydro-1*H*-pyrazol | 308 | |
| "A217" | 3-(2,5-Dichlor-phenyl)-1-[2-(2-amino-benzothiazol-6-yl)-propionyl]-4,5-dihydro-1*H-*pyrazol | 419 | |
| ¹H-NMR (d⁶-DMSO): δ = 1.41 (d, J = 7 Hz, 3H), 3.30 (m, 1H), 3.39 (m, 1H), 3.83 (m, 1H), 3.94 (m, 1H), 4.61 (q, J = 7 Hz, 1H) 7.17 (dd, J₁ = 8 Hz, J₂ = 1.5 Hz, 1H), 7.24 (d, J = 8 Hz, 1H), 7.38 (s, 2H), 7.53 (dd, J₁ = 8.5 Hz, J₂ = 2.5 Hz, 1H), 7.59 (s, 1H), 7.60 (d, J = 8.5 Hz, 1H), 7.71 (d, J = 2.5 Hz, 1H). | | | |
| "A218" | 3-(2,5-Dichlor-phenyl)-1-[2-(2-aminobenzothiazol-6-yl)-acetyl]-4,5-dihydro-1*H-*pyrazol | 405 | |

### Beispiel 9

Die Herstellung von 1-(Benzo[1,2,5]thiadiazol-5-yl-methylcarbonyl)-3-(3-amino-1*H*-indazol-5-yl)-4,5-dihydro-1*H*-pyrazol ("A83") erfolgt analog nachstehendem Schema

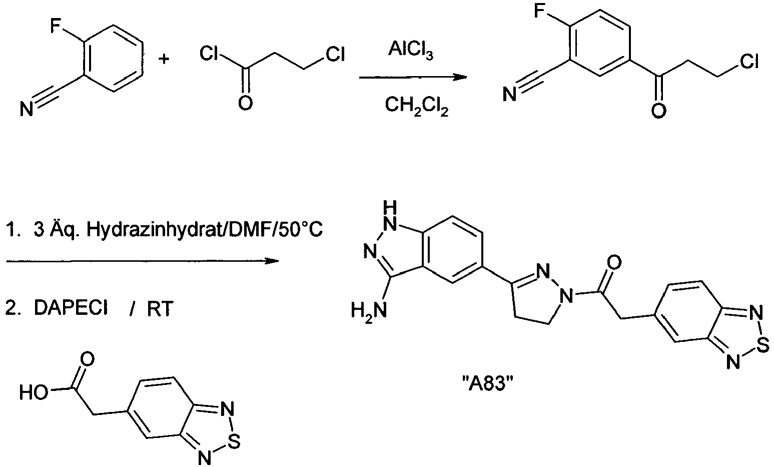

### Beispiel 10

Die Herstellung von 3-(3-Fluor-phenyl)-1-[2-(1*H*-benzimidazol-5-yl)-acetyl]-4,5-dihydro-1*H*-pyrazol ("A151") erfolgt analog nachstehendem Schema 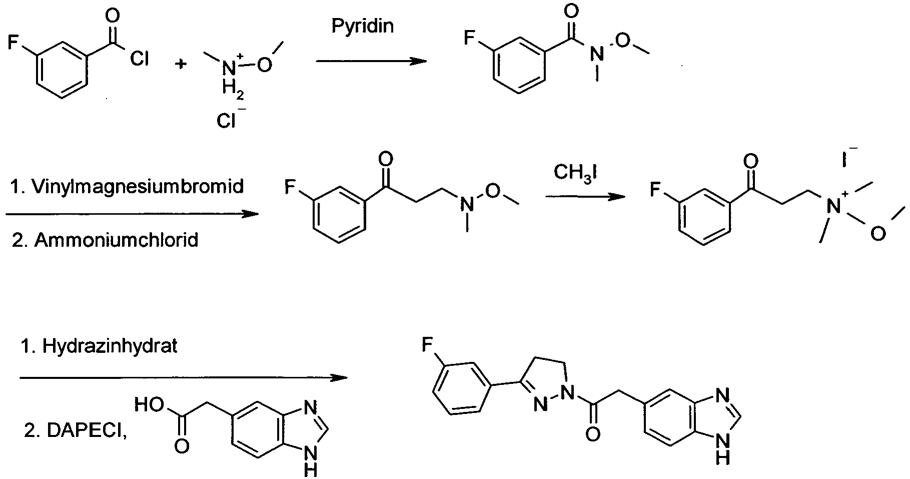
1. Eine auf 0°C gehaltene Lösung von 6.08 ml (50.0 mmol) 3-Fluor-benzoylchlorid und 5.37 g (55.0 mmol) N,O-Dimethylhydroxylamin-Hydrochlorid in 100 ml Dichlormethan wird mit 8.88 ml (110 mmol) Pyridin versetzt. Das Reaktionsgemisch wird 2 Stunden bei Raumtemperatur gerührt, dann mit 10 ml 1 N Salzsäure versetzt. Die organische Phase wird abgetrennt, mit 1 N Salzsäure und Wasser gewaschen, über Natriumsulfat getrocknet und eingedampft: 3-Fluor-N-methoxy-N-methyl-benzamid als farbloses Öl; ESI 184.
2. Zu einer auf 0°C gehaltenen Lösung von 5.80 g (31.6 mmol) 3-Fluor-N-methoxy-N-methyl-benzamid in 70 ml THF wird unter Stickstoff 35 ml einer 1 molaren Lösung Vinylmagnesiumbromid in THF zugetropft. Das Reaktionsgemisch wird 40 Minuten bei Raumtemperatur gerührt, dann wird gesättigte Ammoniumchloridlösung zugegeben und weitere 10 Minuten bei Raumtemperatur gerührt. Das Reaktionsgemisch wird mit tert.-Butylmethylether versetzt. Die organische Phase wird abgetrennt, mit Natriumhydrogencarbonatlösung und Wasser gewaschen und im Vakuum getrocknet: 1-(3-Fluorphenyl)-3-(methoxy-methylamino)-propan-1-on als gelbes Öl; ESI 212.
3. Eine Lösung von 2.40 g (11.4 mmol) 1-(3-Fluorphenyl)-3-(methoxy-methylamino)-propan-1-on in 20 ml THF wird mit 3.54 ml (56.8 mmol) Iodmethan versetzt und 3 Tage bei Raumtemperatur belassen. Die entstandenen Kristalle werden abgesaugt, mit tert.-Butylmethylether gewaschen und im Vakuum getrocknet: [3-(3-Fluor-phenyl)-3-oxo-propyl]-methoxy-dimethyl-ammonium-iodid als gelbliche Kristalle; ESI 226.
4. Eine Lösung von 94 mg (1.89 mmol) Hydrazinhydrat in 2 ml DMF wird unter Stickstoff mit 318 mg (0.90 mmol) [3-(3-Fluor-phenyl)-3-oxo-propyl]-methoxy-dimethyl-ammonium-iodid versetzt. Das Reaktionsgemisch wird unter Stickstoff 1 Stunde auf 50° C erhizt. Man lässt auf Raumtemperatur abkühlen, gibt 159 mg (0.90 mmol) (1H-Benzimidazol-5-yl)-essigsäure und 224 mg (1.17 mmol) DAPECI zu und rührt 18 Stunden bei Raumtemperatur. Das Reaktionsgemisch wird mit Wasser versetzt und mit Dichlormethan extrahiert. Die organische Phase wird über Natriumsulfat getrocknet und eingedampft. Der Rückstand wird durch präparative HPLC gereinigt: 3-(3-Fluor-phenyl)-1-[2-(1*H-*benzimidazol-5-yl)-acetyl]-4,5-dihydro-1*H*-pyrazol als gelbliche Kristalle; ESI 323.

Analog erhält man die nachstehenden Verbindungen

| Nr. | Name Struktur | ESI | |
|---|---|---|---|
| "A131" | 1-[2-(1*H*-Benzimidazol-5-yl)-acetyl]-3-(3-chlor-phenyl)-4,5-dihydro-1*H*-pyrazol | 339 | |
| "A133" | 3-(3-Chlor-phenyl)-1-[2-(benzo[1,2,5]-thiadiazol-5-yl)-acetyl]-4,5-dihydro-1*H-*pyrazol | 357 | |
| "A147" | 3-(3-Fluor-phenyl)-1-[2-(benzo[1,2,5]-thiadiazol-5-yl)-acetyl]-4,5-dihydro-1*H-*pyrazol | 341 | |
| "A157a" | 3-(3,5-Difluor-phenyl)-1-[2-(benzo[1,2,5]-thiadiazol-5-yl)-acetyl]-4,5-dihydro-1*H-*pyrazol | 359 | |
| "A158a" | 3-(3,5-Difluor-phenyl)-1-[2-(1,3-dihydro-2-oxo-benzimidazol-5-yl)-acetyl]-4,5-dihydro-1*H*-pyrazol | 357 | |
| | 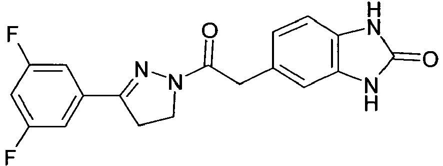 | | |
| "A159" | 3-(3,5-Difluor-phenyl)-1-[2-(benzimidazol-5-yl)-acetyl]-4,5-dihydro-1*H*-pyrazol | 341 | |
| | 1-(3-Nitro-benzylcarbonyl)-3-(3-fluorphenyl)-4,5-dihydro-1*H*-pyrazol | | |
| | | | |

### Beispiel 11

Die Herstellung von (3-{2-[3-(2,5-Dichlor-phenyl)-4,5-dihydro-1*H*-pyrazol-1-yl]-2-oxo-ethyl}-phenyl)-carbaminsäure-3-morpholin-4-yl-propylester ("A154") erfolgt analog nachstehendem Schema 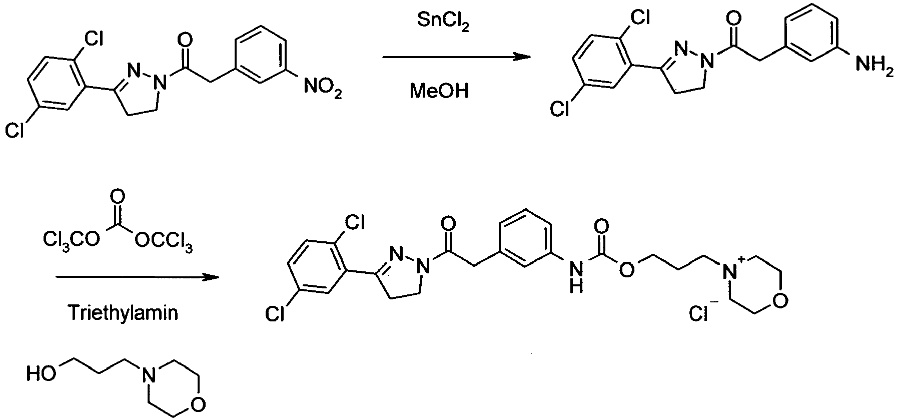
1. Eine Lösung von 3.30 g (6.28 mmol) 1-[3-(2,5-Dichlorphenyl)-4,5-dihydro-pyrazol-1-yl]-2-(3-nitrophenyl)-ethanon in 25 ml Methanol wird mit 5.00 g (25.6 mmol) Zinn(II)-chlorid versetzt. Die Lösung wird drei Stunden zum Sieden erhitzt und 18 Stunden bei Raumtemperatur belassen. Das Reaktionsgemisch wird mit 32 ml 2 N Natronlauge alkalisiert und über Kieselgur abgesaugt. Der Rückstand wird mit Wasser aufgekocht und filtriert. Der Rückstand wird an einer Kieselgelsäule mit Methanol / Dichlor-methan als Laufmittel chromatographiert: 2-(3-Aminophenyl)-1-[3-(2,5-dichlorphenyl)-4,5-dihydropyrazol-1-yl]-ethanon als farbloser Feststoff; ESI 348.
2. Eine Suspension von 174 mg (0.50 mmol) 2-(3-Aminophenyl)-1-[3-(2,5-dichlorphenyl)-4,5-dihydropyrazol-1-yl]-ethanon in 2 ml Dichlormethan wird mit 59.4 mg (0.20 mmol) Bis(trichlormethyl)-carbonat versetzt. Dann wird unter Eiskühlung 102 mg (1.00 mmol) Triethylamin zugetropft und das Reaktionsgemisch 10 Minuten bei Raumtemperatur gerührt. Dann wird 76.1 ml (0.55 mmol) 3-Morpholin-4-yl-propan-1-ol zugegeben und 18 Stunden bei Raumtemperatur gerührt. Das Reaktionsgemisch wird mit Wasser versetzt. Die organische Phase wird abgetrennt und die wässrige Phase mehrmals mit Dichlormethan extrahiert. Die vereinigten organischen Phasen werden über Natriumsulfat getrocknet, eingedampft und im Vakuum getrocknet. Der Rückstand wird an einer Kieselgelsäule mit Dichlormethan/Methanol als Laufmittel chromatographiert. Die Produkte enthaltenden Fraktionen werden vereinigt, eingedampft, in 5 ml einer 0.1 N Lösung von Chlorwasserstoff in 2-Propanol gelöst und die Lösung in 50 ml Diethylether eingetropft. Der entstandene Niederschlag wird abfiltriert, mit Diethylether gewaschen und getrocknet: (3-{2-[3-(2,5-Dichlorphenyl)-4,5-dihydropyrazol-1-yl]-2-oxoethyl}-phenyl)-carbaminsäure-3-morpholin-4-yl-propylester Hydrochlorid ("A154") als farbloser Feststoff; ESI 519.
¹H-NMR (d⁶-DMSO): δ = 2.10 (m, 2H), 3.06 (bs, 2H), 3.17 (m, 2H), 3.4 (m, 2H), 3.42 (t, J = 10 Hz, 2H), 3.81 (bs, 2H), 3.93 (t, J = 10 Hz, 2H), 3.94 (m, 2H), 3.96 (s, 2H), 4.16 (t, J = 6 Hz, 2H), 6.95 (d, J = 8 Hz, 1 H), 7.21 (t, J = 8 Hz, 1H), 7.34 (bd, J = h 8 Hz, 1H), 7.44 (bs, 1H), 7.56 (dd, J₁ = 8.5 Hz, J₂ = 2.5 Hz, 1H), 7.63 (d, J = 8.5 Hz, 1H), 7.79 (d, J = 2.5 Hz, 1 H), 9.67 (s, 1 H), 10.95 (bs, 1 H).

Analog erhält man die nachstehenden Verbindungen

| Nr. | Name Struktur | ESI | |
|---|---|---|---|
| "A144" | (3-{2-[3-(4-Fluor-phenyl)-4,5-dihydro-1*H-*pyrazol-1-yl]-2-oxo-ethyl}-phenyl)-carbaminsäure-3-dimethylamino-propylester | 427 | |
| | 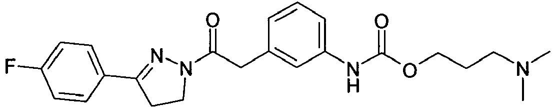 | | |
| "A148" | (3-{2-[3-(2,5-Difluor-phenyl)-4,5-dihydro-1*H-*pyrazol-1-yl]-2-oxo-ethyl}-phenyl)-carbaminsäure-3-dimethylamino-propylester | 445 | |
| "A150" | (3-{2-[3-(4-Fluor-phenyl)-4,5-dihydro-1*H-*pyrazol-1-yl]-2-oxo-ethyl}-phenyl)-carbaminsäure-3-morpholin-4-yl-propyl ester | 469 | |
| | 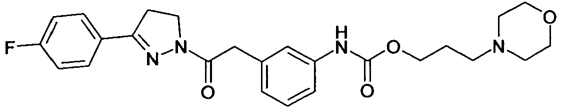 | | |
| "A152" | (3-{2-[3-(2,5-Difluor-phenyl)-4,5-dihydro-1*H-*pyrazol-1-yl]-2-oxo-ethyl}-phenyl)-carbaminsäure-3-morpholin-4-yl-propyl ester | 487 | |
| "A153" | (3-{2-[3-(2,5-Dichlor-phenyl)-4,5-dihydro-1*H-*pyrazol-1-yl]-2-oxo-ethyl}-phenyl)-carbaminsäure-3-dimethylamino-propylester | 477 | |
| "A155" | (3-{2-[3-(3-Fluor-phenyl)-4,5-dihydro-1*H-*pyrazol-1-yl]-2-oxo-ethyl}-phenyl)-carbaminsäure-3-dimethylamino-propylester | 427 | |
| "A156" | (3-{2-[3-(3-Fluor-phenyl)-4,5-dihydro-1*H-*pyrazol-1-yl]-2-oxo-ethyl}-phenyl)-carbaminsäure-3-morpholin-4-yl-propyl ester | 469 | |

### Beispiel 12

Die Herstellung von 1-(4-Fluor-benzylcarbonyl)-3-(3-methoxy-phenyl)-4,5-dihydro-1*H*-pyrazol ("A159a"), ESI 313, erfolgt analog nachstehendem Schema

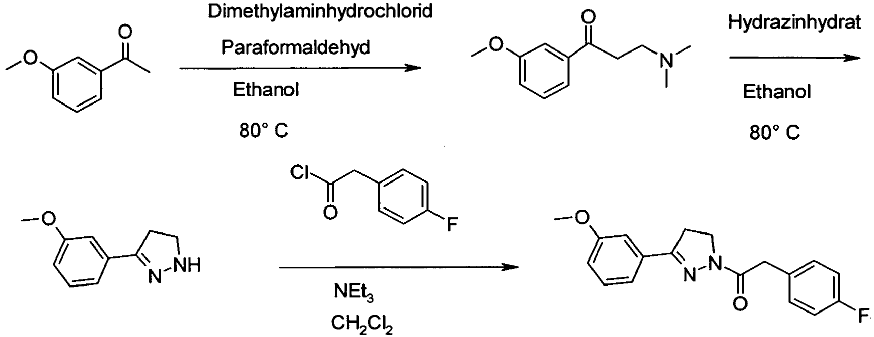

Die erste Stufe, eine Mannich-Reaktion, ist z. B. beschrieben in D. Lewis et al., Bioorganic and Medicinal Chemistry Letters 13 (2003) 1385-1398.

Analog erhält man die nachstehenden Verbindungen

| Nr. | Name Struktur | ESI | |
|---|---|---|---|
| "A160" | 1-(3-Methoxy-benzylcarbonyl)-3-(3-methoxyphenyl)-4,5-dihydro-1*H*-pyrazol | 325 | |
| "A161" | 1-(4-Methoxy-benzylcarbonyl)-3-(3-methoxyphenyl)-4,5-dihydro-1*H*-pyrazol | 325 | |
| "A162" | 3-(3-Methoxy-phenyl)-1-[3-(3,4,5-trimethoxyphenyl)-propionyl]-4,5-dihydro-1*H*-pyrazol | 399 | |
| "A163" | 1-Benzylcarbonyl-3-(3-methoxy-phenyl)-4,5-dihydro-1*H*-pyrazol | 295 | |
| "A164" | 1-(4-Fluor-benzylcarbonyl)-3-(3-nitro-phenyl)-4,5-dihydro-1*H*-pyrazol | 328 | |
| "A165" | 1-(3-Methoxy-benzylcarbonyl)-3-(3-nitro-phenyl)-4,5-dihydro-1*H*-pyrazol | 340 | |
| "A166" | 3-(3-Nitro-phenyl)-1-[2-(thiophen-2-yl)-acetyl]-4,5-dihydro-1*H*-pyrazol | 316 | |
| "A167" | 1-Benzylcarbonyl-3-(3-nitro-phenyl)-4,5-dihydro-1*H*-pyrazol | 310 | |
| "A168" | 1-(4-Fluor-benzylcarbonyl)-3-(furan-2-yl)-4,5-dihydro-1*H*-pyrazol | 273 | |
| "A169" | 1-(3-Methoxy-benzylcarbonyl)-3-(furan-2-yl)-4,5-dihydro-1*H*-pyrazol | 285 | |
| "A170" | 1-(4-Methoxy-benzylcarbonyl)-3-(furan-2-yl)-4,5-dihydro-1*H*-pyrazol | 285 | |
| "A171" | 3-(Furan-2-yl)-1-[2-(thiophen-2-yl)-acetyl]-4,5-dihydro-1*H*-pyrazol | 261 | |
| "A172" | 1-Benzylcarbonyl-3-(furan-2-yl)-4,5-dihydro-1*H-*pyrazol | 255 | |
| "A173" | 1-Benzylcarbonyl-3-[4-(morpholin-4-yl)-phenyl]-4,5-dihydro-1*H*-pyrazol | 350 | |
| "A174" | 1-(3-Methoxy-benzylcarbonyl)-3-(2-fluorphenyl)-4,5-dihydro-1*H*-pyrazol | 313 | |
| "A175" | 1-(4-Methoxy-benzylcarbonyl)-3-(2-fluorphenyl)-4,5-dihydro-1*H*-pyrazol | 313 | |
| "A176" | 1-Benzylcarbonyl-3-(2-fluor-phenyl)-4,5-dihydro-1*H*-pyrazol | 283 | |
| "A177" | 1-(4-Fluor-benzylcarbonyl)-3-(4-cyan-phenyl)-4,5-dihydro-1*H*-pyrazol | 308 | |
| "A178" | 1-(3-Methoxy-benzylcarbonyl)-3-(4-cyan-phenyl)-4,5-dihydro-1*H*-pyrazol | 320 | |
| "A179" | 1-(4-Methoxy-benzylcarbonyl)-3-(4-cyan-phenyl)-4,5-dihydro-1*H*-pyrazol | 320 | |
| "A180" | 1-Benzylcarbonyl-3-(4-cyan-phenyl)-4,5-dihydro-1*H*-pyrazol | 290 | |
| "A181" | 1-(4-Fluor-benzylcarbonyl)-3-(3-cyan-phenyl)-4,5-dihydro-1*H*-pyrazol | 308 | |
| "A182" | 1-(3-Methoxy-benzylcarbonyl)-3-(3-cyan-phenyl)-4,5-dihydro-1*H*-pyrazol | 320 | |
| "A183" | 1-(4-Methoxy-benzylcarbonyl)-3-(3-cyan-phenyl)-4,5-dihydro-1*H*-pyrazol | 320 | |
| "A184" | 1-Benzylcarbonyl-3-(3-cyan-phenyl)-4,5-dihydro-1*H*-pyrazol | 290 | |

### Pharmakologische Daten

### Met-Kinase-Inhibierung

**Tabelle 1**

| Verbindung Nr. | IC₅₀ [M] |
|---|---|
| "A1" | 3.2 x 10⁻⁷ |
| "A2" | 6.1 x 10⁻⁷ |
| "A30" | 9.9 x 10⁻⁷ |
| "A31" | 3.3 x 10⁻⁷ |
| "A100" | 9 x 10⁻⁹ |
| "A108" | 3 x 10⁻⁹ |
| "A120" | 3.6 x 10⁻⁹ |
| "A153" | 8 x 10⁻⁹ |
| "A154" | 2.5 x 10⁻⁹ |
| | |

Die nachfolgenden Beispiele betreffen Arzneimittel:

### Beispiel A: Injektionsgläser

Eine Lösung von 100 g eines Wirkstoffes der Formel I gemäß Anspruch 1 und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I gemäß Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I gemäß Anspruch 1, 9,38 g NaH₂PO₄ · 2 H₂O, 28,48 g Na₂HPO₄ 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I gemäß Anspruch 1 mit 99,5 g Vaseline unter aseptischen Bedingungen.

### Beispiel E: Tabletten

Ein Gemisch von 1 kg Wirkstoff der Formel I gemäß Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I gemäß Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I gemäß Anspruch 1 in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Nr. | Name |
|---|---|
| "A1" | 1-(Benzo[1,2,5]thiadiazol-5-yl-methylcarbonyl)-3-(2,4-dimethoxy-phenyl)-4,5-dihydro-1*H*-pyrazol |
| "A2" | 1-(Benzo[1,2,5]thiadiazol-5-yl-methylcarbonyl)-3-(4-fluor-phenyl)-4,5-dihydro-1*H*-pyrazol |
| "A3" | 1-(Benzo[1,2,5]thiadiazol-5-yl-methylcarbonyl)-3-(3,4-dimethoxy-phenyl)-4,5-dihydro-1*H*-pyrazol |
| "A4" | 1-(3-Methoxy-benzylcarbonyl)-3-(3-chlor-phenyl)-4,5-dihydro-1*H*-pyrazol |
| "A5" | 1-(3-Methoxy-benzylcarbonyl)-3-(4-chlor-phenyl)-4,5-dihydro-1*H*-pyrazol |
| "A6" | 1-(4-Methoxy-benzylcarbonyl)-3-(3-chlor-phenyl)-4,5-dihydro-1*H*-pyrazol |
| "A7" | 1-(Thiophen-2-ylmethylcarbonyl)-3-(3-chlor-phenyl)-4,5-dihydro-1*H*-pyrazol |
| "A8" | 1-Benzylcarbonyl-3-(3-chlor-phenyl)-4,5-dihydro-1*H-*pyrazol |
| "A9" | 1-(4-Fluor-benzylcarbonyl)-3-(4-methoxy-phenyl)-4,5-dihydro-1*H*-pyrazol |
| "A10" | 1-(3-Methoxy-benzylcarbonyl)-3-(4-methoxyphenyl)-4,5-dihydro-1*H*-pyrazol |
| "A11" | 1-(4-Methoxy-benzylcarbonyl)-3-(4-methoxyphenyl)-4,5-dihydro-1*H*-pyrazol |
| "A12" | 1-Benzylcarbonyl-3-(4-methoxy-phenyl)-4,5-dihydro-1*H*-pyrazol |
| "A13" | 1-(4-Fluor-benzylcarbonyl)-3-(2-methoxy-phenyl)-4,5-dihydro-1*H*-pyrazol |
| "A14" | 1-(3-Methoxy-benzylcarbonyl)-3-(2-methoxy-phenyl)-4,5-dihydro-1*H*-pyrazol |
| "A15" | 1-(4-Methoxy-benzylcarbonyl)-3-(2-methoxyphenyl)-4,5-dihydro-1*H*-pyrazol |
| "A16" | 1-(Thiophen-2-ylmethylcarbonyl)-3-(2-methoxyphenyl)-4,5-dihydro-1*H*-pyrazol |
| "A17" | 1-Benzylcarbonyl-3-(2-methoxy-phenyl)-4,5-dihydro-1*H*-pyrazol |
| "A18" | 1-(3-Methoxy-benzylcarbonyl)-3-(4-dimethylaminophenyl)-4,5-dihydro-1*H*-pyrazol |
| "A19" | 1-(4-Methoxy-benzylcarbonyl)-3-(4-dimethylaminophenyl)-4,5-dihydro-1*H*-pyrazol |
| "A20" | 1-(Thiophen-2-ylmethylcarbonyl)-3-(4-dimethylamino-phenyl)-4,5-dihydro-1*H*-pyrazol |
| "A21" | 1-(4-Fluor-benzylcarbonyl)-3-(4-fluor-phenyl)-4,5-dihydro-1*H*-pyrazol |
| "A22" | 1-(4-Methoxy-benzylcarbonyl)-3-(4-fluor-phenyl)-4,5-dihydro-1*H*-pyrazol |
| "A23" | 1-(Thiophen-2-ylmethylcarbonyl)-3-(4-fluor-phenyl)-4,5-dihydro-1*H*-pyrazol |
| "A24" | 1-Benzylcarbonyl-3-(4-fluor-phenyl)-4,5-dihydro-1*H-*pyrazol |
| "A25" | 1-(Benzo[1,2,5]thiadiazol-5-yl-methylcarbonyl)-3-(3,4-dimethoxy-phenyl)-5,5-dimethyl-4,5-dihydro-1*H-*pyrazol |
| "A26" | 1-(Benzo[1,2,5]thiadiazol-5-yl-methylcarbonyl)-3-(3,4-dimethoxy-phenyl)-4,4-dimethyl-4,5-dihydro-1*H-*pyrazol |
| "A27" | 1-[3-(2-Morpholin-4-ylethoxy)-benzylcarbonyl]-3-(5-Chlor-thiophen-2-yl)-4,5-dihydro-1*H*-pyrazol |
| "A28" | 1-[3-(3-Diethylaminopropoxy)-benzylcarbonyl]-3-(5-Chlor-thiophen-2-yl)-4,5-dihydro-1*H*-pyrazol |
| "A29" | 1-[3-(3-Diethylaminopropoxy)-benzylcarbonyl]-3-(3,4-dimethoxy-phenyl)-4,5-dihydro-1*H*-pyrazol |
| "A30" | 1-(3-Hydroxy-benzylcarbonyl)-3-(2,4-dimethoxyphenyl)-4,5-dihydro-1*H*-pyrazol |
| "A31" | 1-(3-Hydroxy-benzylcarbonyl)-3-(4-fluor-phenyl)-4,5-dihydro-1*H*-pyrazol |
| "A32" | 1-(Benzo[1,2,5]thiadiazol-5-yl-methylcarbonyl)-3-phenyl-4,5-dihydro-1*H*-pyrazol |
| "A33" | 1-(Benzo[1,2,5]thiadiazol-5-yl-methylcarbonyl)-3-(4-chlor-phenyl)-4,5-dihydro-1*H*-pyrazol |
| "A34" | 1-(Benzo[1,2,5]thiadiazol-5-yl-methylcarbonyl)-3-(5-chlor-thiophen-2-yl)-4,5-dihydro-1*H*-pyrazol |
| "A35" | 1-(Benzo[1,2,5]thiadiazol-5-yl-methylcarbonyl)-3-(4-brom-phenyl)-4,5-dihydro-1*H*-pyrazol |
| "A36" | 1-(3-Hydroxy-benzylcarbonyl)-3-(4-chlor-phenyl)-4,5-dihydro-1*H*-pyrazol |
| "A37" | 1-(Benzo[1,2,5]thiadiazol-5-yl-methylcarbonyl)-3-(4-methylsulfonamido-phenyl)-4,5-dihydro-1*H*-pyrazol |
| "A38" | 1-(3-Hydroxy-benzylcarbonyl)-3-(3,4-dihydro-2-oxo-1*H*-chinazolin-6-yl)-4,5-dihydro-1*H*-pyrazol |
| "A39" | 1-(Benzo[1,2,5]thiadiazol-5-yl-methylcarbonyl)-3-(3,4-dihydro-2-oxo-1*H*-chinazolin-6-yl)-4,5-dihydro-1*H*-pyrazol |
| "A40" | 1-(Benzo[1,2,5]thiadiazol-5-yl-methylcarbonyl)-3-(4-hydroxy-phenyl)-4.,5-dihydro-1*H*-pyrazol |
| "A41" | 1-(Benzo[1,2,5]thiadiazol-5-yl-methylcarbonyl)-3-(2-hydroxy-phenyl)-4,5-dihydro-1*H*-pyrazol |
| "A42" | 1-(Benzo[1,2,5]thiadiazol-5-yl-methylcarbonyl)-3-(2-hydroxy-5-chlor-phenyl)-4,5-dihydro-1*H*-pyrazol |
| "A43" | 1-(Benzo[1,2,5]thiadiazol-5-yl-methylcarbonyl)-3-(3-chlor-6-methoxy-phenyl)-4,5-dihydro-1*H*-pyrazol |
| "A44" | 1-(Benzo[1,2,5]thiadiazol-5-yl-methylcarbonyl)-3-(3-chlor-4-methoxy-phenyl)-4,5-dihydro-1*H*-pyrazol |
| "A45" | 1-(3-Hydroxy-benzylcarbonyl)-3-(5-chlor-thiophen-2-yl)-4,5-dihydro-1*H*-pyrazol |
| "A46" | 1-(3-Methoxy-benzylcarbonyl)-3-(5-chlor-thiophen-2-yl)-4,5-dihydro-1*H*-pyrazol |
| "A47" | 1-[3-(3-oxo-morpholin-4-yl)-benzylcarbonyl]-3-(3,4-dimethoxy-phenyl)-4,5-dihydro-1*H*-pyrazol |
| "A48" | 1-(Phenethyl-carbonyl)-3-(3,4-dimethoxy-phenyl)-4,5-dihydro-1*H*-pyrazol |
| "A49" | 1-[4-(Morpholin-4-ylcarbonyl)-phenyl-methylcarbonyl]-3-(3,4-dimethoxy-phenyl)-4,5-dihydro-1*H*-pyrazol |
| "A50" | 1-[3-(3-Diethylamino-propoxycarbonylamino)-phenyl-methylcarbonyl]-3-(3,4-dimethoxy-phenyl)-4,5-dihydro-1*H*-pyrazol |
| "A51" | 1-{3-[3-(3-Diethylamino-propyl)-ureido]-phenyl-methylcarbonyl}-3-(3,4-dimethoxy-phenyl)-4,5-dihydro-1*H*-pyrazol |
| "A52" | 1-Benzylcarbonyl-3-(2,4-dimethoxy-phenyl)-4,5-dihydro-1*H*-pyrazol |
| "A53" | 1-(3-Methoxy-benzylcarbonyl)-3-(2,4-dimethoxyphenyl)-4,5-dihydro-1*H*-pyrazol |
| "A54" | 1-(3-Methoxy-benzylcarbonyl)-3-(4-fluor-phenyl)-4,5-dihydro-1*H*-pyrazol |
| "A55" | 1-(3-Methoxy-benzylcarbonyl)-3-(2,5-dimethoxy-phenyl)-4,5-dihydro-1*H*-pyrazol |
| "A56" | 1-(Benzo[1,2,5]thiadiazol-5-yl-methylcarbonyl)-3-(4-methylsulfanil-phenyl)-4,5-dihydro-1*H*-pyrazol |
| "A57" | 1-(Benzo[1,2,5]thiadiazol-5-yl-methylcarbonyl)-3-(dibenzofuran-2-yl)-4,5-dihydro-1*H*-pyrazol |
| "A58" | 1-(4-Hydroxy-benzylcarbonyl)-3-(4-chlor-phenyl)-4,5-dihydro-1*H*-pyrazol |
| "A59" | 1-[4-(Morpholin-4-ylethoxy)-benzylcarbonyl]-3-(3,4-dimethoxy-phenyl)-4,5-dihydro-1*H*-pyrazol |
| "A60" | 1-[4-(3-Diethylaminopropoxy)-benzylcarbonyl]-3-(5-Chlor-thiophen-2-yl)-4,5-dihydro-1*H*-pyrazol |
| "A61" | 1-(4-Hydroxy-benzylcarbonyl)-3-(2,4-dimethoxyphenyl)-4,5-dihydro-1*H*-pyrazol |
| "A62" | 1-(3-Hydroxy-benzylcarbonyl)-3-(3,4-dimethoxyphenyl)-4,5-dihydro-1*H*-pyrazol |
| "A63" | 1-(3-Chlor-benzylcarbonyl)-3-(2,4-dimethoxyphenyl)-4,5-dihydro-1*H*-pyrazol |
| "A64" | 1-(2-Benzo[1,2,5]thiadiazol-5-yl-propionyl)-3-(4-fluor-phenyl)-4,5-dihydro-1*H*-pyrazol |
| "A65" | 1-[2-Hydroxy-2-(3-hydroxy-phenyl)-acetyl]-3-(4-fluor-phenyl)-4,5-dihydro-1*H*-pyrazol |
| "A66" | 1-[2-Hydroxy-2-(3-hydroxy-phenyl)-acetyl]-3-(4-chlor-phenyl)-4,5-dihydro-1*H*-pyrazol |
| "A67" | 1-(2-Hydroxy-benzylcarbonyl)-3-(2,4-dimethoxyphenyl)-4,5-dihydro-1*H*-pyrazol |
| "A68" | 1-(3-Hydroxy-benzylcarbonyl)-3-(2-oxo-1,3-dihydro-indol-5-yl)-4,5-dihydro-1*H*-pyrazol |
| "A69" | 1-(4-Hydroxy-benzylcarbonyl)-3-(4-fluor-phenyl)-4,5-dihydro-1*H*-pyrazol |
| "A70" | 1-(2-Hydroxy-benzylcarbonyl)-3-(4-fluor-phenyl)-4,5-dihydro-1*H*-pyrazol |
| "A71" | 1-[4-(Morpholin-4-ylethoxy)-benzylcarbonyl]-3-(5-Chlor-thiophen-2-yl)-4,5-dihydro-1*H*-pyrazol |
| "A72" | 1-(4-Fluor-benzylcarbonyl)-3-(5-chlor-thiophen-2-yl)-4,5-dihydro-1*H*-pyrazol |
| "A73" | 1-(4-Methoxy-benzylcarbonyl)-3-(5-chlor-thiophen-2-yl)-4,5-dihydro-1*H*-pyrazol |
| "A74" | 1-(4-Acetamido-benzylcarbonyl)-3-(5-chlor-thiophen-2-yl)-4,5-dihydro-1*H*-pyrazol |
| "A75" | 1-(Benzo[1,2,5]thiadiazol-5-yl-methylcarbonyl)-3-(5-chlor-furan-2-yl)-4,5-dihydro-1*H*-pyrazol |
| "A76" | 1-(4-Hydroxy-3-methoxy-benzylcarbonyl)-3-(5-chlor-thiophen-2-yl)-4,5-dihydro-1*H*-pyrazol |
| "A77" | 1-(4-Methylsulfonyl-benzylcarbonyl)-3-(5-chlor-thiophen-2-yl)-4,5-dihydro-1*H*-pyrazol |
| "A78" | 1-(4-Ethoxy-benzylcarbonyl)-3-(5-chlor-thiophen-2-yl)-4,5-dihydro-1*H*-pyrazol |
| "A79" | 1-(4-Hydroxy-3-chlor-benzylcarbonyl)-3-(5-chlor-thiophen-2-yl)-4,5-dihydro-1*H*-pyrazol |
| "A80" | 1-(2-(4-Hydroxy-phenyl)-propionyl)-3-(5-chlor-thiophen-2-yl)-4,5-dihydro-1*H*-pyrazol |
| "A81" | 1-(2-(Benzo[1,2,5]thiadiazol-5-yl)-propionyl)-3-(5-chlor-thiophen-2-yl)-4,5-dihydro-1*H*-pyrazol |
| "A82" | 1-(2-(4-Fluor-phenyl)-propionyl)-3-(5-chlor-thiophen-2-yl)-4,5-dihydro-1*H*-pyrazol |
| "A83" | 1-(Benzo[1,2,5]thiadiazol-5-yl-methylcarbonyl)-3-(3-amino-1*H*-indazol-5-yl)-4,5-dihydro-1*H*-pyrazol |
| "A84" | 1-(Benzo[1,2,5]thiadiazol-5-yl-methylcarbonyl)-3-(2-hydroxy-phenyl)-4,5-dihydro-1*H*-pyrazol |
| "A85" | 1-(3-Chlor-benzylcarbonyl)-3-(3,4-dimethoxyphenyl)-4,5-dihydro-1*H*-pyrazol |
| "A86" | 1-(Benzo[1,2,5]thiadiazol-5-yl-methylcarbonyl)-3-(2-oxo-3,4-dihydro-1*H*-chinazolin-6-yl)-4,5-dihydro-1*H-*pyrazol |
| "A87" | 1-(Benzo[1,2,5]thiadiazol-5-yl-methylcarbonyl)-3-(4-hydroxy-phenyl)-4,5-dihydro-1*H*-pyrazol |
| "A88" | 1-(3-Hydroxy-benzylcarbonyl)-3-(2-oxo-3,4-dihydro-1*H*-chinazolin-6-yl)-4,5-dihydro-1*H*-pyrazol |
| "A89" | 1-(Benzo[1,2,5]thiadiazol-5-yl-methylcarbonyl)-3-(3-chlor-6-hydroxy-phenyl)-4,5-dihydro-1*H*-pyrazol |
| "A90" | 1-(3-Hydroxy-benzylcarbonyl)-3-(5-chlor-thiophen-2-yl)-4,5-dihydro-1*H*-pyrazol |
| "A91" | 1-(3-Hydroxy-benzylcarbonyl)-3-(4-hydroxyphenyl)-4,5-dihydro-1*H*-pyrazol |
| "A92" | 1-(Benzo[1,3]dioxol-5-yl-methylcarbonyl)-3-(5-chlor-thiophen-2-yl)-4,5-dihydro-1*H*-pyrazol |
| "A93" | 1-(4-Hydroxy-benzylcarbonyl)-3-(5-chlor-thiophen-2-yl)-4,5-dihydro-1*H*-pyrazol |
| "A94" | 1-(4-Methoxy-benzylcarbonyl)-3-(5-chlor-thiophen-2-yl)-4,5-dihydro-1*H*-pyrazol |
| "A95" | 1-(Benzo[1,2,5]thiadiazol-5-yl-methylcarbonyl)-3-(3-chlor-6-methoxy-phenyl)-4,5-dihydro-1*H*-pyrazol |
| "A96" | 1-(4-Hydroxy-benzylcarbonyl)-3-(3,4-dimethoxyphenyl)-4,5-dihydro-1*H*-pyrazol |
| "A97" | 1-(3-Chlor-4-hydroxy-benzylcarbonyl)-3-(5-chlor-thiophen-2-yl)-4,5-dihydro-1*H*-pyrazol |
| "A98" | 3-(5-Chlor-thiophen-2-yl)-1-[2-(4-hydroxy-phenyl)-propionyl]-4,5-dihydro-1*H*-pyrazol |
| | 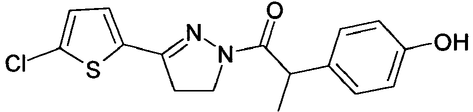 |
| "A99" | 1-(4-Methoxy-benzylcarbonyl)-3-(3,4-dimethoxyphenyl)-4,5-dihydro-1*H*-pyrazol |
| "A100" | 3-(2-Oxo-3,4-dihydro-1*H*-chinazolin-6-yl)-1-[2-(4-hydroxy-phenyl)-propionyl]-4,5-dihydro-1*H*-pyrazol |
| | 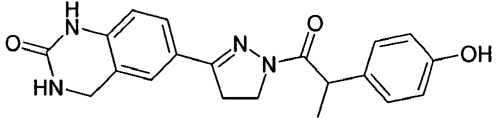 |
| "A101" | 1-[3-(3-Oxo-morpholin-4-yl)-benzylcarbonyl]-3-(5-chlor-thiophen-2-yl)-4,5-dihydro-1*H*-pyrazol |
| | 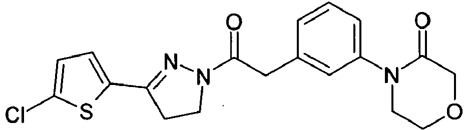 |
| "A102" | 1-[3-(3-Oxo-morpholin-4-yl)-benzylcarbonyl]-3-(3,4-dimethoxy-phenyl)-4,5-dihydro-1*H*-pyrazol |
| "A103" | 3-(3-Chlor-6-hydroxy-phenyl)-1-[2-(benzo[1,2,5]-thiadiazol-5-yl)-propionyl]-4,5-dihydro-1*H*-pyrazol |
| | 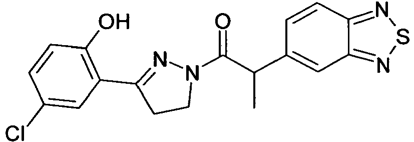 |
| "A104" | 1-[4-(3-Morpholin-4-yl-propoxy)-benzylcarbonyl]-3-(5-chlor-thiophen-2-yl)-4,5-dihydro-1*H*-pyrazol |
| | 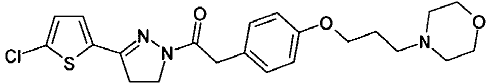 |
| "A105" | 1-[2-(1*H*-Benzimidazol-5-yl)-acetyl]-3-(5-chlor-thiophen-2-yl)-4,5-dihydro-1*H*-pyrazol |
| | 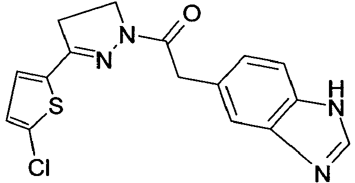 |
| "A106" | 3-(4-Chlor-phenyl)1-(3-phenyl-propionyl)-4,5-dihydro-1*H*-pyrazol |
| | 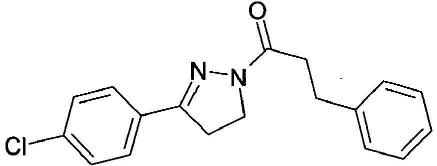 |
| "A107" | 1-(4-Methylsulfonyl-benzylcarbonyl)-3-(4-chlorphenyl)-4,5-dihydro-1*H*-pyrazol |
| "A108" | 1-[2-(1*H*-Benzimidazol-5-yl)-acetyl]-3-(3-chlor-6-hydroxy-phenyl)-4,5-dihydro-1*H*-pyrazol |
| "A109" | 1-[3-(2-Morpholin-4-yl-ethoxy)-benzylcarbonyl]-3-(5-chlor-thiophen-2-yl)-4,5-dihydro-1*H*-pyrazol |
| "A110" | 1-(Benzo[1,3]dioxol-5-yl-methylcarbonyl)-3-(3-chlor-6-hydroxy-phenyl)-4,5-dihydro-1*H*-pyrazol |
| "A111" | 3-(4-Chlor-phenyl)1-[2-(4-hydroxy-phenyl)-propionyl]-4,5-dihydro-1*H*-pyrazol |
| "A112" | 1-[2-(1*H*-Benzimidazol-5-yl)-acetyl]-3-(4-chlor-phenyl)-4,5-dihydro-1*H*-pyrazol |
| "A113" | 1-(3-Chlor-4-hydroxy-benzylcarbonyl)-3-(3-chlor-6-hydroxy-phenyl)-4,5-dihydro-1*H*-pyrazol |
| "A114" | 1-(4-Hydroxy-benzylcarbonyl)-3-(3-chlor-6-methoxyphenyl)-4,5-dihydro-1*H*-pyrazol |
| "A115" | 1-(Benzo[1,3]dioxol-5-yl-methylcarbonyl)-3-(2-oxo-3,4-dihydro-1*H*-chinazolin-6-yl)-4,5-dihydro-1*H-*pyrazol |
| "A116" | 3-(3,4-Dimethoxy-phenyl)-1-[2-(benzo[1,2,5]-thiadiazol-5-yl)-acetyl]-5,5-dimethyl-4,5-dihydro-1*H-*pyrazol |
| "A117" | 1-{4-[3-(N,N-Diethoxy-amino)-propoxy]-benzylcarbonyl}-3-(4-chlor-phenyl)-4,5-dihydro-1*H-*pyrazol |
| "A118" | 3-(5-Chlor-thiophen-2-yl)-1-[2-(chinoxalin-6-yl)-acetyl]-4,5-dihydro-1*H*-pyrazol |
| "A119" | 3-(3-Chlor-6-hydroxy-phenyl)-1-[2-(chinoxalin-6-yl)-acetyl]-4,5-dihydro-1*H*-pyrazol |
| "A120" | 3-(3-Chlor-6-hydroxy-phenyl)-1-[2-(4-hydroxyphenyl)-propionyl]-4,5-dihydro-1*H*-pyrazol |
| "A121" | 1-(4-Hydroxy-benzylcarbonyl)-3-(3-chlor-6-hydroxyphenyl)-4,5-dihydro-1*H*-pyrazol |
| "A122" | 1-(3-Chlor-benzylcarbonyl)-3-(3-chlor-6-hydroxyphenyl)-4,5-dihydro-1*H*-pyrazol |
| "A123" | 1-(2-{4-[3-(N,N-Diethoxy-amino)-propoxy]-phenyl}-propionyl)-3-(4-chlor-phenyl)-4,5-dihydro-1*H*-pyrazol |
| | 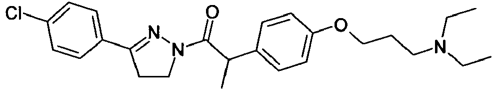 |
| "A124" | 1-[4-(Morpholin-4-ylcarbonyl)-benzylcarbonyl]-3-(5-chlor-thiophen-2-yl)-4,5-dihydro-1*H*-pyrazol |
| "A125" | 1-[4-(1-Methyl-piperazin-4-ylcarbonyl)-benzylcarbonyl]-3-(5-chlor-thiophen-2-yl)-4,5-dihydro-1*H*-pyrazol |
| "A126" | 1-[2-(Pyridin-3-yl)-acetyl]-3-(3-chlor-6-hydroxyphenyl)-4,5-dihydro-1*H*-pyrazol |
| "A127" | 1-[2-(Pyridin-2-yl)-acetyl]-3-(3-chlor-6-hydroxyphenyl)-4,5-dihydro-1*H*-pyrazol |
| "A128" | 1-(4-Cyan-benzylcarbonyl)-3-(5-chlor-thiophen-2-yl)-4,5-dihydro-1*H*-pyrazol |
| "A129" | 1-[2-(Pyridin-3-yl)-acetyl]-3-(5-chlor-thiophen-2-yl)-4,5-dihydro-1*H*-pyrazol |
| "A130" | 1-[2-(Pyridin-2-yl)-acetyl]-3-(5-chlor-thiophen-2-yl)-4,5-dihydro-1*H*-pyrazol |
| "A131" | 1-[2-(1*H*-Benzimidazol-5-yl)-acetyl]-3-(3-chlor-phenyl)-4,5-dihydro-1*H*-pyrazol |
| "A132" | 1-(9*H*-Carbazol-3-yl-methylcarbonyl)-3-(3-chlor-6-hydroxy-phenyl)-4,5-dihydro-1*H*-pyrazol |
| "A133" | 3-(3-Chlor-phenyl)-1-[2-(benzo[1,2,5]thiadiazol-5-yl)-acetyl]-4,5-dihydro-1*H*-pyrazol |
| "A134" | 1-{2-[2-(5-Chlor-thiophen-2-yl)-(1*H*-benzimidazol-5-yl]-acetyl}-3-(5-chlor-thiophen-2-yl)-4,5-dihydro-1*H-*pyrazol |
| | 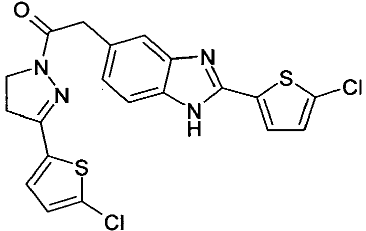 |
| "A135" | 1-{2-[2-(5-Chlor-thiophen-2-yl)-(1*H*-benzimidazol-5-yl]-acetyl}-3-(3-chlor-6-hydroxy-phenyl)-4,5-dihydro-1*H*-pyrazol |
| "A136" | 3-(3-C hlor-6-hyd roxy-phenyl)-1-[2-(2-aminobenzothiazol-6-yl)-propionyl]-4,5-dihydro-1*H*-pyrazol |
| | 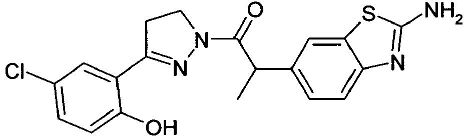 |
| "A137" | 3-(4-Fluor-phenyl)-1-[2-(2-amino-benzothiazol-6-yl)-propionyl]-4,5-dihydro-1*H*-pyrazol |
| "A138" | 3-(4-Fluor-phenyl)-1-[2-(2-amino-benzimidazol-6-yl)-acetyl]-4,5-dihydro-1*H*-pyrazol |
| "A139" | 1-(3-Nitro-benzylcarbonyl)-3-(3-chlor-6-hydroxy)-4,5-dihydro-1*H*-pyrazol |
| "A140" | 3-(2,5-Dichlor-phenyl)-1-[2-(benzo[1,2,5]thiadiazol-5-yl)-acetyl]-4,5-dihydro-1*H*-pyrazol |
| "A141" | 3-(2,5-Dichlor-phenyl)-1-[2-(1*H*-benzimidazol-5-yl)-acetyl]-4,5-dihydro-1*H*-pyrazol |
| "A142" | 3-(4-Fluor-phenyl)-1-[2-(1*H*-benzotriazol-5-yl)-acetyl]-4,5-dihydro-1*H*-pyrazol |
| "A143" | 3-(3-Chlor-6-hydroxy-phenyl)-1-[2-(1*H*-benzotriazol-5-yl)-acetyl]-4,5-dihydro-1*H*-pyrazol |
| "A144" | (3-{2-[3-(4-Fluor-phenyl)-4,5-dihydro-1*H*-pyrazol-1-yl]-2-oxo-ethyl}-phenyl)-carbaminsäure-3-dimethylamino-propylester |
| | 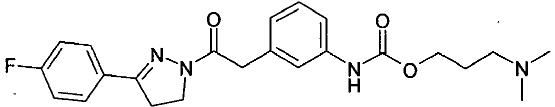 |
| "A145" | 3-(2,5-Difluor-phenyl)-1-[2-(1*H*-benzimidazol-5-yl)-acetyl]-4,5-dihydro-1*H*-pyrazol |
| "A146" | 3-(2,5-Difluor-phenyl)-1-[2-(4,5-dihydro-3-oxo-2H-pyridazin-6-yl)-acetyl]-4,5-dihydro-1*H*-pyrazol |
| | 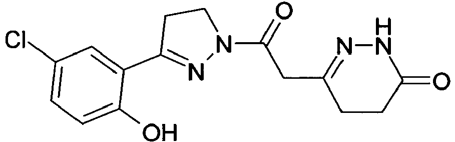 |
| "A147" | 3-(3-Fluor-phenyl)-1-[2-(benzo[1,2,5]thiadiazol-5-yl)-acetyl]-4,5-dihydro-1*H*-pyrazol |
| "A148" | (3-{2-[3-(2,5-Difluor-phenyl)-4,5-dihydro-1*H*-pyrazol-1-yl]-2-oxo-ethyl}-phenyl)-carbaminsäure-3-dimethylamino-propylester |
| "A149" | 1-(4-Hydroxy-benzylcarbonyl)-3-phenyl-4,5-dihydro-1*H*-pyrazol |
| "A150" | (3-{2-[3-(4-Fluor-phenyl)-4,5-dihydro-1*H*-pyrazol-1-yl]-2-oxo-ethyl}-phenyl)-carbaminsäure-3-morpholin-4-yl-propyl ester |
| | 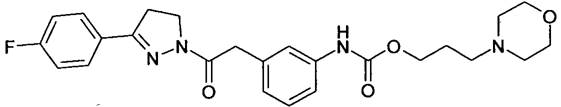 |
| "A151" | 3-(3-Fluor-phenyl)-1-[2-(1*H*-benzimidazol-5-yl)-acetyl]-4,5-dihydro-1*H*-pyrazol |
| "A152" | (3-{2-[3-(2,5-Difluor-phenyl)-4,5-dihydro-1*H*-pyrazol-1-yl]-2-oxo-ethyl}-phenyl)-carbaminsäure-3-morpholin-4-yl-propyl ester |
| "A153" | (3-{2-[3-(2,5-Dichlor-phenyl)-4,5-dihydro-1*H*-pyrazol-1-yl]-2-oxo-ethyl}-phenyl)-carbaminsäure-3-dimethylamino-propylester |
| "A155" | (3-{2-[3-(3-Fluor-phenyl)-4,5-dihydro-1*H*-pyrazol-1-yl]-2-oxo-ethyl}-phenyl)-carbaminsäure-3-dimethylamino-propylester |
| "A156" | (3-{2-[3-(3-Fluor-phenyl)-4,5-dihydro-1*H*-pyrazol-1-yl]-2-oxo-ethyl}-phenyl)-carbaminsäure-3-morpholin-4-yl-propyl ester |
| "A157" | 1-(4-Fluor-benzylcarbonyl)-3-(4-chlor-phenyl)-4,5-dihydro-1*H*-pyrazol |
| "A158" | 3-(4-Chlor-phenyl)-1-[2-(thiophen-2-yl)-acetyl]-4,5-dihydro-1*H*-pyrazol |
| "A157a" | 3-(3,5-Difluor-phenyl)-1-[2-(benzo[1,2,5]thiadiazol-5-yl)-acetyl]-4,5-dihydro-1*H*-pyrazol |
| "A158a" | 3-(3,5-Difluor-phenyl)-1-[2-(1,3-dihydro-2-oxo-benzimidazol-5-yl)-acetyl]-4,5-dihydro-1*H*-pyrazol |
| | 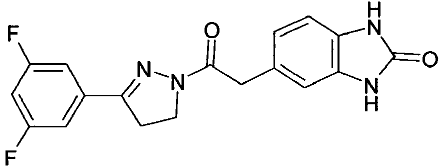 |
| "A159" | 3-(3,5-Difluor-phenyl)-1-[2-(benzimidazol-5-yl)-acetyl]-4,5-dihydro-1*H*-pyrazol |
| "A159a" | 1-(4-Fluor-benzylcarbonyl)-3-(3-methoxy-phenyl)-4,5-dihydro-1*H*-pyrazol |
| "A160" | 1-(3-Methoxy-benzylcarbonyl)-3-(3-methoxyphenyl)-4,5-dihydro-1*H*-pyrazol |
| "A161" | 1-(4-Methoxy-benzylcarbonyl)-3-(3-methoxyphenyl)-4,5-dihydro-1*H*-pyrazol |
| "A162" | 3-(3-Methoxy-phenyl)-1-[3-(3,4,5-trimethoxy-phenyl)-propionyl]-4,5-dihydro-1*H*-pyrazol |
| "A163" | 1-Benzylcarbonyl-3-(3-methoxy-phenyl)-4,5-dihydro-1*H*-pyrazol |
| "A164" | 1-(4-Fluor-benzylcarbonyl)-3-(3-nitro-phenyl)-4,5-dihydro-1*H*-pyrazol |
| "A165" | 1-(3-Methoxy-benzylcarbonyl)-3-(3-nitro-phenyl)-4,5-dihydro-1*H*-pyrazol |
| "A166" | 3-(3-Nitro-phenyl)-1-[2-(thiophen-2-yl)-acetyl]-4,5-dihydro-1*H*-pyrazol |
| "A167" | 1-Benzylcarbonyl-3-(3-nitro-phenyl)-4,5-dihydro-1*H-*pyrazol |
| "A168" | 1-(4-Fluor-benzylcarbonyl)-3-(furan-2-yl)-4,5-dihydro-1*H*-pyrazol |
| "A169" | 1-(3-Methoxy-benzylcarbonyl)-3-(furan-2-yl)-4,5-dihydro-1*H*-pyrazol |
| "A170" | 1-(4-Methoxy-benzylcarbonyl)-3-(furan-2-yl)-4,5-dihydro-1*H*-pyrazol |
| "A171" | 3-(Furan-2-yl)-1-[2-(thiophen-2-yl)-acetyl]-4,5-dihydro-1*H*-pyrazol |
| "A172" | 1-Benzylcarbonyl-3-(furan-2-yl)-4,5-dihydro-1*H-*pyrazol |
| "A173" | 1-Benzylcarbonyl-3-[4-(morpholin-4-yl)-phenyl]-4,5-dihydro-1*H*-pyrazol |
| "A174" | 1-(3-Methoxy-benzylcarbonyl)-3-(2-fluor-phenyl)-4,5-dihydro-1*H*-pyrazol |
| "A175" | 1-(4-Methoxy-benzylcarbonyl)-3-(2-fluor-phenyl)-4,5-dihydro-1*H*-pyrazol |
| "A176" | 1-Benzylcarbonyl-3-(2-fluor-phenyl)-4,5-dihydro-1*H-*pyrazol |
| "A177" | 1-(4-Fluor-benzylcarbonyl)-3-(4-cyan-phenyl)-4,5-dihydro-1*H*-pyrazol |
| "A178" | 1-(3-Methoxy-benzylcarbonyl)-3-(4-cyan-phenyl)-4,5-dihydro-1*H*-pyrazol |
| "A179" | 1-(4-Methoxy-benzylcarbonyl)-3-(4-cyan-phenyl)-4,5-dihydro-1*H*-pyrazol |
| "A180" | 1-Benzylcarbonyl-3-(4-cyan-phenyl)-4,5-dihydro-1*H-*pyrazol |
| "A181" | 1-(4-Fluor-benzylcarbonyl)-3-(3-cyan-phenyl)-4,5-dihydro-1*H*-pyrazol |
| "A182" | 1-(3-Methoxy-benzylcarbonyl)-3-(3-cyan-phenyl)-4,5-dihydro-1*H*-pyrazol |
| "A183" | 1-(4-Methoxy-benzylcarbonyl)-3-(3-cyan-phenyl)-4,5-dihydro-1*H*-pyrazol |
| "A184" | 1-Benzylcarbonyl-3-(3-cyan-phenyl)-4,5-dihydro-1*H-*pyrazol |
| "A185" | 1-(4-Fluor-benzylcarbonyl)-3-(4-methyl-phenyl)-4,5-dihydro-1*H*-pyrazol |
| "A186" | 1-(3-Methoxy-benzylcarbonyl)-3-(4-methyl-phenyl)-4,5-dihydro-1*H*-pyrazol |
| "A187" | 1-Benzylcarbonyl-3-(4-methyl-phenyl)-4,5-dihydro-1*H*-pyrazol |
| "A188" | 1-(4-Fluor-benzylcarbonyl)-3-(3,4-dimethoxy-phenyl)-4,5-dihydro-1*H*-pyrazol |
| "A189" | 1-(3-Methoxy-benzylcarbonyl)-3-(3,4-dimethoxy-phenyl)-4,5-dihydro-1*H*-pyrazol |
| "A190" | 1-Benzylcarbonyl-3-(3,4-dimethoxy-phenyl)-4,5-dihydro-1*H*-pyrazol |
| "A191" | 1-(4-Fluor-benzylcarbonyl)-3-(benzo[1,3]dioxol-5-yl)-4,5-dihydro-1*H*-pyrazol |
| "A192" | 1-(3-Methoxy-benzylcarbonyl)-3-(benzo[1,3]dioxol-5-yl)-4,5-dihydro-1*H*-pyrazol |
| "A193" | 1-(4-Methoxy-benzylcarbonyl)-3-(benzo[1,3]dioxol-5-yl)-4,5-dihydro-1*H*-pyrazol |
| "A194" | 1-[2-(Thiophen-2-yl)-acetyl]-3-(benzo[1,3]dioxol-5-yl)-4,5-dihydro-1*H*-pyrazol |
| "A195" | 1-Benzylcarbonyl-3-(benzo[1,3]dioxol-5-yl)-4,5-dihydro-1*H*-pyrazol |
| "A196" | 1-(4-Fluor-benzylcarbonyl)-3-(4-ethoxy-phenyl)-4,5-dihydro-1*H*-pyrazol |
| "A197" | 1-(3-Methoxy-benzylcarbonyl)-3-(4-ethoxy-phenyl)-4,5-dihydro-1*H*-pyrazol |
| "A198" | 1-(4-Methoxy-benzylcarbonyl)-3-(4-ethoxy-phenyl)-4,5-dihydro-1*H*-pyrazol |
| "A199" | 1-Benzylcarbonyl-3-(4-ethoxy-phenyl)-4,5-dihydro-1*H*-pyrazol |
| "A200" | 1-(4-Fluor-benzylcarbonyl)-3-(thiophen-2-yl)-4,5-dihydro-1*H*-pyrazol |
| "A201" | 1-(4-Methoxy-benzylcarbonyl)-3-(thiophen-2-yl)-4,5-dihydro-1*H*-pyrazol |
| "A202" | 1-[2-(Thiophen-2-yl)-acetyl]-3-(thiophen-2-yl)-4,5-dihydro-1*H*-pyrazol |
| "A203" | 1-Benzylcarbonyl-3-(thiophen-2-yl)-4,5-dihydro-1*H-*pyrazol |
| "A204" | 1-(4-Fluor-benzylcarbonyl)-3-[4-(morpholin-4-yl)-phenyl]-4,5-dihydro-1*H*-pyrazol |
| "A205" | 1-(3-Methoxy-benzylcarbonyl)-3-[4-(morpholin-4-yl)-phenyl]-4,5-dihydro-1*H*-pyrazol |
| "A206" | 1-(4-Methoxy-benzylcarbonyl)-3-[4-(morpholin-4-yl)-phenyl]-4,5-dihydro-1*H*-pyrazol |
| "A207" | 1-[2-(Thiophen-2-yl)-acetyl]-3-[4-(morpholin-4-yl)-phenyl]-4,5-dihydro-1*H*-pyrazol |
| "A208" | 1-(4-Fluor-benzylcarbonyl)-3-(2-fluor-phenyl)-4,5-dihydro-1*H*-pyrazol |
| "A209" | 1-(4-Fluor-benzylcarbonyl)-3-(2,5-dimethoxy-phenyl)-4,5-dihydro-1*H*-pyrazol |
| "A210" | 1-(4-Methoxy-benzylcarbonyl)-3-(2,5-dimethoxy-phenyl)-4,5-dihydro-1*H*-pyrazol |
| "A211" | 1-[2-(Thiophen-2-yl)-acetyl]-3-(2,5-dimethoxy-phenyl)-4,5-dihydro-1*H*-pyrazol |
| "A212" | 1-Benzylcarbonyl-3-(2,5-dimethoxy-phenyl)-4,5-dihydro-1*H*-pyrazol |
| "A213" | 1-[2-(Thiophen-2-yl)-acetyl]-3-(2,3,4-trimethoxy-phenyl)-4,5-dihydro-1*H*-pyrazol |
| "A214" | 1-Benzylcarbonyl-3-(2,3,4-trimethoxy-phenyl)-4,5-dihydro-1*H*-pyrazol |
| "A215" | 1-(4-Fluor-benzylcarbonyl)-3-(4-dimethylaminophenyl)-4,5-dihydro-1*H*-pyrazol |
| "A216" | 1-Benzylcarbonyl-3-(4-dimethylamino-phenyl)-4,5-dihydro-1*H*-pyrazol |
| "A217" | 3-(2,5-Dichlor-phenyl)-1-[2-(2-amino-benzothiazol-6-yl)-propionyl]-4,5-dihydro-1*H*-pyrazol |
| "A218" | 3-(2,5-Dichlor-phenyl)-1-[2-(2-amino-benzothiazol-6-yl)-acetyl]-4,5-dihydro-1*H*-pyrazol |
sowie ihre pharmazeutisch verwendbaren Solvate, Salze, Tautomere und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Solvate, Tautomeren und Stereoisomeren, einschließlich deren Mischungen in allen Verhältnissen, sowie gegebenenfalls Träger- und/oder Hilfsstoffe.

3. Verwendung von Verbindungen gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, wobei die zu behandelnde Krankheit ein fester Tumor oder ein Tumor des Blut- und Immunsystems ist.

4. Verwendung nach Anspruch 3, wobei der feste Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Kehlkopfs und/oder der Lunge stammt.

5. Verwendung nach Anspruch 3, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

6. Verwendung nach Anspruch 3, wobei der feste Tumor aus der Gruppe der Lungenadenokarzinom, kleinzellige Lungenkarzinome, Bauchspeicheldrüsenkrebs, Glioblastome, Kolonkarzinom und Brustkarzinom stammt.

7. Verwendung nach Anspruch 3, wobei der Tumor aus der Gruppe der akuten myelotischen Leukämie, der chronischen myelotischen Leukämie, akuten lymphatischen Leukämie und/oder chronischen lymphatischen Leukämie stammt.

8. Arzneimittel enthaltend mindestens eine Verbindung gemäß Anspruch 1, und/oder ihre pharmazeutisch verwendbaren Salze, Solvate und Stereoisomere, einschließlich deren Mischungen in allen Verhältnissen, und mindestens einen weiteren Arzneimittelwirkstoff.

9. Set (Kit), bestehend aus getrennten Packungen von
(a) einer wirksamen Menge an einer Verbindung gemäß Anspruch 1, und/oder ihrer pharmazeutisch verwendbaren Solvate, Salze und Stereoisomere, einschließlich deren Mischungen in allen Verhältnissen,
und
(b) einer wirksamen Menge eines weiteren Arzneimittelswirkstoffs.

## Claims

1. Compounds selected from the group
| No. | Name |
|---|---|
| "A1" | 1-(Benzo-[1,2,5]thiadiazol-5-ylmethylcarbonyl)-3-(2,4-dimethoxyphenyl)-4,5-dihydro-1*H*-pyrazole |
| "A2" | 1-(Benzo-[1,2,5]thiadiazol-5-ylmethylcarbonyl)-3-(4-fluorophenyl)-4,5-dihydro-1*H*-pyrazole |
| "A3" | 1-(Benzo-[1,2,5]thiadiazol-5-ylmethylcarbonyl)-3-(3,4-dimethoxyphenyl)-4,5-dihydro-1*H*-pyrazole |
| "A4" | 1-(3-Methoxybenzylcarbonyl)-3-(3-chlorophenyl)-4,5-dihydro-1*H*-pyrazole |
| "A5" | 1-(3-Methoxybenzylcarbonyl)-3-(4-chlorophenyl)-4,5-dihydro-1*H*-pyrazole |
| "A6" | 1-(4-Methoxybenzylcarbonyl)-3-(3-chlorophenyl)-4,5-dihydro-1*H*-pyrazole |
| "A7" | 1-(Thiophen-2-ylmethylcarbonyl)-3-(3-chlorophenyl)-4,5-dihydro-1*H*-pyrazole |
| "A8" | 1-Benzylcarbonyl-3-(3-chlorophenyl)-4,5-dihydro-1*H*-pyrazole |
| "A9" | 1-(4-Fluorobenzylcarbonyl)-3-(4-methoxyphenyl)-4,5-dihydro-1*H*-pyrazole |
| "A10" | 1-(3-Methoxybenzylcarbonyl)-3-(4-methoxyphenyl)-4,5-dihydro-1*H*-pyrazole |
| "A11" | 1-(4-Methoxybenzylcarbonyl)-3-(4-methoxyphenyl)-4,5-dihydro-1*H*-pyrazole |
| "A12" | 1-Benzylcarbonyl-3-(4-methoxyphenyl)-4,5-dihydro-1*H*-pyrazole |
| "A13" | 1-(4-Fluorobenzylcarbonyl)-3-(2-methoxyphenyl)-4,5-dihydro-1*H*-pyrazole |
| "A14" | 1-(3-Methoxybenzylcarbonyl)-3-(2-methoxyphenyl)-4,5-dihydro-1*H*-pyrazole |
| "A15" | 1-(4-Methoxybenzylcarbonyl)-3-(2-methoxyphenyl)-4,5-dihydro-1*H*-pyrazole |
| "A16" | 1-(Thiophen-2-ylmethylcarbonyl)-3-(2-methoxyphenyl)-4,5-dihydro-1*H*-pyrazole |
| "A17" | 1-Benzylcarbonyl-3-(2-methoxyphenyl)-4,5-dihydro-1*H*-pyrazole |
| "A18" | 1-(3-Methoxybenzylcarbonyl)-3-(4-dimethylaminophenyl)-4,5-dihydro-1*H*-pyrazole |
| "A19" | 1-(4-Methoxybenzylcarbonyl)-3-(4-dimethylamino-phenyl)-4,5-dihydro-1*H*-pyrazole |
| "A20" | 1-(Thiophen-2-ylmethylcarbonyl)-3-(4-dimethylaminophenyl)-4,5-dihydro-1*H*-pyrazole |
| "A21" | 1-(4-Fluorobenzylcarbonyl)-3-(4-fluorophenyl)-4,5-dihydro-1*H*-pyrazole |
| "A22" | 1-(4-Methoxybenzylcarbonyl)-3-(4-fluorophenyl)-4,5-dihydro-1*H*-pyrazole |
| "A23" | 1-(Thiophen-2-ylmethylcarbonyl)-3-(4-fluorophenyl)-4,5-dihydro-1*H*-pyrazole |
| "A24" | 1-Benzylcarbonyl-3-(4-fluorophenyl)-4,5-dihydro-1*H-*pyrazole |
| "A25" | 1-(Benzo-[1,2,5]thiadiazol-5-ylmethylcarbonyl)-3-(3,4-dimethoxyphenyl)-5,5-dimethyl-4,5-dihydro-1*H-*pyrazole |
| "A26" | 1-(Benzo-[1,2,5]thiadiazol-5-ylmethylcarbonyl)-3-(3,4-dimethoxyphenyl)-4,4-dimethyl-4,5-dihydro-1*H-*pyrazole |
| "A27" | 1-[3-(2-Morpholin-4-ylethoxy)benzylcarbonyl]-3-(5-chlorothiophen-2-yl)-4,5-dihydro-1*H*-pyrazole |
| "A28" | 1-[3-(3-Diethylaminopropoxy)benzylcarbonyl]-3-(5-chlorothiophen-2-yl)-4,5-dihydro-1*H*-pyrazole |
| "A29" | 1-[3-(3-Diethylaminopropoxy)benzylcarbonyl]-3-(3,4-dimethoxyphenyl)-4,5-dihydro-1*H*-pyrazole |
| "A30" | 1-(3-Hyd roxybenzylcarbonyl)-3-(2,4-d imethoxy-phenyl)-4,5-dihydro-1*H*-pyrazole |
| "A31" | 1-(3-Hydroxybenzylcarbonyl)-3-(4-fluorophenyl)-4,5-dihydro-1*H*-pyrazole |
| "A32" | 1-(Benzo-[1,2,5]thiadiazol-5-ylmethylcarbonyl)-3-phenyl-4,5-dihydro-1*H*-pyrazole |
| "A33" | 1-(Benzo-[1,2,5]thiadiazol-5-ylmethylcarbonyl)-3-(4-chlorophenyl)-4,5-dihydro-1*H*-pyrazole |
| "A34" | 1-(Benzo-[1,2,5]thiadiazol-5-ylmethylcarbonyl)-3-(5-chlorothiophen-2-yl)-4,5-dihydro-1*H*-pyrazole |
| "A35" | 1-(Benzo-[1,2,5]thiadiazol-5-ylmethylcarbonyl)-3-(4-bromophenyl)-4,5-dihydro-1*H*-pyrazole |
| "A36" | 1-(3-Hydroxybenzylcarbonyl)-3-(4-chlorophenyl)-4,5-dihydro-1*H*-pyrazole |
| "A37" | 1-(Benzo-[1,2,5]thiadiazol-5-ylmethylcarbonyl)-3-(4-methylsulfonamidophenyl)-4,5-dihydro-1*H*-pyrazole |
| "A38" | 1-(3-Hydroxybenzylcarbonyl)-3-(3,4-dihydro-2-oxo-1*H*-quinazolin-6-yl)-4,5-dihydro-1*H*-pyrazole |
| "A39" | 1-(Benzo-[1,2,5]thiadiazol-5-ylmethylcarbonyl)-3-(3,4-dihydro-2-oxo-1*H*-quinazolin-6-yl)-4,5-dihydro-1*H*-pyrazole |
| "A40" | 1-(Benzo-[1,2,5]thiadiazol-5-ylmethylcarbonyl)-3-(4-hydroxyphenyl)-4,5-dihydro-1*H*-pyrazole |
| "A41" | 1-(Benzo-[1,2,5]thiadiazol-5-ylmethylcarbonyl)-3-(2-hydroxyphenyl)-4,5-dihydro-1*H*-pyrazole |
| "A42" | 1-(Benzo-[1,2,5]thiadiazol-5-ylmethylcarbonyl)-3-(2-hydroxy-5-chlorophenyl)-4,5-dihydro-1*H*-pyrazole |
| "A43" | 1-(Benzo-[1,2,5]thiadiazol-5-ylmethylcarbonyl)-3-(3-chloro-6-methoxyphenyl)-4,5-dihydro-1*H*-pyrazole |
| "A44" | 1-(Benzo-[1,2,5]thiadiazol-5-ylmethylcarbonyl)-3-(3-chloro-4-methoxyphenyl)-4,5-dihydro-1*H*-pyrazole |
| "A45" | 1-(3-Hydroxybenzylcarbonyl)-3-(5-chlorothiophen-2-yl)-4,5-dihydro-1*H*-pyrazole |
| "A46" | 1-(3-Methoxybenzylcarbonyl)-3-(5-chlorothiophen-2-yl)-4,5-dihydro-1*H*-pyrazole |
| "A47" | 1-[3-(3-Oxomorpholin-4-yl)benzylcarbonyl]-3-(3,4-dimethoxyphenyl)-4,5-dihydro-1*H*-pyrazole |
| "A48" | 1-(Phenethylcarbonyl)-3-(3,4-dimethoxyphenyl)-4,5-dihydro-1*H*-pyrazole |
| "A49" | 1-[4-(Morpholin-4-ylcarbonyl)phenylmethylcarbonyl]-3-(3,4-dimethoxyphenyl)-4,5-dihydro-1*H*-pyrazole |
| "A50" | 1-[3-(3-Diethylaminopropoxycarbonylamino)phenyl-methylcarbonyl]-3-(3,4-dimethoxyphenyl)-4,5-dihydro-1*H*-pyrazole |
| "A51" | 1-{3-[3-(3-Diethylaminopropyl)ureido]phenylmethyl-carbonyl}-3-(3,4-dimethoxyphenyl)-4,5-dihydro-1*H-*pyrazole |
| "A52" | 1-Benzylcarbonyl-3-(2,4-dimethoxyphenyl)-4,5-dihydro-1*H*-pyrazole |
| "A53" | 1-(3-Methoxybenzylcarbonyl)-3-(2,4-dimethoxy-phenyl)-4,5-dihydro-1*H*-pyrazole |
| "A54" | 1-(3-Methoxybenzylcarbonyl)-3-(4-fluorophenyl)-4,5-dihydro-1*H*-pyrazole |
| "A55" | 1-(3-Methoxybenzylcarbonyl)-3-(2,5-dimethoxy-phenyl)-4,5-dihydro-1*H*-pyrazole |
| "A56" | 1-(Benzo-[1,2,5]thiadiazol-5-ylmethylcarbonyl)-3-(4-methylsulfanilephenyl)-4,5-dihydro-1*H*-pyrazole |
| "A57" | 1-(Benzo-[1,2,5]thiadiazol-5-ylmethylcarbonyl)-3-(dibenzofuran-2-yl)-4,5-dihydro-1*H*-pyrazole |
| "A58" | 1-(4-Hydroxybenzylcarbonyl)-3-(4-chlorophenyl)-4,5-dihydro-1*H*-pyrazole |
| "A59" | 1-[4-(Morpholin-4-ylethoxy)benzylcarbonyl]-3-(3,4-dimethoxyphenyl)-4,5-dihydro-1*H*-pyrazole |
| "A60" | 1-[4-(3-Diethylaminopropoxy)benzylcarbonyl]-3-(5-chlorothiophen-2-yl)-4,5-dihydro-1*H*-pyrazole |
| "A61" | 1-(4-Hydroxybenzylcarbonyl)-3-(2,4-dimethoxy-phenyl)-4,5-dihydro-1*H*-pyrazole |
| "A62" | 1-(3-Hydroxybenzylcarbonyl)-3-(3,4-dimethoxy-phenyl)-4,5-dihydro-1*H*-pyrazole |
| "A63" | 1-(3-Chlorobenzylcarbonyl)-3-(2,4-dimethoxy-phenyl)-4,5-dihydro-1*H*-pyrazole |
| "A64" | 1-(2-Benzo-[1,2,5]thiadiazol-5-ylpropionyl)-3-(4-fluorophenyl)-4,5-dihydro-1*H*-pyrazole |
| "A65" | 1-[2-Hydroxy-2-(3-hydroxyphenyl)acetyl]-3-(4-fluorophenyl)-4,5-dihydro-1*H*-pyrazole |
| "A66" | 1-[2-Hydroxy-2-(3-hydroxyphenyl)acetyl]-3-(4-chloro-phenyl)-4,5-dihydro-1*H*-pyrazole |
| "A67" | 1-(2-Hydroxybenzylcarbonyl)-3-(2,4-dimethoxy-phenyl)-4,5-dihydro-1*H*-pyrazole |
| "A68" | 1-(3-Hydroxybenzylcarbonyl)-3-(2-oxo-1,3-dihydro-indol-5-yl)-4,5-dihydro-1*H*-pyrazole |
| "A69" | 1-(4-Hydroxybenzylcarbonyl)-3-(4-fluorophenyl)-4,5-dihydro-1*H*-pyrazole |
| "A70" | 1-(2-Hydroxybenzylcarbonyl)-3-(4-fluorophenyl)-4,5-dihydro-1*H*-pyrazole |
| "A71" | 1-[4-(Morpholin-4-ylethoxy)benzylcarbonyl]-3-(5-chlorothiophen-2-yl)-4,5-dihydro-1*H*-pyrazole |
| "A72" | 1-(4-Fluorobenzylcarbonyl)-3-(5-chlorothiophen-2-yl)-4,5-dihydro-1*H*-pyrazole |
| "A73" | 1-(4-Methoxybenzylcarbonyl)-3-(5-chlorothiophen-2-yl)-4,5-dihydro-1*H*-pyrazole |
| "A74" | 1-(4-Acetamidobenzylcarbonyl)-3-(5-chlorothiophen-2-yl)-4,5-dihydro-1*H*-pyrazole |
| "A75" | 1-(Benzo-[1,2,5]thiadiazol-5-ylmethylcarbonyl)-3-(5-chlorofuran-2-yl)-4,5-dihydro-1*H*-pyrazole |
| "A76" | 1-(4-Hydroxy-3-methoxybenzylcarbonyl)-3-(5-chloro-thiophen-2-yl)-4,5-dihydro-1*H*-pyrazole |
| "A77" | 1-(4-Methylsulfonylbenzylcarbonyl)-3-(5-chlorothio-phen-2-yl)-4,5-dihydro-1*H*-pyrazole |
| "A78" | 1-(4-Ethoxybenzylcarbonyl)-3-(5-chlorothiophen-2-yl)-4,5-dihydro-1*H*-pyrazole |
| "A79" | 1-(4-Hydroxy-3-chlorobenzylcarbonyl)-3-(5-chloro-thiophen-2-yl)-4,5-dihydro-1*H*-pyrazole |
| "A80" | 1-(2-(4-Hydroxyphenyl)propionyl)-3-(5-chlorothio-phen-2-yl)-4,5-dihydro-1*H*-pyrazole |
| "A81" | 1-(2-(Benzo-[1,2,5]thiadiazol-5-yl)propionyl)-3-(5-chlorothiophen-2-yl)-4,5-dihydro-1*H*-pyrazole |
| "A82" | 1-(2-(4-Fluorophenyl)propionyl)-3-(5-chlorothiophen-2-yl)-4,5-dihydro-1*H*-pyrazole |
| "A83" | 1-(Benzo-[1,2,5]thiadiazol-5-ylmethylcarbonyl)-3-(3-amino-1*H*-indazol-5-yl)-4,5-dihydro-1*H*-pyrazole |
| "A84" | 1-(Benzo-[1,2,5]thiadiazol-5-ylmethylcarbonyl)-3-(2-hydroxyphenyl)-4,5-dihydro-1*H*-pyrazole |
| "A85" | 1-(3-Chlorobenzylcarbonyl)-3-(3,4-dimethoxy-phenyl)-4,5-dihydro-1*H*-pyrazole |
| "A86" | 1-(Benzo-[1,2,5]thiadiazol-5-ylmethylcarbonyl)-3-(2-oxo-3,4-dihydro-1*H*-quinazolin-6-yl)-4,5-dihydro-1*H-*pyrazole |
| "A87" | 1-(Benzo-[1,2,5]thiadiazol-5-ylmethylcarbonyl)-3-(4-hydroxyphenyl)-4,5-dihydro-1*H*-pyrazole |
| "A88" | 1-(3-Hydroxybenzylcarbonyl)-3-(2-oxo-3,4-dihydro-1*H*-quinazolin-6-yl)-4,5-dihydro-1*H*-pyrazole |
| "A89" | 1-(Benzo-[1,2,5]thiadiazol-5-ylmethylcarbonyl)-3-(3-chloro-6-hydroxyphenyl)-4,5-dihydro-1*H*-pyrazole |
| "A90" | 1-(3-Hydroxybenzylcarbonyl)-3-(5-chlorothiophen-2-yl)-4,5-dihydro-1*H*-pyrazole |
| "A91" | 1-(3-Hyd roxybenzylcarbonyl)-3-(4-hyd roxyphenyl)-4,5-dihydro-1*H*-pyrazole |
| "A92" | 1-(Benzo-[1,3]dioxol-5-ylmethylcarbonyl)-3-(5-chlorothiophen-2-yl)-4,5-dihydro-1*H*-pyrazole |
| "A93" | 1-(4-Hydroxybenzylcarbonyl)-3-(5-chlorothiophen-2-yl)-4,5-dihydro-1*H*-pyrazole |
| "A94" | 1-(4-Methoxybenzylcarbonyl)-3-(5-chlorothiophen-2-yl)-4,5-dihydro-1*H*-pyrazole |
| "A95" | 1-(Benzo-[1,2,5]thiadiazol-5-ylmethylcarbonyl)-3-(3-chloro-6-methoxyphenyl)-4,5-dihydro-1*H*-pyrazole |
| "A96" | 1-(4-Hydroxybenzylcarbonyl)-3-(3,4-dimethoxy-phenyl)-4,5-dihydro-1*H*-pyrazole |
| "A97" | 1-(3-Chloro-4-hydroxybenzylcarbonyl)-3-(5-chloro-thiophen-2-yl)-4,5-dihydro-1*H*-pyrazole |
| "A98" | 3-(5-Chlorothiophen-2-yl)-1-[2-(4-hydroxyphenyl)-propionyl]-4,5-dihydro-1*H*-pyrazole |
| | 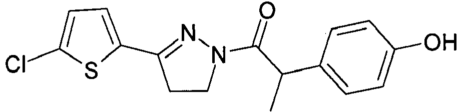 |
| "A99" | 1-(4-Methoxybenzylcarbonyl)-3-(3,4-dimethoxy-phenyl)-4,5-dihydro-1*H*-pyrazole |
| "A100" | 3-(2-Oxo-3,4-dihydro-1*H*-quinazolin-6-yl)-1-[2-(4-hydroxyphenyl)propionyl]-4,5-dihydro-1*H*-pyrazole |
| | 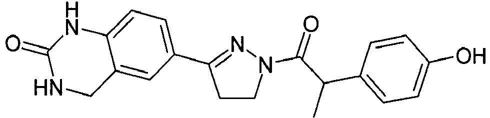 |
| "A101" | 1-[3-(3-Oxomorpholin-4-yl)benzylcarbonyl]-3-(5-chlorothiophen-2-yl)-4,5-dihydro-1*H*-pyrazole |
| | 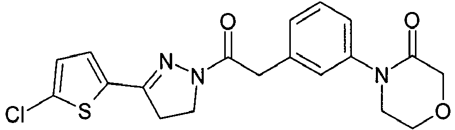 |
| "A102" | 1-[3-(3-Oxomorpholin-4-yl)benzylcarbonyl]-3-(3,4-dimethoxyphenyl)-4,5-dihydro-1*H*-pyrazole |
| "A103" | 3-(3-Chloro-6-hydroxyphenyl)-1-[2-(benzo-1,2,5-thiadiazol-5-yl)propionyl]-4,5-dihydro-1*H*-pyrazole |
| | 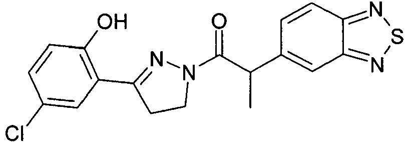 |
| "A104" | 1-[4-(3-Morpholin-4-ylpropoxy)benzylcarbonyl]-3-(5-chlorothiophen-2-yl)-4,5-dihydro-1*H*-pyrazole |
| | 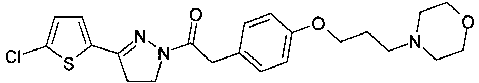 |
| "A105" | 1-[2-(1*H*-Benzimidazol-5-yl)acetyl]-3-(5-chlorothio-phen-2-yl)-4,5-dihydro-1*H*-pyrazole |
| | 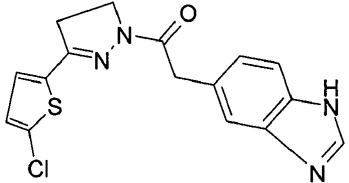 |
| "A106" | 3-(4-Chlorophenyl)-1-(3-phenylpropionyl)-4,5-dihydro-1*H*-pyrazole |
| | 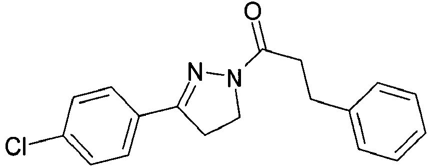 |
| "A107" | 1-(4-Methylsulfonylbenzylcarbonyl)-3-(4-chloro-phenyl)-4,5-dihydro-1*H*-pyrazole |
| "A108" | 1-[2-(1*H*-Benzimidazol-5-yl)acetyl]-3-(3-chloro-6-hydroxyphenyl)-4,5-dihydro-1*H*-pyrazole |
| "A109" | 1-[3-(2-Morpholin-4-ylethoxy)benzylcarbonyl]-3-(5-chlorothiophen-2-yl)-4,5-dihydro-1*H*-pyrazole |
| "A110" | 1-(Benzo-[1,3]dioxol-5-ylmethylcarbonyl)-3-(3-chloro-6-hydroxyphenyl)-4,5-dihydro-1*H*-pyrazole |
| "A111" | 3-(4-Chlorophenyl)-1-[2-(4-hydroxyphenyl)-propionyl]-4,5-dihydro-1*H*-pyrazole |
| "A112" | 1-[2-(1*H*-Benzimidazol-5-yl)acetyl]-3-(4-chlorophenyl)-4,5-dihydro-1*H*-pyrazole |
| "A113" | 1-(3-Chloro-4-hydroxybenzylcarbonyl)-3-(3-chloro-6-hydroxyphenyl)-4,5-dihydro-1*H*-pyrazole |
| "A114" | 1-(4-Hydroxybenzylcarbonyl)-3-(3-chloro-6-methoxyphenyl)-4,5-dihydro-1*H*-pyrazole |
| "A115" | 1-(Benzo-[1,3]dioxol-5-ylmethylcarbonyl)-3-(2-oxo-3,4-dihydro-1H-quinazolin-6-yl)-4,5-dihydro-1*H-*pyrazole |
| "A116" | 3-(3,4-Dimethoxyphenyl)-1-[2-(benzo-[1,2,5]thiadiazol-5-yl)acetyl]-5,5-dimethyl-4,5-dihydro-1*H*-pyrazole |
| "A117" | 1-{4-[3-(N,N-Diethoxyamino)propoxy]-benzylcarbonyl}-3-(4-chlorophenyl)-4,5-dihydro-1*H-*pyrazole |
| "A118" | 3-(5-Chlorothiophen-2-yl)-1-[2-(quinoxalin-6-yl)-acetyl]-4,5-dihydro-1*H*-pyrazole |
| "A119" | 3-(3-Chloro-6-hydroxyphenyl)-1-[2-(quinoxalin-6-yl)-acetyl]-4,5-dihydro-1*H*-pyrazole |
| "A120" | 3-(3-Chloro-6-hydroxyphenyl)-1-[2-(4-hydroxyphenyl)propionyl]-4,5-dihydro-1*H*-pyrazole |
| "A121" | 1-(4-Hydroxybenzylcarbonyl)-3-(3-chloro-6-hydroxyphenyl)-4,5-dihydro-1*H*-pyrazole |
| "A122" | 1-(3-Chlorobenzylcarbonyl)-3-(3-chloro-6-hydroxyphenyl)-4,5-dihydro-1*H*-pyrazole |
| "A123" | 1-(2-{4-[3-(N,N-Diethoxyamino)propoxy]phenyl}-propionyl)-3-(4-chlorophenyl)-4,5-dihydro-1*H-*pyrazole |
| | 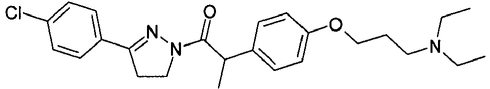 |
| "A124" | 1-[4-(Morpholin-4-ylcarbonyl)benzylcarbonyl]-3-(5-chlorothiophen-2-yl)-4,5-dihydro-1*H*-pyrazole |
| "A125" | 1-[4-(1-Methylpiperazin-4-ylcarbonyl)-benzylcarbonyl]-3-(5-chlorothiophen-2-yl)-4,5-dihydro-1*H*-pyrazole |
| "A126" | 1-[2-(Pyridin-3-yl)acetyl]-3-(3-chloro-6-hydroxyphenyl)-4,5-dihydro-1*H*-pyrazole |
| "A127" | 1-[2-(Pyrid in-2-yl)acetyl]-3-(3-ch loro-6-hyd roxy-phenyl)-4,5-dihydro-1*H*-pyrazole |
| "A128" | 1-(4-Cyanobenzylcarbonyl)-3-(5-chlorothiophen-2-yl)-4,5-dihydro-1*H*-pyrazole |
| "A129" | 1-[2-(Pyridin-3-yl)acetyl]-3-(5-chlorothiophen-2-yl)-4,5-dihydro-1*H*-pyrazole |
| "A130" | 1-[2-(Pyridin-2-yl)acetyl]-3-(5-chlorothiophen-2-yl)-4,5-dihydro-1*H*-pyrazole |
| "A131" | 1-[2-(1*H*-Benzimidazol-5-yl)acetyl]-3-(3-chlorophenyl)-4,5-dihydro-1*H*-pyrazole |
| "A132" | 1-(9*H*-Carbazol-3-ylmethylcarbonyl)-3-(3-chloro-6-hydroxyphenyl)-4,5-dihydro-1*H*-pyrazole |
| "A133" | 3-(3-Chlorophenyl)-1-[2-(benzo-1,2,5-thiadiazol-5-yl)acetyl]-4,5-dihydro-1*H*-pyrazole |
| "A134" | 1-{2-[2-(5-Chlorothiophen-2-yl)-(1*H*-benzimidazol-5-yl]acetyl}-3-(5-chlorothiophen-2-yl)-4,5-dihydro-1*H-*pyrazole |
| | 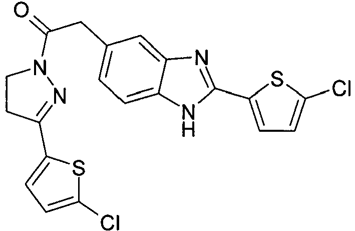 |
| "A135" | 1-{2-[2-(5-Chlorothiophen-2-yl)-(1*H*-benzimidazol-5-yl]acetyl}-3-(3-chloro-6-hydroxyphenyl)-4,5-dihydro-1*H*-pyrazole |
| "A136" | 3-(3-Chloro-6-hydroxyphenyl)-1-[2-(2-amino-benzothiazol-6-yl)propionyl]-4,5-dihydro-1*H*-pyrazole |
| | 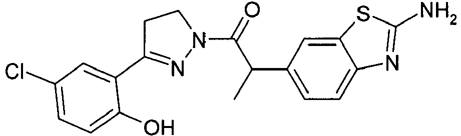 |
| "A137" | 3-(4-Fluorophenyl)-1-[2-(2-aminobenzothiazol-6-yl)-propionyl]-4,5-dihydro-1*H*-pyrazole |
| "A138" | 3-(4-Fluorophenyl)-1-[2-(2-aminobenzimidazol-6-yl)-acetyl]-4,5-dihydro-1*H*-pyrazole |
| "A139" | 1-(3-Nitrobenzylcarbonyl)-3-(3-chloro-6-hydroxy)-4,5-dihydro-1*H*-pyrazole |
| "A140" | 3-(2,5-Dichlorophenyl)-1-[2-(benzo-1,2,5-thiadiazol-5-yl)acetyl]-4,5-dihydro-1*H*-pyrazole |
| "A141" | 3-(2,5-Dichlorophenyl)-1-[2-(1*H*-benzimidazol-5-yl)-acetyl]-4,5-dihydro-1*H*-pyrazole |
| "A142" | 3-(4-Fluorophenyl)-1-[2-(1*H*-benzotriazol-5-yl)-acetyl]-4,5-dihydro-1*H*-pyrazole |
| "A143" | 3-(3-Chloro-6-hydroxyphenyl)-1-[2-(1*H*-benzotriazol-5-yl)acetyl]-4,5-dihydro-1*H*-pyrazole |
| "A144" | 3-Dimethylaminopropyl (3-{2-[3-(4-fluorophenyl)-4,5-dihydro-1*H*-pyrazol-1-yl]-2-oxoethyl}phenyl)-carbamate |
| | 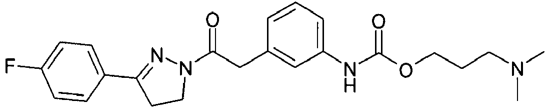 |
| "A145" | 3-(2,5-Difluorophenyl)-1-[2-(1*H*-benzimidazol-5-yl)-acetyl]-4,5-dihydro-1*H*-pyrazole |
| "A146" | 3-(2,5-Difluorophenyl)-1-[2-(4,5-dihydro-3-oxo-2H-pyridazin-6-yl)acetyl]-4,5-dihydro-1*H*-pyrazole |
| | 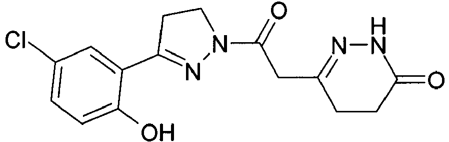 |
| "A147" | 3-(3-Fluorophenyl)-1-[2-(benzo-1,2,5-thiadiazol-5-yl)acetyl]-4,5-dihydro-1*H*-pyrazole |
| "A148" | 3-Dimethylaminopropyl (3-{2-[3-(2,5-difluorophenyl)-4,5-dihydro-1*H*-pyrazol-1-yl]-2-oxoethyl}phenyl)-carbamate |
| "A149" | 1-(4-Hydroxybenzylcarbonyl)-3-phenyl-4,5-dihydro-1*H*-pyrazole |
| "A150" | 3-Morpholin-4-ylpropyl (3-{2-[3-(4-fluorophenyl)-4,5-dihydro-1*H*-pyrazol-1-yl]-2-oxoethyl}phenyl)-carbamate |
| | 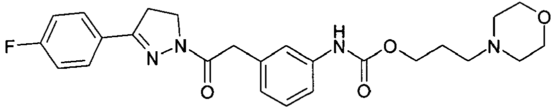 |
| "A151" | 3-(3-Fluorophenyl)-1-[2-(1*H*-benzimidazol-5-yl)-acetyl]-4,5-dihydro-1*H*-pyrazole |
| "A152" | 3-Morpholin-4-ylpropyl (3-{2-[3-(2,5-difluorophenyl)-4,5-dihydro-1*H*-pyrazol-1-yl]-2-oxoethyl}phenyl)-carbamate |
| "A153" | 3-Dimethylaminopropyl (3-{2-[3-(2,5-dichlorophenyl)-4,5-dihydro-1*H*-pyrazol-1-yl]-2-oxoethyl}phenyl)-carbamate |
| "A155" | 3-Dimethylaminopropyl (3-{2-[3-(3-fluorophenyl)-4,5-dihydro-1*H*-pyrazol-1-yl]-2-oxoethyl}phenyl)-carbamate |
| "A156" | 3-Morpholin-4-ylpropyl (3-{2-[3-(3-fluorophenyl)-4,5-dihydro-1*H*-pyrazol-1-yl]-2-oxoethyl}phenyl)-carbamate |
| "A157" | 1-(4-Fluorobenzylcarbonyl)-3-(4-chlorophenyl)-4,5-dihydro-1*H*-pyrazole |
| "A158" | 3-(4-Chlorophenyl)-1-[2-(thiophen-2-yl)acetyl]-4,5-dihydro-1*H*-pyrazole |
| "A157a" | 3-(3,5-Difluorophenyl)-1-[2-(benzo-1,2,5-thiadiazol-5-yl)acetyl]-4,5-dihydro-1*H*-pyrazole |
| "A158a" | 3-(3,5-Difluorophenyl)-1-[2-(1,3-dihydro-2-oxo-benzimidazol-5-yl)acetyl]-4,5-dihydro-1*H*-pyrazole |
| | 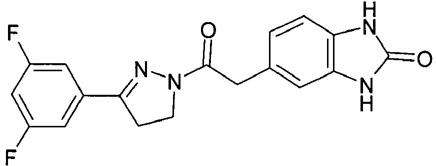 |
| "A159" | 3-(3,5-Difluorophenyl)-1-[2-(benzimidazol-5-yl)-acetyl]-4,5-dihydro-1*H*-pyrazole |
| "A159a" | 1-(4-Fluorobenzylcarbonyl)-3-(3-methoxyphenyl)-4,5-dihydro-1*H*-pyrazole |
| "A160" | 1-(3-Methoxybenzylcarbonyl)-3-(3-methoxyphenyl)-4,5-dihydro-1*H*-pyrazole |
| "A161" | 1-(4-Methoxybenzylcarbonyl)-3-(3-methoxyphenyl)-4,5-dihydro-1*H*-pyrazole |
| "A162" | 3-(3-Methoxyphenyl)-1-[3-(3,4,5-trimethoxyphenyl)-propionyl]-4,5-dihydro-1*H*-pyrazole |
| "A163" | 1-Benzylcarbonyl-3-(3-methoxyphenyl)-4,5-dihydro-1*H*-pyrazole |
| "A164" | 1-(4-Fluorobenzylcarbonyl)-3-(3-nitrophenyl)-4,5-dihydro-1*H*-pyrazole |
| "A165" | 1-(3-Methoxybenzylcarbonyl)-3-(3-nitrophenyl)-4,5-dihydro-1*H*-pyrazole |
| "A166" | 3-(3-Nitrophenyl)-1-[2-(thiophen-2-yl)acetyl]-4,5-dihydro-1*H*-pyrazole |
| "A167" | 1-Benzylcarbonyl-3-(3-nitrophenyl)-4,5-dihydro-1*H-*pyrazole |
| "A168" | 1-(4-Fluorobenzylcarbonyl)-3-(furan-2-yl)-4,5-dihydro-1*H*-pyrazole |
| "A169" | 1-(3-Methoxybenzylcarbonyl)-3-(furan-2-yl)-4,5-dihydro-1*H*-pyrazole |
| "A170" | 1-(4-Methoxybenzylcarbonyl)-3-(furan-2-yl)-4,5-dihydro-1*H*-pyrazole |
| "A171" | 3-(Furan-2-yl)-1-[2-(thiophen-2-yl)acetyl]-4,5-dihydro-1*H*-pyrazole |
| "A172" | 1-Benzylcarbonyl-3-(furan-2-yl)-4,5-dihydro-1*H-*pyrazole |
| "A173" | 1-Benzylcarbonyl-3-[4-(morpholin-4-yl)phenyl]-4,5-dihydro-1*H*-pyrazole |
| "A174" | 1-(3-Methoxybenzylcarbonyl)-3-(2-fluorophenyl)-4,5-dihydro-1*H*-pyrazole |
| "A175" | 1-(4-Methoxybenzylcarbonyl)-3-(2-fluorophenyl)-4,5-dihydro-1*H*-pyrazole |
| "A176" | 1-Benzylcarbonyl-3-(2-fluorophenyl)-4,5-dihydro-1*H-*pyrazole |
| "A177" | 1-(4-Fluorobenzylcarbonyl)-3-(4-cyanophenyl)-4,5-dihydro-1*H*-pyrazole |
| "A178" | 1-(3-Methoxybenzylcarbonyl)-3-(4-cyanophenyl)-4,5-dihydro-1*H*-pyrazole |
| "A179" | 1-(4-Methoxybenzylcarbonyl)-3-(4-cyanophenyl)-4,5-dihydro-1*H*-pyrazole |
| "A180" | 1-Benzylcarbonyl-3-(4-cyanophenyl)-4,5-dihydro-1*H*-pyrazole |
| "A181" | 1-(4-Fluorobenzylcarbonyl)-3-(3-cyanophenyl)-4,5-dihydro-1*H*-pyrazole |
| "A182" | 1-(3-Methoxybenzylcarbonyl)-3-(3-cyanophenyl)-4,5-dihydro-1*H*-pyrazole |
| "A183" | 1-(4-Methoxybenzylcarbonyl)-3-(3-cyanophenyl)-4,5-dihydro-1*H*-pyrazole |
| "A184" | 1-Benzylcarbonyl-3-(3-cyanophenyl)-4,5-dihydro-1*H*-pyrazole |
| "A185" | 1-(4-Fluorobenzylcarbonyl)-3-(4-methylphenyl)-4,5-dihydro-1*H*-pyrazole |
| "A186" | 1-(3-Methoxybenzylcarbonyl)-3-(4-methylphenyl)-4,5-dihydro-1*H*-pyrazole |
| "A187" | 1-Benzylcarbonyl-3-(4-methylphenyl)-4,5-dihydro-1*H*-pyrazole |
| "A188" | 1-(4-Fluorobenzylcarbonyl)-3-(3,4-dimethoxy-phenyl)-4,5-dihydro-1*H*-pyrazole |
| "A189" | 1-(3-Methoxybenzylcarbonyl)-3-(3,4-dimethoxy-phenyl)-4,5-dihydro-1*H*-pyrazole |
| "A190" | 1-Benzylcarbonyl-3-(3,4-dimethoxyphenyl)-4,5-dihydro-1*H*-pyrazole |
| "A191" | 1-(4-Fluorobenzylcarbonyl)-3-(benzo-[1,3]dioxol-5-yl)-4,5-dihydro-1*H*-pyrazole |
| "A192" | 1-(3-Methoxybenzylcarbonyl)-3-(benzo-[1,3]dioxol-5-yl)-4,5-dihydro-1*H*-pyrazole |
| "A193" | 1-(4-Methoxybenzylcarbonyl)-3-(benzo-[1,3]dioxol-5-yl)-4,5-dihydro-1*H*-pyrazole |
| "A194" | 1-[2-(Thiophen-2-yl)acetyl]-3-(benzo-[1,3]dioxol-5-yl)-4,5-dihydro-1*H*-pyrazole |
| "A195" | 1-Benzylcarbonyl-3-(benzo-[1,3]dioxol-5-yl)-4,5-dihydro-1*H*-pyrazole |
| "A196" | 1-(4-Fluorobenzylcarbonyl)-3-(4-ethoxyphenyl)-4,5-dihydro-1*H*-pyrazole |
| "A197" | 1-(3-Methoxybenzylcarbonyl)-3-(4-ethoxyphenyl)-4,5-dihydro-1*H*-pyrazole |
| "A198" | 1-(4-Methoxybenzylcarbonyl)-3-(4-ethoxyphenyl)-4,5-dihydro-1*H*-pyrazole |
| "A199" | 1-Benzylcarbonyl-3-(4-ethoxyphenyl)-4,5-dihydro-1*H*-pyrazole |
| "A200" | 1-(4-Fluorobenzylcarbonyl)-3-(thiophen-2-yl)-4,5-dihydro-1*H*-pyrazole |
| "A201" | 1-(4-Methoxybenzylcarbonyl)-3-(thiophen-2-yl)-4,5-dihydro-1*H*-pyrazole |
| "A202" | 1-[2-(Thiophen-2-yl)acetyl]-3-(thiophen-2-yl)-4,5-dihydro-1*H*-pyrazole |
| "A203" | 1-Benzylcarbonyl-3-(thiophen-2-yl)-4,5-dihydro-1*H-*pyrazole |
| "A204" | 1-(4-Fluorobenzylcarbonyl)-3-[4-(morpholin-4-yl)-phenyl]-4,5-dihydro-1*H*-pyrazole |
| "A205" | 1-(3-Methoxybenzylcarbonyl)-3-[4-(morpholin-4-yl)-phenyl]-4,5-dihydro-1H-pyrazole |
| "A206" | 1-(4-Methoxybenzylcarbonyl)-3-[4-(morpholin-4-yl)-phenyl]-4,5-dihydro-1H-pyrazole |
| "A207" | 1-[2-(Thiophen-2-yl)acetyl]-3-[4-(morpholin-4-yl)-phenyl]-4,5-dihydro-1H-pyrazole |
| "A208" | 1-(4-Fluorobenzylcarbonyl)-3-(2-fluorophenyl)-4,5-dihydro-1H-pyrazole |
| "A209" | 1-(4-Fluorobenzylcarbonyl)-3-(2,5-dimethoxy-phenyl)-4,5-dihydro-1*H*-pyrazole |
| "A210" | 1-(4-Methoxybenzylcarbonyl)-3-(2,5-dimethoxyphenyl)-4,5-dihydro-1*H*-pyrazole |
| "A211" | 1-[2-(Thiophen-2-yl)acetyl]-3-(2,5-dimethoxyphenyl)-4,5-dihydro-1*H*-pyrazole |
| "A212" | 1-Benzylcarbonyl-3-(2,5-dimethoxyphenyl)-4,5-dihydro-1*H*-pyrazole |
| "A213" | 1-[2-(Thiophen-2-yl)acetyl]-3-(2,3,4-trimethoxy-phenyl)-4,5-dihydro-1*H*-pyrazole |
| "A214" | 1-Benzylcarbonyl-3-(2,3,4-trimethoxyphenyl)-4,5-dihydro-1*H*-pyrazole |
| "A215" | 1-(4-Fluorobenzylcarbonyl)-3-(4-dimethylaminophenyl)-4,5-dihydro-1*H*-pyrazole |
| "A216" | 1-Benzylcarbonyl-3-(4-dimethylaminophenyl)-4,5-dihydro-1*H*-pyrazole |
| "A217" | 3-(2,5-Dichlorophenyl)-1-[2-(2-aminobenzothiazol-6-yl)propionyl]-4,5-dihydro-1*H*-pyrazole |
| "A218" | 3-(2,5-Dichlorophenyl)-1-[2-(2-aminobenzothiazol-6-yl)acetyl]-4,5-dihydro-1*H*-pyrazole |
and pharmaceutically usable solvates, salts, tautomers and stereoisomers thereof, including mixtures thereof in all ratios.

2. Medicaments comprising at least one compound according to Claim 1 and/or pharmaceutically usable salts, solvates, tautomers and stereoisomers thereof, including mixtures thereof in all ratios, and optionally excipients and/or adjuvants.

3. Use of compounds according to Claim 1, and pharmaceutically usable salts, solvates and stereoisomers thereof, including mixtures thereof in all ratios,
for the preparation of a medicament for the treatment of diseases,
where the disease to be treated is a solid tumour or a tumour of the blood and immune system.

4. Use according to Claim 3, where the solid tumour originates from the group of tumours of the squamous epithelium, the bladder, the stomach, the kidneys, of head and neck, the oesophagus, the cervix, the thyroid, the intestine, the liver, the brain, the prostate, the urogenital tract, the lymphatic system, the larynx and/or the lung.

5. Use according to Claim 3, where the solid tumour originates from the group monocytic leukaemia, lung adenocarcinoma, small-cell lung carcinomas, pancreatic cancer, glioblastomas and breast carcinoma.

6. Use according to Claim 3, where the solid tumour originates from the group of lung adenocarcinoma, small-cell lung carcinomas, pancreatic cancer, glioblastomas, colon carcinoma and breast carcinoma.

7. Use according to Claim 3, where the tumour originates from the group of acute myeloid leukaemia, chronic myeloid leukaemia, acute lymphatic leukaemia and/or chronic lymphatic leukaemia.

8. Medicaments comprising at least one compound according to Claim 1, and/or pharmaceutically usable salts, solvates and stereoisomers thereof, including mixtures thereof in all ratios, and at least one further medicament active compound.

9. Set (kit) consisting of separate packs of
(a) an effective amount of a compound according to Claim 1, and/or pharmaceutically usable solvates, salts and stereoisomers thereof, including mixtures thereof in all ratios,
and
(b) an effective amount of a further medicament active compound.

## Revendications

1. Composés choisis dans le groupe constitué par
| n° | Nom |
|---|---|
| « A1 » | 1-(Benzo-[1,2,5]thiadiazol-5-ylméthylcarbonyl)-3-(2,4-diméthoxyphényl)-4,5-dihydro-1*H*-pyrazole |
| « A2 » | 1-(Benzo-[1,2,5]thiadiazol-5-ylméthylcarbonyl)-3-(4-fluorophényl)-4,5-dihydro-1*H*-pyrazole |
| « A3 » | 1-(Benzo-[1,2,5]thiadiazol-5-ylméthylcarbonyl)-3-(3,4-diméthoxyphényl)-4,5-dihydro-1*H*-pyrazole |
| « A4 » | 1-(3-Méthoxybenzylcarbonyl)-3-(3-chlorophényl)-4,5-dihydro-1*H*-pyrazole |
| « A5 » | 1-(3-Méthoxybenzylcarbonyl)-3-(4-chlorophényl)-4,5-dihydro-1*H*-pyrazole |
| « A6 » | 1-(4-Méthoxybenzylcarbonyl)-3-(3-chlorophényl)-4,5-dihydro-1*H*-pyrazole |
| « A7 » | 1-(Thiophén-2-ylméthylcarbonyl)-3-(3-chlorophényl)-4,5-dihydro-1*H*-pyrazole |
| « A8 » | 1-Benzylcarbonyl-3-(3-chlorophényl)-4,5-dihydro-1*H*-pyrazole |
| « A9 » | 1-(4-Fluorobenzylcarbonyl)-3-(4-méthoxyphényl)-4,5-dihydro-1*H*-pyrazole |
| « A10 » | 1-(3-Méthoxybenzylcarbonyl)-3-(4-méthoxyphényl)-4,5-dihydro-1*H*-pyrazole |
| « A11 » | 1-(4-Méthoxybenzylcarbonyl)-3-(4-méthoxyphényl)-4,5-dihydro-1*H*-pyrazole |
| « A12 » | 1-Benzylcarbonyl-3-(4-méthoxyphényl)-4,5-dihydro-1*H*-pyrazole |
| « A13 » | 1-(4-Fluorobenzylcarbonyl)-3-(2-méthoxyphényl)-4,5-dihydro-1*H*-pyrazole |
| « A14 » | 1-(3-Méthoxybenzylcarbonyl)-3-(2-méthoxyphényl)-4,5-dihydro-1*H*-pyrazole |
| « A15 » | 1-(4-Méthoxybenzylcarbonyl)-3-(2-méthoxyphényl)-4,5-dihydro-1*H*-pyrazole |
| « A16 » | 1-(Thiophén-2-ylméthylcarbonyl)-3-(2-méthoxyphényl)-4,5-dihydro-1*H*-pyrazole |
| « A17 » | 1-Benzylcarbonyl-3-(2-méthoxyphényl)-4,5-dihydro-1*H*-pyrazole |
| « A18 » | 1-(3-Méthoxybenzylcarbonyl)-3-(4-diméthylaminophényl)-4,5-dihydro-1*H*-pyrazole |
| « A19 » | 1-(4-Méthoxybenzylcarbonyl)-3-(4-diméthylaminophényl)-4,5-dihydro-1*H*-pyrazole |
| « A20 » | 1-(Thiophén-2-ylméthylcarbonyl)-3-(4-diméthylaminophényl)-4,5-dihydro-1*H*-pyrazole |
| « A21 » | 1-(4-Fluorobenzylcarbonyl)-3-(4-fluorophényl)-4,5-dihydro-1*H*-pyrazole |
| « A22 » | 1-(4-Méthoxybenzylcarbonyl)-3-(4-fluorophényl)-4,5-dihydro-1*H*-pyrazole |
| « A23 » | 1-(Thiophén-2-ylméthylcarbonyl)-3-(4-fluorophényl)-4,5-dihydro-1*H*-pyrazole |
| « A24 » | 1-Benzylcarbonyl-3-(4-fluorophényl)-4,5-dihydro-1*H*-pyrazole |
| « A25 » | 1-(Benzo-[1,2,5]thiadiazol-5-ylméthylcarbonyl)-3-(3,4-diméthoxyphényl)-5,5-diméthyl-4,5-dihydro-1*H*-pyrazole |
| « A26 » | 1-(Benzo-[1,2,5]thiadiazol-5-ylméthylcarbonyl)-3-(3,4-diméthoxyphényl)-4,4-diméthyl-4,5-dihydro-1*H*-pyrazole |
| « A27 » | 1-[3-(2-Morpholin-4-yléthoxy)benzylcarbonyl]-3-(5-chlorothiophén-2-yl)-4,5-dihydro-1*H*-pyrazole |
| « A28 » | 1-[3-(3-Diéthylaminopropoxy)benzylcarbonyl]-3-(5-chlorothiophén-2-yl)-4,5-dihydro-1*H*-pyrazole |
| « A29 » | 1-[3-(3-Diéthylaminopropoxy)benzylcarbonyl]-3-(3,4-diméthoxyphényl)-4,5-dihydro-1*H*-pyrazole |
| « A30 » | 1-(3-Hydroxybenzylcarbonyl)-3-(2,4-diméthoxyphényl)-4,5-dihydro-1*H*-pyrazole |
| « A31 » | 1-(3-Hydroxybenzylcarbonyl)-3-(4-fluorophényl)-4,5-dihydro-1*H*-pyrazole |
| « A32 » | 1-(Benzo-[1,2,5]thiadiazol-5-ylméthylcarbonyl)-3-phényl-4,5-dihydro-1*H*-pyrazole |
| « A33 » | 1-(Benzo-[1,2,5]thiadiazol-5-ylméthylcarbonyl)-3-(4-chlorophényl)-4,5-dihydro-1*H*-pyrazole |
| « A34 » | 1-(Benzo-[1,2,5]thiadiazol-5-ylméthylcarbonyl)-3-(5-chlorothiophén-2-yl)-4,5-dihydro-1*H*-pyrazole |
| « A35 » | 1-(Benzo-[1,2,5]thiadiazol-5-ylméthylcarbonyl)-3-(4-bromophényl)-4,5-dihydro-1*H*-pyrazole |
| « A36 » | 1-(3-Hydroxybenzylcarbonyl)-3-(4-chlorophényl)-4,5-dihydro-1*H*-pyrazole |
| « A37 » | 1-(Benzo-[1,2,5]thiadiazol-5-ylméthylcarbonyl)-3-(4-méthylsulfonamidophényl)-4,5-dihydro-1*H-*pyrazole |
| « A38 » | 1-(3-Hydroxybenzylcarbonyl)-3-(3,4-dihydro-2-oxo-1*H*-quinazolin-6-yl)-4,5-dihydro-1*H*-pyrazole |
| « A39 » | 1-(Benzo-[1,2,5]thiadiazol-5-ylméthylcarbonyl)-3-(3,4-dihydro-2-oxo-1*H*-quinazolin-6-yl)-4,5-dihydro-1*H*-pyrazole |
| « A40 » | 1-(Benzo-[1,2,5]thiadiazol-5-ylméthylcarbonyl)-3-(4-hydroxyphényl)-4,5-dihydro-1*H*-pyrazole |
| « A41 » | 1-(Benzo-[1,2,5]thiadiazol-5-ylméthylcarbonyl)-3-(2-hydroxyphényl)-4,5-dihydro-1*H*-pyrazole |
| « A42 » | 1-(Benzo-[1,2,5]thiadiazol-5-ylméthylcarbonyl)-3-(2-hydroxy-5-chlorophényl)-4,5-dihydro-1*H-*pyrazole |
| « A43 » | 1-(Benzo-[1,2,5]thiadiazol-5-ylméthylcarbonyl)-3-(3-chloro-6-méthoxyphényl)-4,5-dihydro-1*H-*pyrazole |
| « A44 » | 1-(Benzo-[1,2,5]thiadiazol-5-ylméthylcarbonyl)-3-(3-chloro-4-méthoxyphényl)-4,5-dihydro-1*H-*pyrazole |
| « A45 » | 1-(3-Hydroxybenzylcarbonyl)-3-(5-chlorothiophén-2-yl)-4,5-dihydro-1*H*-pyrazole |
| « A46 » | 1-(3-Méthoxybenzylcarbonyl)-3-(5-chlorothiophén-2-yl)-4,5-dihydro-1*H*-pyrazole |
| « A47 » | 1-[3-(3-Oxomorpholin-4-yl)benzylcarbonyl]-3-(3,4-diméthoxyphényl)-4,5-dihydro-1*H*-pyrazole |
| « A48 » | 1-(Phénéthylcarbonyl)-3-(3,4-diméthoxyphényl)-4,5-dihydro-1*H*-pyrazole |
| « A49 » | 1-[4-(Morpholin-4-ylcarbonyl)-phénylméthylcarbonyl]-3-(3,4-diméthoxyphényl)-4,5-dihydro-1*H*-pyrazole |
| « A50 » | 1-[3-(3-Diéthylaminopropoxycarbonylamino)-phénylméthylcarbonyl]-3-(3,4-diméthoxyphényl)-4,5-dihydro-1*H*-pyrazole |
| « A51 » | 1-{3-[3-(3-Diéthylaminopropyl)uréido]-phénylméthylcarbonyl}-3-(3,4-diméthoxyphényl)-4,5-dihydro-1*H*-pyrazole |
| « A52 » | 1-Benzylcarbonyl-3-(2,4-diméthoxyphényl)-4,5-dihydro-1*H*-pyrazole |
| « A53 » | 1-(3-Méthoxybenzylcarbonyl)-3-(2,4-diméthoxyphényl)-4,5-dihydro-1*H*-pyrazole |
| « A54 » | 1-(3-Méthoxybenzylcarbonyl)-3-(4-fluorophényl)-4,5-dihydro-1*H*-pyrazole |
| « A55 » | 1-(3-Méthoxybenzylcarbonyl)-3-(2,5-diméthoxyphényl)-4,5-dihydro-1*H*-pyrazole |
| « A56 » | 1-(Benzo-[1,2,5]thiadiazol-5-ylméthylcarbonyl)-3-(4-méthylsulfanylphényl)-4,5-dihydro-1*H*-pyrazole |
| « A57 » | 1-(Benzo-[1,2,5]thiadiazol-5-ylméthylcarbonyl)-3-(dibenzofuran-2-yl)-4,5-dihydro-1*H*-pyrazole |
| « A58 » | 1-(4-Hydroxybenzylcarbonyl)-3-(4-chlorophényl)-4,5-dihydro-1*H*-pyrazole |
| « A59 » | 1-[4-(Morpholin-4-yléthoxy)benzylcarbonyl]-3-(3,4-diméthoxyphényl)-4,5-dihydro-1*H*-pyrazole |
| « A60 » | 1-[4-(3-Diéthylaminopropoxy)benzylcarbonyl]-3-(5-chlorothiophén-2-yl)-4,5-dihydro-1*H*-pyrazole |
| « A61 » | 1-(4-Hydroxybenzylcarbonyl)-3-(2,4-diméthoxyphényl)-4,5-dihydro-1*H*-pyrazole |
| « A62 » | 1-(3-Hydroxybenzylcarbonyl)-3-(3,4-diméthoxyphényl)-4,5-dihydro-1*H*-pyrazole |
| « A63 » | 1-(3-Chlorobenzylcarbonyl)-3-(2,4-diméthoxyphényl)-4,5-dihydro-1*H*-pyrazole |
| « A64 » | 1-(2-Benzo-[1,2,5]thiadiazol-5-ylpropionyl)-3-(4-fluorophényl)-4,5-dihydro-1*H*-pyrazole |
| « A65 » | 1-[2-Hydroxy-2-(3-hydroxyphényl)acétyl]-3-(4-fluorophényl)-4,5-dihydro-1*H*-pyrazole |
| « A66 » | 1-[2-Hydroxy-2-(3-hydroxyphényl)acétyl]-3-(4-chlorophényl)-4,5-dihydro-1*H*-pyrazole |
| « A67 » | 1-(2-Hydroxybenzylcarbonyl)-3-(2,4-diméthoxyphényl)-4,5-dihydro-1*H*-pyrazole |
| « A68 » | 1-(3-Hydroxybenzylcarbonyl)-3-(2-oxo-1,3-dihydroindol-5-yl)-4,5-dihydro-1*H*-pyrazole |
| « A69 » | 1-(4-Hydroxybenzylcarbonyl)-3-(4-fluorophényl)-4,5-dihydro-1*H*-pyrazole |
| « A70 » | 1-(2-Hydroxybenzylcarbonyl)-3-(4-fluorophényl)-4,5-dihydro-1*H*-pyrazole |
| « A71 » | 1-[4-(Morpholin-4-yléthoxy)benzylcarbonyl]-3-(5-chlorothiophén-2-yl)-4,5-dihydro-1*H*-pyrazole |
| « A72 » | 1-(4-Fluorobenzylcarbonyl)-3-(5-chlorothiophén-2-yl)-4,5-dihydro-1*H*-pyrazole |
| « A73 » | 1-(4-Méthoxybenzylcarbonyl)-3-(5-chlorothiophén-2-yl)-4,5-dihydro-1*H*-pyrazole |
| « A74 » | 1-(4-Acétamidobenzylcarbonyl)-3-(5-chlorothiophén-2-yl)-4,5-dihydro-1*H*-pyrazole |
| « A75 » | 1-(Benzo-[1,2,5]thiadiazol-5-ylméthylcarbonyl)-3-(5-chlorofuran-2-yl)-4,5-dihydro-1*H*-pyrazole |
| « A76 » | 1-(4-Hyd roxy-3-méthoxybenzylcarbonyl)-3-(5-chlorothiophén-2-yl)-4,5-dihydro-1*H*-pyrazole |
| « A77 » | 1-(4-Méthylsulfonylbenzylcarbonyl)-3-(5-chlorothiophén-2-yl)-4,5-dihydro-1*H*-pyrazole |
| « A78 » | 1-(4-Éthoxybenzylcarbonyl)-3-(5-chlorothiophén-2-yl)-4,5-dihydro-1*H*-pyrazole |
| « A79 » | 1-(4-Hydroxy-3-chlorobenzylcarbonyl)-3-(5-chlorothiophén-2-yl)-4,5-dihydro-1*H*-pyrazole |
| « A80 » | 1-(2-(4-Hydroxyphényl)propionyl)-3-(5-chlorothiophén-2-yl)-4,5-dihydro-1*H*-pyrazole |
| « A81 » | 1-(2-(Benzo-[1,2,5]thiadiazol-5-yl)propionyl)-3-(5-chlorothiophén-2-yl)-4,5-dihydro-1*H*-pyrazole |
| « A82 » | 1-(2-(4-Fluorophényl)propionyl)-3-(5-chlorothiophén-2-yl)-4,5-dihydro-1*H*-pyrazole |
| « A83 » | 1-(Benzo-[1,2,5]thiadiazol-5-ylméthylcarbonyl)-3-(3-amino-1*H*-indazol-5-yl)-4,5-dihydro-1*H*-pyrazole |
| « A84 » | 1-(Benzo-[1,2,5]thiadiazol-5-ylméthylcarbonyl)-3-(2-hydroxyphényl)-4,5-dihydro-1*H*-pyrazole |
| « A85 » | 1-(3-Chlorobenzylcarbonyl)-3-(3,4-diméthoxyphényl)-4,5-dihydro-1*H*-pyrazole |
| « A86 » | 1-(Benzo-[1,2,5]thiadiazol-5-ylméthylcarbonyl)-3-(2-oxo-3,4-dihydro-1*H*-quinazolin-6-yl)-4,5-dihydro-1*H*-pyrazole |
| « A87 » | 1-(Benzo-[1,2,5]thiadiazol-5-ylméthylcarbonyl)-3-(4-hydroxyphényl)-4,5-dihydro-1*H*-pyrazole |
| « A88 » | 1-(3-Hydroxybenzylcarbonyl)-3-(2-oxo-3,4-dihydro-1*H*-quinazolin-6-yl)-4,5-dihydro-1*H*-pyrazole |
| « A89 » | 1-(Benzo-[1,2,5]thiadiazol-5-ylméthylcarbonyl)-3-(3-chloro-6-hydroxyphényl)-4,5-dihydro-1*H-*pyrazole |
| « A90 » | 1-(3-Hydroxybenzylcarbonyl)-3-(5-chlorothiophén-2-yl)-4,5-dihydro-1*H*-pyrazole |
| « A91 » | 1-(3-Hydroxybenzylcarbonyl)-3-(4-hydroxyphényl)-4,5-dihydro-1*H*-pyrazole |
| « A92 » | 1-(Benzo-[1,3]dioxol-5-ylméthylcarbonyl)-3-(5-chlorothiophén-2-yl)-4,5-dihydro-1*H*-pyrazole |
| « A93 » | 1-(4-Hydroxybenzylcarbonyl)-3-(5-chlorothiophén-2-yl)-4,5-dihydro-1*H*-pyrazole |
| « A94 » | 1-(4-Méthoxybenzylcarbonyl)-3-(5-chlorothiophén-2-yl)-4,5-dihydro-1*H*-pyrazole |
| « A95 » | 1-(Benzo-[1,2,5]thiadiazol-5-ylméthylcarbonyl)-3-(3-chloro-6-méthoxyphényl)-4,5-dihydro-1*H-*pyrazole |
| « A96 » | 1-(4-Hydroxybenzylcarbonyl)-3-(3,4-diméthoxyphényl)-4,5-dihydro-1*H*-pyrazole |
| « A97 » | 1-(3-Chloro-4-hydroxybenzylcarbonyl)-3-(5-chlorothiophén-2-yl)-4,5-dihydro-1*H*-pyrazole |
| « A98 » | 3-(5-Chlorothiophén-2-yl)-1-[2-(4-hydroxyphényl)propionyl]-4,5-dihydro-1*H*-pyrazole |
| | 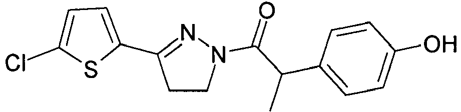 |
| « A99 » | 1-(4-Méthoxybenzylcarbonyl)-3-(3,4-diméthoxyphényl)-4,5-dihydro-1*H*-pyrazole |
| « A100 » | 3-(2-Oxo-3,4-dihydro-1*H*-quinazolin-6-yl)-1-[2-(4-hydroxyphényl)propionyl]-4,5-dihydro-1*H*-pyrazole |
| | 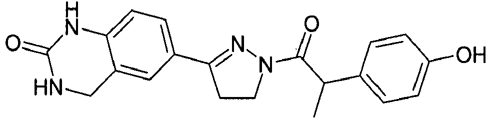 |
| « A101 » | 1-[3-(3-Oxomorpholin-4-yl)benzylcarbonyl]-3-(5-chlorothiophén-2-yl)-4,5-dihydro-1*H*-pyrazole |
| | 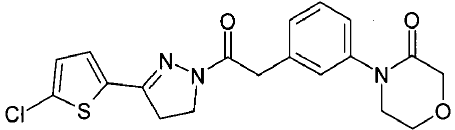 |
| « A102 » | 1-[3-(3-Oxomorpholin-4-yl)benzylcarbonyl]-3-(3,4-diméthoxyphényl)-4,5-dihydro-1*H*-pyrazole |
| « A103 » | 3-(3-Chloro-6-hydroxyphényl)-1-[2-(benzo-1,2,5-thiadiazol-5-yl)propionyl]-4,5-dihydro-1*H*-pyrazole |
| | 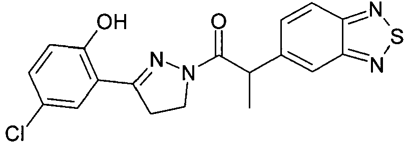 |
| « A104 » | 1-[4-(3-Morpholin-4-ylpropoxy)benzylcarbonyl]-3-(5-chlorothiophén-2-yl)-4,5-dihydro-1*H*-pyrazole |
| | 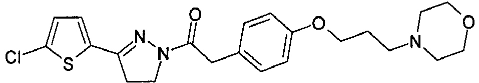 |
| « A105 » | 1-[2-(1*H*-Benzimidazol-5-yl)acétyl]-3-(5-chlorothiophén-2-yl)-4,5-dihydro-1*H*-pyrazole |
| | 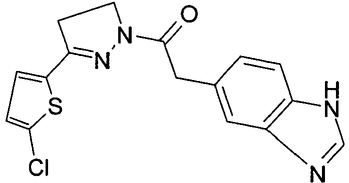 |
| « A106 » | 3-(4-Chlorophényl)-1-(3-phénylpropionyl)-4,5-dihydro-1*H*-pyrazole |
| | 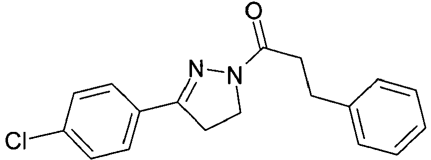 |
| « A107 » | 1-(4-Méthylsulfonylbenzylcarbonyl)-3-(4-chlorophényl)-4,5-dihydro-1*H*-pyrazole |
| « A108 » | 1-[2-(1*H*-Benzimidazol-5-yl)acétyl]-3-(3-chloro-6-hydroxyphényl)-4,5-dihydro-1*H*-pyrazole |
| « A109 » | 1-[3-(2-Morpholin-4-yléthoxy)benzylcarbonyl]-3-(5-chlorothiophén-2-yl)-4,5-dihydro-1*H*-pyrazole |
| « A110 » | 1-(Benzo-[1,3]dioxol-5-ylméthylcarbonyl)-3-(3-chloro-6-hydroxyphényl)-4,5-dihydro-1*H*-pyrazole |
| « A111 » | 3-(4-Chlorophényl)-1-[2-(4-hydroxyphényl)propionyl]-4,5-dihydro-1*H*-pyrazole |
| « A112 » | 1-[2-(1*H*-Benzimidazol-5-yl)acétyl]-3-(4-chlorophényl)-4,5-dihydro-1*H*-pyrazole |
| « A113 » | 1-(3-Chloro-4-hydroxybenzylcarbonyl)-3-(3-chloro-6-hydroxyphényl)-4,5-dihydro-1*H*-pyrazole |
| « A114 » | 1-(4-Hydroxybenzylcarbonyl)-3-(3-chloro-6-méthoxyphényl)-4,5-dihydro-1*H*-pyrazole |
| « A115 » | 1-(Benzo-[1,3]dioxol-5-ylméthylcarbonyl)-3-(2-oxo-3,4-dihydro-1*H*-quinazolin-6-yl)-4,5-dihydro-1*H-*pyrazole |
| « A116 » | 3-(3,4-Diméthoxyphényl)-1-[2-(benzo-[1,2,5]thiadiazol-5-yl)acétyl]-5,5-diméthyl-4,5-dihydro-1*H*-pyrazole |
| « A117 » | 1-{4-[3-(N,N-Diéthoxyamino)propoxy]-benzylcarbonyl}-3-(4-chlorophényl)-4,5-dihydro-1*H-*pyrazole |
| « A118 » | 3-(5-Chlorothiophén-2-yl)-1-[2-(quinoxalin-6-yl)acétyl]-4,5-dihydro-1*H*-pyrazole |
| « A119 » | 3-(3-Chloro-6-hydroxyphényl)-1-[2-(quinoxalin-6-yl)acétyl]-4,5-dihydro-1*H*-pyrazole |
| « A120 » | 3-(3-Chloro-6-hydroxyphényl)-1-[2-(4-hydroxyphényl)propionyl]-4,5-dihydro-1*H*-pyrazole |
| « A121 » | 1-(4-Hydroxybenzylcarbonyl)-3-(3-chloro-6-hydroxyphényl)-4,5-dihydro-1*H*-pyrazole |
| « A122 » | 1-(3-Chlorobenzylcarbonyl)-3-(3-chloro-6-hydroxyphényl)-4,5-dihydro-1*H*-pyrazole |
| « A123 » | 1-(2-{4-[3-(N, N-Diéthoxyamino)-propoxy]phényl}propionyl)-3-(4-chlorophényl)-4,5-dihydro-1*H*-pyrazole |
| | 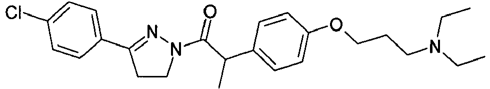 |
| « A124 » | 1-[4-(Morpholin-4-ylcarbonyl)benzylcarbonyl]-3-(5-chlorothiophén-2-yl)-4,5-dihydro-1*H*-pyrazole |
| « A125 » | 1-[4-(1-Méthylpipérazin-4-ylcarbonyl)-benzylcarbonyl]-3-(5-chlorothiophén-2-yl)-4,5-dihydro-1*H*-pyrazole |
| « A126 » | 1-[2-(Pyridin-3-yl)acétyl]-3-(3-chloro-6-hydroxyphényl)-4,5-dihydro-1*H*-pyrazole |
| « A127 » | 1-[2-(Pyridin-2-yl)acétyl]-3-(3-chloro-6-hydroxyphényl)-4,5-dihydro-1*H*-pyrazole |
| « A128 » | 1-(4-Cyanobenzylcarbonyl)-3-(5-chlorothiophén-2-yl)-4,5-dihydro-1*H*-pyrazole |
| « A129 » | 1-[2-(Pyridin-3-yl)acétyl]-3-(5-chlorothiophén-2-yl)-4,5-dihydro-1*H*-pyrazole |
| « A130 » | 1-[2-(Pyridin-2-yl)acétyl]-3-(5-chlorothiophén-2-yl)-4,5-dihydro-1*H*-pyrazole |
| « A131 » | 1-[2-(1H-Benzimidazol-5-yl)acétyl]-3-(3-chlorophényl)-4,5-dihydro-1*H*-pyrazole |
| « A132 » | 1-(9*H*-Carbazol-3-ylméthylcarbonyl)-3-(3-chloro-6-hydroxyphényl)-4,5-dihydro-1*H*-pyrazole |
| « A133 » | 3-(3-Chlorophényl)-1-[2-(benzo-1,2,5-thiadiazol-5-yl)acétyl]-4,5-dihydro-1*H*-pyrazole |
| « A134 » | 1-{2-[2-(5-Chlorothiophén-2-yl)-(1*H*-benzimidazol-5-yl]acétyl}-3-(5-chlorothiophén-2-yl)-4,5-dihydro-1*H*-pyrazole |
| | 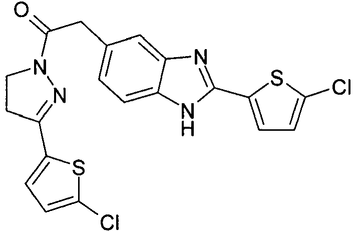 |
| « A135 » | 1-{2-[2-(5-Chlorothiophén-2-yl)-(1*H*-benzimidazol-5-yl]acétyl}-3-(3-chloro-6-hydroxyphényl)-4,5-dihydro-1*H*-pyrazole |
| « A136 » | 3-(3-Chloro-6-hydroxyphényl)-1-[2-(2-aminobenzothiazol-6-yl)propionyl]-4,5-dihydro-1*H-*pyrazole |
| | 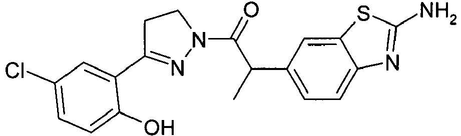 |
| « A137 » | 3-(4-Fluorophényl)-1-[2-(2-aminobenzothiazol-6-yl)propionyl]-4,5-dihydro-1*H*-pyrazole |
| « A138 » | 3-(4-Fluorophényl)-1-[2-(2-aminobenzimidazol-6-yl)acétyl]-4,5-dihydro-1*H*-pyrazole |
| « A139 » | 1-(3-Nitrobenzylcarbonyl)-3-(3-chloro-6-hydroxy)-4,5-dihydro-1*H*-pyrazole |
| « A140 » | 3-(2,5-Dichlorophényl)-1-[2-(benzo-1,2,5-thiadiazol-5-yl)acétyl]-4,5-dihydro-1*H*-pyrazole |
| « A141 » | 3-(2,5-Dichlorophényl)-1-[2-(1*H*-benzimidazol-5-yl)acétyl]-4,5-dihydro-1*H*-pyrazole |
| « A142 » | 3-(4-Fluorophényl)-1-[2-(1*H*-benzotriazol-5-yl)acétyl]-4,5-dihydro-1*H*-pyrazole |
| « A143 » | 3-(3-Chloro-6-hydroxyphényl)-1-[2-(1*H-*benzotriazol-5-yl)acétyl]-4,5-dihydro-1*H*-pyrazole |
| « A144 » | (3-{2-[3-(4-Fluorophényl)-4,5-dihydro-1*H*-pyrazol-1-yl]-2-oxoéthyl}phényl)carbamate de 3-diméthylaminopropyle |
| | 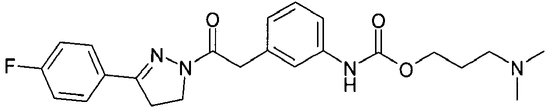 |
| « A145 » | 3-(2,5-Difluorophényl)-1-[2-(1*H*-benzimidazol-5-yl)acétyl]-4,5-dihydro-1*H*-pyrazole |
| « A146 » | 3-(2,5-Difluorophényl)-1-[2-(4,5-dihydro-3-oxo-2H-pyridazin-6-yl)acétyl]-4,5-dihydro-1*H*-pyrazole |
| | 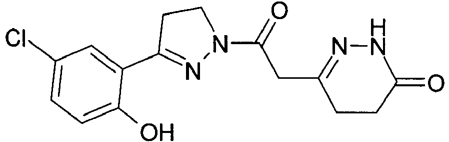 |
| « A147 » | 3-(3-Fluorophényl)-1-[2-(benzo-1,2,5-thiadiazol-5-yl)acétyl]-4,5-dihydro-1*H*-pyrazole |
| « A148 » | (3-{2-[3-(2,5-Difluorophényl)-4,5-dihydro-1*H-*pyrazol-1-yl]-2-oxoéthyl}phényl)carbamate de 3-diméthylaminopropyle |
| « A149 » | 1-(4-Hydroxybenzylcarbonyl)-3-phényl-4,5-dihydro-1*H*-pyrazole |
| « A150 » | (3-{2-[3-(4-Fluorophényl)-4,5-dihydro-1*H*-pyrazol-1-yl]-2-oxoéthyl}phényl)carbamate de 3-morpholin-4-ylpropyle |
| | 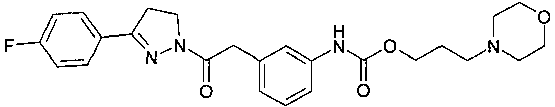 |
| « A151 » | 3-(3-Fluorophényl)-1-[2-(1*H*-benzimidazol-5-yl)acétyl]-4,5-dihydro-1*H*-pyrazole |
| « A152 » | (3-{2-[3-(2,5-Difluorophényl)-4,5-dihydro-1*H-*pyrazol-1-yl]-2-oxoéthyl}phényl)carbamate de 3-morpholin-4-ylpropyle |
| « A153 » | (3-{2-[3-(2,5-Dichlorophényl)-4,5-dihydro-1*H-*pyrazol-1-yl]-2-oxoéthyl}phényl)carbamate de 3-diméthylaminopropyle |
| « A155 » | (3-{2-[3-(3-Fluorophényl)-4,5-dihydro-1*H*-pyrazol-1-yl]-2-oxoéthyl}phényl)carbamate de 3-diméthylaminopropyle |
| « A156 » | (3-{2-[3-(3-Fluorophényl)-4,5-dihydro-1*H*-pyrazol-1-yl]-2-oxoéthyl}phényl)carbamate de 3-morpholin-4-ylpropyle |
| « A157 » | 1-(4-Fluorobenzylcarbonyl)-3-(4-chlorophényl)-4,5-dihydro-1*H*-pyrazole |
| « A158 » | 3-(4-Chlorophényl)-1-[2-(thiophén-2-yl)acétyl]-4,5-dihydro-1*H*-pyrazole |
| « A157a » | 3-(3,5-Difluorophényl)-1-[2-(benzo-1,2,5-thiadiazol-5-yl)acétyl]-4,5-dihydro-1*H*-pyrazole |
| « A158a » | 3-(3,5-Difluorophényl)-1-[2-(1,3-dihydro-2-oxobenzimidazol-5-yl)acétyl]-4,5-dihydro-1*H-*pyrazole |
| | 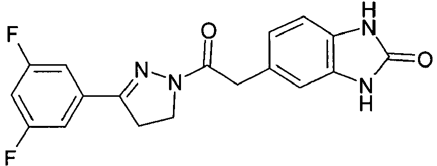 |
| « A159 » | 3-(3,5-Difluorophényl)-1-[2-(benzimidazol-5-yl)acétyl]-4,5-dihydro-1*H*-pyrazole |
| « A159a » | 1-(4-Fluorobenzylcarbonyl)-3-(3-méthoxyphényl)-4,5-dihydro-1*H*-pyrazole |
| « A160 » | 1-(3-Méthoxybenzylcarbonyl)-3-(3-méthoxyphényl)-4,5-dihydro-1*H*-pyrazole |
| « A161 » | 1-(4-Méthoxybenzylcarbonyl)-3-(3-méthoxyphényl)-4,5-dihydro-1*H*-pyrazole |
| « A162 » | 3-(3-Méthoxyphényl)-1-[3-(3,4,5-triméthoxyphényl)propionyl]-4,5-dihydro-1*H-*pyrazole |
| « A163 » | 1-Benzylcarbonyl-3-(3-méthoxyphényl)-4,5-dihydro-1*H*-pyrazole |
| « A164 » | 1-(4-Fluorobenzylcarbonyl)-3-(3-nitrophényl)-4,5-dihydro-1*H*-pyrazole |
| « A165 » | 1-(3-Méthoxybenzylcarbonyl)-3-(3-nitrophényl)-4,5-dihydro-1*H*-pyrazole |
| « A166 » | 3-(3-Nitrophényl)-1-[2-(thiophén-2-yl)acétyl]-4,5-dihydro-1*H*-pyrazole |
| « A167 » | 1-Benzylcarbonyl-3-(3-nitrophényl)-4,5-dihydro-1*H-*pyrazole |
| « A168 » | 1-(4-Fluorobenzylcarbonyl)-3-(furan-2-yl)-4,5-dihydro-1*H*-pyrazole |
| « A169 » | 1-(3-Méthoxybenzylcarbonyl)-3-(furan-2-yl)-4,5-dihydro-1*H*-pyrazole |
| « A170 » | 1-(4-Méthoxybenzylcarbonyl)-3-(furan-2-yl)-4,5-dihydro-1*H*-pyrazole |
| « A171 » | 3-(Furan-2-yl)-1-[2-(thiophén-2-yl)acétyl]-4,5-dihydro-1*H*-pyrazole |
| « A172 » | 1-Benzylcarbonyl-3-(furan-2-yl)-4,5-dihydro-1*H-*pyrazole |
| « A173 » | 1-Benzylcarbonyl-3-[4-(morpholin-4-yl)phényl]-4,5-dihydro-1*H*-pyrazole |
| « A174 » | 1-(3-Méthoxybenzylcarbonyl)-3-(2-fluorophényl)-4,5-dihydro-1*H*-pyrazole |
| « A175 » | 1-(4-Méthoxybenzylcarbonyl)-3-(2-fluorophényl)-4,5-dihydro-1*H*-pyrazole |
| « A176 » | 1-Benzylcarbonyl-3-(2-fluorophényl)-4,5-dihydro-1*H*-pyrazole |
| « A177 » | 1-(4-Fluorobenzylcarbonyl)-3-(4-cyanophényl)-4,5-dihydro-1*H*-pyrazole |
| « A178 » | 1-(3-Méthoxybenzylcarbonyl)-3-(4-cyanophényl)-4,5-dihydro-1*H*-pyrazole |
| « A179 » | 1-(4-Méthoxybenzylcarbonyl)-3-(4-cyanophényl)-4,5-dihydro-1*H*-pyrazole |
| « A180 » | 1-Benzylcarbonyl-3-(4-cyanophényl)-4,5-dihydro-1*H*-pyrazole |
| « A181 » | 1-(4-Fluorobenzylcarbonyl)-3-(3-cyanophényl)-4,5-dihydro-1*H*-pyrazole |
| « A182 » | 1-(3-Méthoxybenzylcarbonyl)-3-(3-cyanophényl)-4,5-dihydro-1*H*-pyrazole |
| « A183 » | 1-(4-Méthoxybenzylcarbonyl)-3-(3-cyanophényl)-4,5-dihydro-1*H*-pyrazole |
| « A184 » | 1-Benzylcarbonyl-3-(3-cyanophényl)-4,5-dihydro-1*H*-pyrazole |
| « A185 » | 1-(4-Fluorobenzylcarbonyl)-3-(4-méthylphényl)-4,5-dihydro-1*H*-pyrazole |
| « A186 » | 1-(3-Méthoxybenzylcarbonyl)-3-(4-méthylphényl)-4,5-dihydro-1*H*-pyrazole |
| « A187 » | 1-Benzylcarbonyl-3-(4-méthylphényl)-4,5-dihydro-1*H*-pyrazole |
| « A188 » | 1-(4-Fluorobenzylcarbonyl)-3-(3,4-diméthoxyphényl)-4,5-dihydro-1*H*-pyrazole |
| « A189 » | 1-(3-Méthoxybenzylcarbonyl)-3-(3,4-diméthoxyphényl)-4,5-dihydro-1*H*-pyrazole |
| « A190 » | 1-Benzylcarbonyl-3-(3,4-diméthoxyphényl)-4,5-dihydro-1*H*-pyrazole |
| « A191 » | 1-(4-Fluorobenzylcarbonyl)-3-(benzo-[1,3]dioxol-5-yl)-4,5-dihydro-1*H*-pyrazole |
| « A192 » | 1-(3-Méthoxybenzylcarbonyl)-3-(benzo-[1,3]dioxol-5-yl)-4,5-dihydro-1*H*-pyrazole |
| « A193 » | 1-(4-Méthoxybenzylcarbonyl)-3-(benzo-[1,3]dioxol-5-yl)-4,5-dihydro-1*H*-pyrazole |
| « A194 » | 1-[2-(Thiophén-2-yl)acétyl]-3-(benzo-[1,3]dioxol-5-yl)-4,5-dihydro-1*H*-pyrazole |
| « A195 » | 1-Benzylcarbonyl-3-(benzo-[1,3]dioxol-5-yl)-4,5-dihydro-1*H*-pyrazole |
| « A196 » | 1-(4-Fluorobenzylcarbonyl)-3-(4-éthoxyphényl)-4,5-dihydro-1*H*-pyrazole |
| « A197 » | 1-(3-Méthoxybenzylcarbonyl)-3-(4-éthoxyphényl)-4,5-dihydro-1*H*-pyrazole |
| « A198 » | 1-(4-Méthoxybenzylcarbonyl)-3-(4-éthoxyphényl)-4,5-dihydro-1*H*-pyrazole |
| « A199 » | 1-Benzylcarbonyl-3-(4-éthoxyphényl)-4,5-dihydro-1*H*-pyrazole |
| « A200 » | 1-(4-Fluorobenzylcarbonyl)-3-(thiophén-2-yl)-4,5-dihydro-1*H*-pyrazole |
| « A201 » | 1-(4-Méthoxybenzylcarbonyl)-3-(thiophén-2-yl)-4,5-dihydro-1*H*-pyrazole |
| « A202 » | 1-[2-(Thiophén-2-yl)acétyl]-3-(thiophén-2-yl)-4,5-dihydro-1*H*-pyrazole |
| « A203 » | 1-Benzylcarbonyl-3-(thiophén-2-yl)-4,5-dihydro-1*H-*pyrazole |
| « A204 » | 1-(4-Fluorobenzylcarbonyl)-3-[4-(morpholin-4-yl)phényl]-4,5-dihydro-1*H*-pyrazole |
| « A205 » | 1-(3-Méthoxybenzylcarbonyl)-3-[4-(morpholin-4-yl)phényl]-4,5-dihydro-1*H*-pyrazole |
| « A206 » | 1-(4-Méthoxybenzylcarbonyl)-3-[4-(morpholin-4-yl)phényl]-4,5-dihydro-1*H*-pyrazole |
| « A207 » | 1-[2-(Thiophén-2-yl)acétyl]-3-[4-(morpholin-4-yl)phényl]-4,5-dihydro-1*H*-pyrazole |
| « A208 » | 1-(4-Fluorobenzylcarbonyl)-3-(2-fluorophényl)-4,5-dihydro-1*H*-pyrazole |
| « A209 » | 1-(4-Fluorobenzylcarbonyl)-3-(2,5-diméthoxyphényl)-4,5-dihydro-1*H*-pyrazole |
| « A210 » | 1-(4-Méthoxybenzylcarbonyl)-3-(2,5-diméthoxyphényl)-4,5-dihydro-1*H*-pyrazole |
| « A211 » | 1-[2-(Thiophén-2-yl)acétyl]-3-(2,5-diméthoxyphényl)-4,5-dihydro-1*H*-pyrazole |
| « A212 » | 1-Benzylcarbonyl-3-(2,5-diméthoxyphényl)-4,5-dihydro-1*H*-pyrazole |
| « A213 » | 1-[2-(Thiophén-2-yl)acétyl]-3-(2,3,4-triméthoxyphényl)-4,5-dihydro-1*H*-pyrazole |
| « A214 » | 1-Benzylcarbonyl-3-(2,3,4-triméthoxyphényl)-4,5-dihydro-1*H*-pyrazole |
| « A215 » | 1-(4-Fluorobenzylcarbonyl)-3-(4-diméthylaminophényl)-4,5-dihydro-1*H*-pyrazole |
| « A216 » | 1-Benzylcarbonyl-3-(4-diméthylaminophényl)-4,5-dihydro-1*H*-pyrazole |
| « A217 » | 3-(2,5-Dichlorophényl)-1-[2-(2-aminobenzothiazol-6-yl)propionyl]-4,5-dihydro-1*H*-pyrazole |
| « A218 » | 3-(2,5-Dichlorophényl)-1-[2-(2-aminobenzothiazol-6-yl)acétyl]-4,5-dihydro-1*H*-pyrazole |
et les solvats, sels, tautomères et stéréoisomères pharmaceutiquement utilisables de ceux-ci, y compris des mélanges de ceux-ci en toutes proportions.

2. Médicaments comprenant au moins un composé selon la revendication 1 et/ou des sels, solvats, tautomères et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1, et de sels, solvats et stéréoisomères pharmaceutiquement utilisables de ceux-ci, y compris des mélanges de ceux-ci en toutes proportions,
pour la préparation d'un médicament destiné au traitement de maladies, où la maladie à traiter est une tumeur solide ou une tumeur du sang et du système immunitaire.

4. Utilisation selon la revendication 3, dans laquelle la tumeur solide provient du groupe constitué par les tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, du larynx et/ou du poumon.

5. Utilisation selon la revendication 3, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

6. Utilisation selon la revendication 3, dans laquelle la tumeur solide provient du groupe constitué par l'adénocarcinome du poumon, les carcinomes du poumon à petites cellules, le cancer du pancréas, les glioblastomes, le carcinome du côlon et le carcinome du sein.

7. Utilisation selon la revendication 3, dans laquelle la tumeur provient du groupe constitué par la leucémie myéloïde aiguë, la leucémie myéloïde chronique, la leucémie lymphatique aiguë et/ou la leucémie lymphatique chronique.

8. Médicaments comprenant au moins un composé selon la revendication 1, et/ou des sels, solvats et stéréoisomères pharmaceutiquement utilisables de celui-ci, y compris des mélanges de ceux-ci en toutes proportions, et au moins un autre composé actif médicamenteux.

9. Ensemble (kit) constitué de conditionnements séparés
(a) d'une quantité efficace d'un composé selon la revendication 1, et/ou de solvats, sels et stéréoisomères pharmaceutiquement utilisables de celui-ci, y compris des mélanges de ceux-ci en toutes proportions,
et
(b) d'une quantité efficace d'un autre composé actif médicamenteux.
